# EUROPEAN PATENT APPLICATION

(11) **EP 4 790 061 A2**
(43) Date of publication of application: **12.08.2026**
(21) Application number: 26165474.3
(22) Date of filing: 24.10.2018
(51) Int. Cl.: C07J 71/00

(54) **NOVEL DEOXYCHOLIC ACID PRODUCTS AND PREPARATION THEROF**

(30) Priority: 24.10.2017 EP 17198074
(62) Document number(s) of the earlier application(s) in accordance with Art. 76 EPC: 18788792.2 / 3 700 916
(71) Applicant: Curia Spain S.A.U., 47151 Boecillo, Valladolid (ES)
(72) Inventor: HERRAIZ SIERRA, Ignacio, 47151 Valladolid (ES); PÉREZ ENCABO, Alfonso, 47011 Valladolid (ES); LORENTE BONDE-LARSEN, Antonio, 47151 Valladolid (ES); GUERRA NAVARRO, Francisco Javier, 47151 Valladolid (ES); FERNANDEZ SAINZ, Yolanda, 47151 Valladolid (ES); BARREDO FUENTE, José Luis, 24009 Léon (ES); TURIEL HERNANDEZ, José Angel, 47011 Valladolid (ES)
(74) Representative: Plougmann Vingtoft a/s

(57) **Abstract**

The present invention relates to to novel DCA products and pharmaceutically acceptable salts thereof derived partially or solely from plant sources, as well as to new and improved processes for the preparation of deoxycholic acid (DCA) and pharmaceutically acceptable salts thereof, the carbon atoms of which are derived partially or solely from plant sources.

## Description

### Field of the invention

The present invention relates to novel DCA products and pharmaceutically acceptable salts thereof derived partially or solely from plant sources. The present invention further relates to new and improved processes for the preparation of deoxycholic acid (DCA) and pharmaceutically acceptable salts thereof..

### Background of the invention

Deoxycholic acid (DCA) is a known drug compound. DCA has the CAS number [83-44-3], and is also known as deoxycholate, cholanoic acid, and 3α,12β-dihydroxy-5β-cholanate. Pure DCA is a white to off-white crystalline powder.

DCA is one of the secondary bile acids, which are metabolic byproducts of intestinal bacteria.

Since its discovery DCA has been used in various fields of human medicine. In the human body DCA is used in the emulsification of fats for the absorption in the intestine. Also, when injected into submental fat, DCA helps destroying fat cells.

In the United States, DCA has been approved by the Food and Drug Administration (FDA) for reducing moderate-to-severe fat below the chin, and is marketed under the trademark Kybella^{®}. Kybella^{®} is produced by Kythera Biopharmaceuticals.

Recent patent applications describing DCAs fat-reducing properties include WO 2005/117900, WO 2005/112942, US 2005/0261258, US 2005/0267080, US 2006/127468 and US 2006/0154906.

Pharmaceutical preparations containing bile acids are commercially available at rather low costs, because bile acids are easily available from animal corpses, such as cows and sheep.

However, bile acids obtained from animal sources may contain pathogens, such as prions, or other harmful agents, such as toxins.

Bile acids from animal sources are typically purified in order to exclude impurities. In practice such purified compositions contain a mixture of bile acids. For example, commercially available compositions of DCA of animal origin contain some chenodoxycholic acid and cholic acid.

Accordingly, bile acids, including DCA, obtained either synthetically or from plant sources, have recently gained increased interest since the above-mentioned problems associated with bile acids from animal origin can thereby be eliminated.

Thus, there is a need for novel and efficient synthetic routes for preparing bile acids, including DCA, where the starting compounds are steroids, sterols or fermented phytosterols of vegetable origin.

It is known to prepare DCA starting from phytosterols obtained by fermentation of a *Mycobacterium* strain. For example, WO 2008/157635 and WO 2013/044119 describe the synthesis of DCA from 9-hydroxy-4-androstene-3,17-dione:

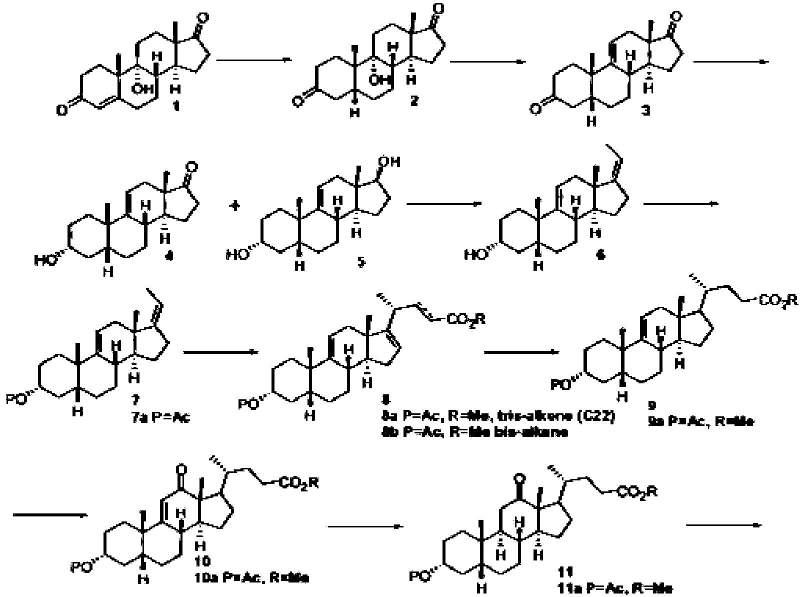

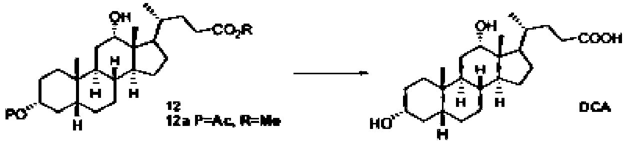

However, this process involves at least 11 steps plus additional purifications. In addition, the carbon chain (at position 17) is generated without a defined stereochemistry. Accordingly, the overall yield is low, which makes the process less attractive from an industrial point of view.

WO 2008/157635 and WO 2012/047495 teach preparation of DCA starting from cortisone and hydrocortisone. However, these processes involve numerous individual steps and, in addition, cortisone and hydrocortisone are rather expensive starting materials.

Some molecules are expensive to produce petrochemically, for example molecules that incorporate oxygen into petrochemical raw materials wherein the fossil-petrol way involves a considerable cost in money and also ecological cost. For instance, the malonate reagent, wherein a common starting raw materials is monochloroacetic acid which is not a cheap starting material comparing with polysaccharides raw materials that are inexpensive, ton-scale accessible and provide a really worked chemistry.

As will be understood from the above review, there is still a need for providing synthetic routes for preparing DCA in good yield and high purity and where the starting compound is of vegetable origin. Moreover, there is still a need for providing synthetic routes for preparing DCA from starting compounds of vegetable origin, which are simpler than those described in the prior art.

In addition there is a need to prepare DCA partially or solely from plant sources.

### Summary of the invention

In a first aspect, the present invention relates to a deoxycholic acid or a pharmaceutically acceptable salt thereof of formula (DCA): 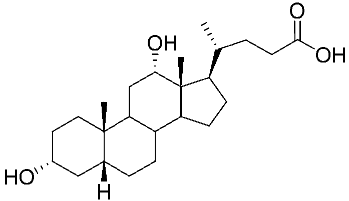 wherein the deoxycholic acid or the pharmaceutically acceptable salt thereof comprises a fossil carbon percentage of less than 3 percent, such as less than 1 percent, like less than 0.1 percent, such as less than 0.01 percent, and wherein the deoxycholic acid has a mean δ¹³C value in the range from -20‰ to -40‰.

In one embodiment, the carbon atoms of the deoxycholic acid or the pharmaceutically acceptable salt thereof are derived soley from plant sources.

In a further embodiment, the carbon atoms of the deoxycholic acid or the pharmaceutically acceptable salt thereof is derived partially from phytosterols or phytosterol derivatives.

In a further embodiment, the deoxycholic acid has a mean δ¹³C value in the range from -21‰ to -35‰, like from -20‰ to -32‰, such as from - 25‰ to -28‰, like around - 26‰.

In a second aspect, the present invention relates to a deoxycholic acid or a pharmaceutically acceptable salt thereof of formula (DCA): 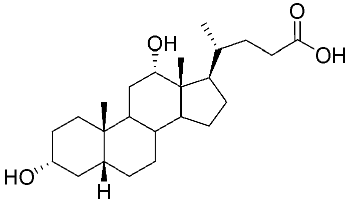 obtained by
i) providing an intermediate of the general formula **Int A8:** 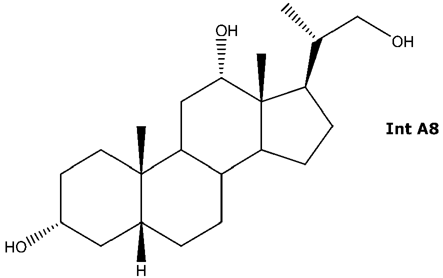 and
ii) elongating the carbon chain of the compound of the general formula **Int A8** to obtain deoxycholic acid (DCA): 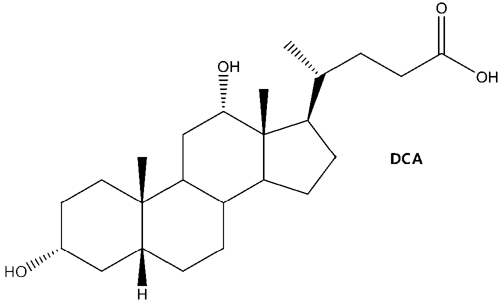 wherein all carbon atoms of the compounds and intermediates derive from plant sources.

In one embodiment, the deoxycholic acid or a pharmaceutically acceptable salt thereof comprises a fossil carbon percentage of less than 3 percent, such as less than 1 percent, like less than 0.1 percent, such as less than 0.01 percent, and a a mean δ¹³C value in the range from -20‰ to - 40‰.

In a further embodiment, the deoxycholic acid has a mean δ¹³C value in the range from -21‰ to -35‰, like from -20‰ to -32‰, such as from - 25‰ to -28‰, like around - 26‰.

In a further embodiment, the carbon chain of the compound of the general formula **Int A8** is elongated to DCA as described below:
i) converting the primary alcohol in the general formula **Int A8** into a leaving group (X) to obtain an intermediate of the general formula **Int A9:** 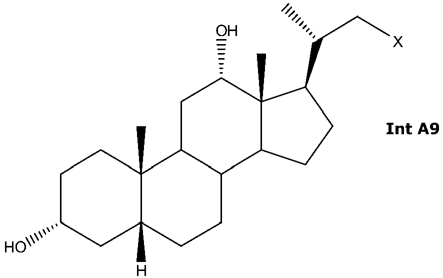 where X is OMs, OTs or halogen, preferably, Cl, Br or I; and
ii) elongating the carbon chain of the compound of the general formula **Int A9** to obtain **DCA:**

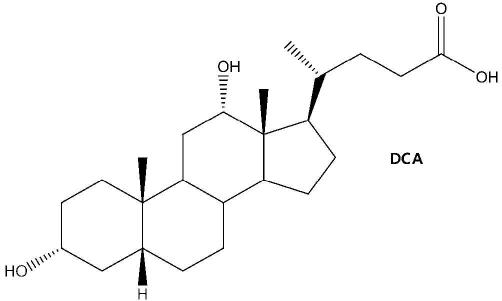

In a further embodiment, the compound of the general formula **Int A9** is converted into **Int A10,** which is then converted into **DCA.** 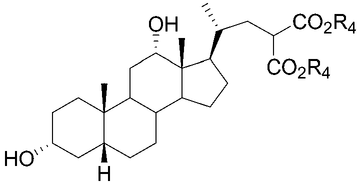 wherein R₄ is methyl or ethyl.

In an even further embodiment, **Int A10** is converted into **Int A11,** which is converted into **DCA.**

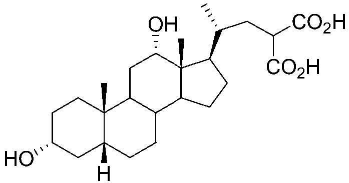

In a third aspect, the present invention relates to a compound of the general formula **Int A10** 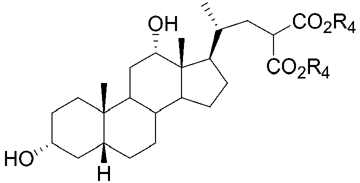 wherein R₄ is methyl or ethyl and wherein the carbon atoms of the compound are derived solely from plant sources.

In a fourth aspect, the present invention relates to a compound of the general formula **Int A11** 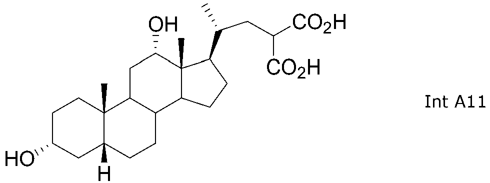 wherein the carbon atoms of the compound are derived solely from plant sources.

In a fifth aspect, the present invention relates to a deoxycholic acid mixture comprising a deoxycholic acid or a pharmaceutically acceptable salt thereof as described herein and deoxycholic acid or a pharmaceutically acceptable salt thereof obtained from an animal origin.

In a sixth aspect, the present invention relates to a process for the preparation of deoxycholic acid (DCA) or a pharmaceutically acceptable salt thereof, comprising the following steps:
i) providing a compound of the general formula **SM-a:** 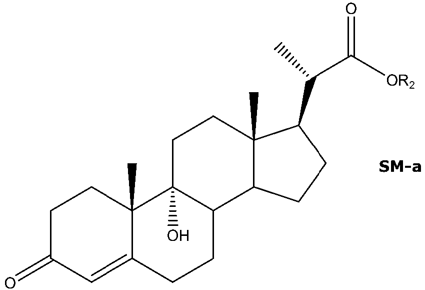
ii) reducing the compound of the general formula **SM-a** to obtain an intermediate of the general formula **Int A1:** 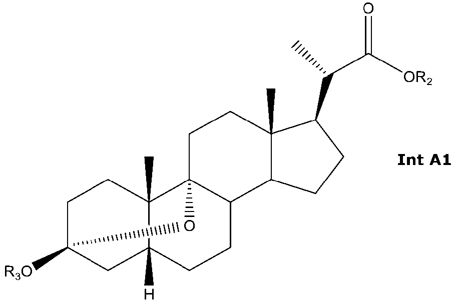
iii) converting the intermediate of the general formula **Int A1** into an intermediate of the general formula **Int A2:** 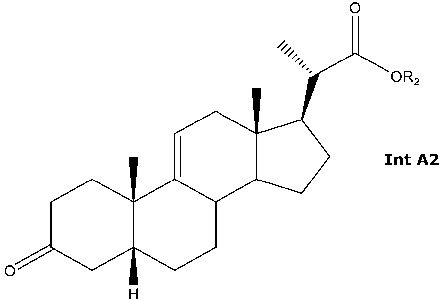
iv) reducing the intermediate of the general formula **Int A2** into an intermediate of the general formula **Int A3:** 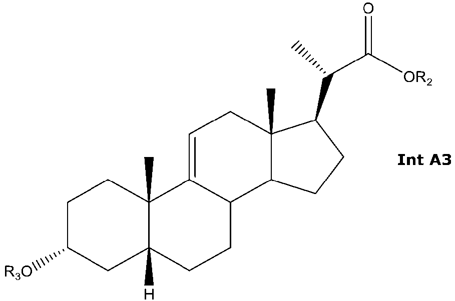
v) oxidising the intermediate of the general formula **Int A3** into an intermediate of the general formula **Int A5:** 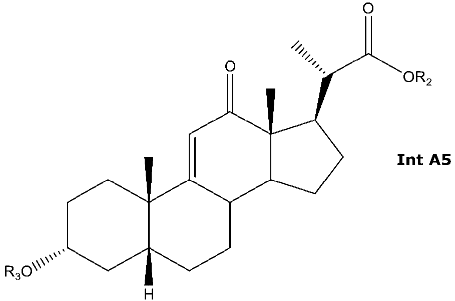
vi) reducing the intermediate of the general formula **Int A5** into an intermediate of the general formula **Int A6:** 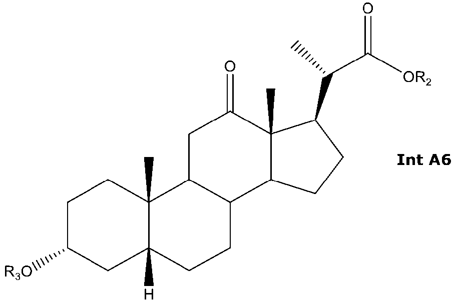
vii) reducing the intermediate of the general formula **Int A6** into an intermediate of the general formula **Int A7:** 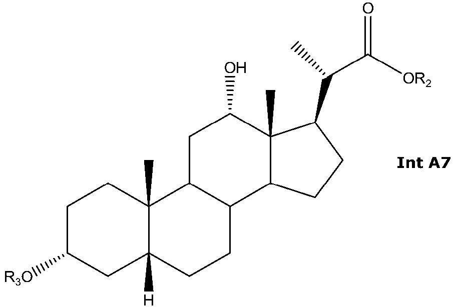
viii) reducing the compound of the general formula **Int A7** into an intermediate of the general formula **Int A8:** 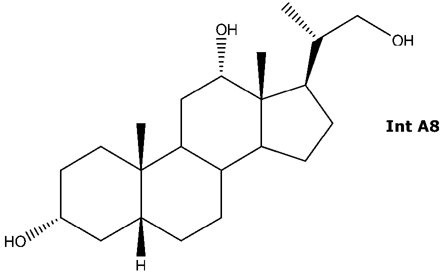
ix) converting the primary alcohol in the genral formula **Int A8** into a leaving group (X) to obtain an intermediate of the genral formula **Int A9:** 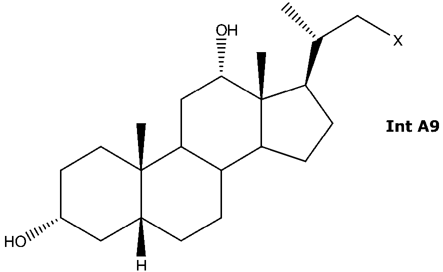 where X is OMs, OTs or halogen, preferably, Cl, Br or I;
x) converting the compound of the general formula **Int A9** into deoxycholic acid (DCA): 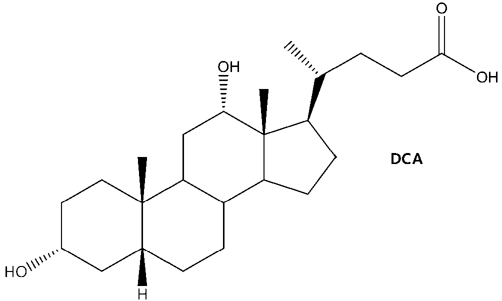
by means of route P1:
Pi) converting the compound of the general formula **Int A9** into the compound of the general formula **Int A10:** 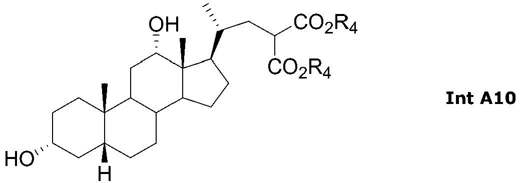
Pii) optionally, converting the compound of the general formula **Int A10** into the compound of the general formula **Int A11:** 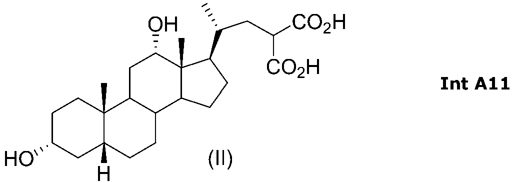
Piii) elongating the carbon chain of the compound of the general formula **Int A10** or **Int A11** to obtain deoxycholic acid (DCA)
or by means of route P2:
Pi') converting the general formula **Int A9** into the compound of general formula **A10b** 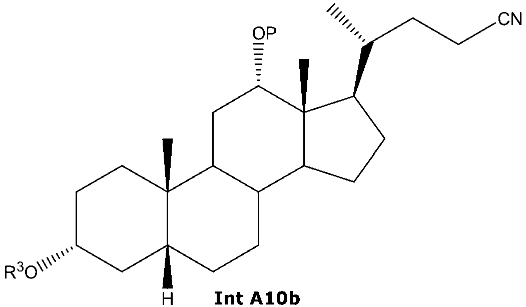
Pii') hydrolysing the coumpound of general formula **Int A10b** to obtain deoxycholic acid (DCA)
x) optionally converting deoxycholic acid to a pharmaceutically acceptable salt thereof,
wherein
R₂ is H or a linear or branched C₁-C₆-alkyl group;
R₃ is H, R₂ or an alcohol protection group;
P is an alcohol protection group;
R₄ is methyl or ethyl.

In an embodiment, the compound of the general formula **Int A9** is converted into the compound of the general formula **Int A10** using diethyl malonate, preferably obtained from plant sources such as sugar fermentation.

In a further embodiment, the acetonitrile is prepared from acetic acid, which is obtained from a plant source such as from a fermentation process.

In a still further embodiment, the deoxycholic acid is obtained by refluxing a reaction mixture of sodium chloride and the compound of the general formula **Int A11.**

In a still further embodiment, the deoxycholic acid is obtained by reacting the compound of the general formula **Int A10** with sodium hydroxide.

In a seventh aspect, the present invention relates to a compound of the general formula Int A10 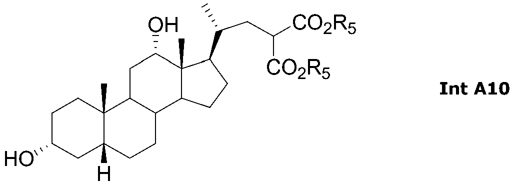
wherein R₅ is methyl or ethyl and
obtained by a method as described herein.

In an eight aspect, the present invention relates to a compound of the general formula Int A11 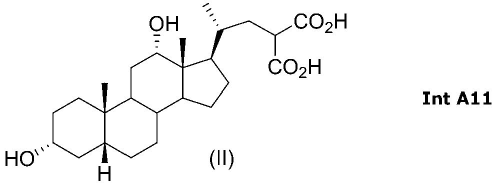 obtained by a method as described herein.

In a ninth aspect, the present invention relates to a compound of the general formula **Int A10b** 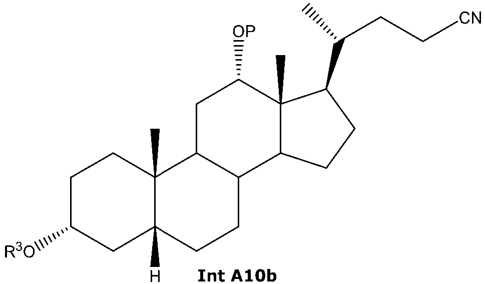
obtained by a method as described herein;
wherein
R₂ is H or a linear or branched C₁-C₆-alkyl group;
P is an alcohol protection group;
R₃ is H, R₂ or an alcohol protection group ₂.

### Detailed description of the invention

### Definitions

In the present context, the compound of the general formula I is to be understood as either a compound of formula Ia or Ib as shown below. 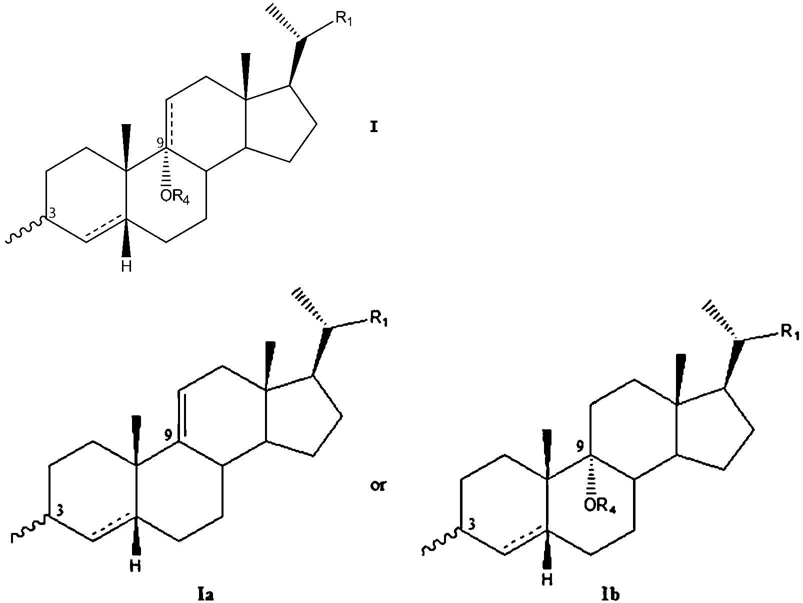 wherein
R₁ is COOR₂, CH₂OP, CH₂X, CH₂CHO, CH₂-CH₂-OH, CH₂-CH₂OP or CH₂-CH₂X ;
R₂ is H or a linear or branched C₁-C₆-alkyl group;
P is an alcohol protection group with the proviso that P is not Ac or Pv;
X is a halogen atom;
-̅ -̅ -̅ -̅ -̅ -̅ is either a C-C bond or a C=C bond;
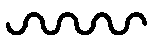 is either =O or 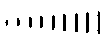 OR₃, where R₃ is either P or R₂;
OR₄ is either OH or R₄ is the C3 carbon in the A ring;

In the present context, the term "C₁-C₆-alkyl group" is intended to mean a linear or branched saturated carbon chain having from 1 to 6 carbon atoms. Specific examples of a C₁-C₆-alkyl group are methyl, ethyl, n-propyl, isopropyl, n-butyl, isobutyl, tert-butyl, n-pentyl, isopentyl, n-hexyl and iso-hexyl. Preferred examples include methyl, ethyl, n-propyl and isopropyl, in particular methyl and ethyl. Most preferably, the C₁-C₆-alkyl group is methyl.

Herein, the term "C₁-C₆-alkanol" means a linear or branched saturated alcohol having from 1 to 6 carbon atoms. Specific examples of a C₁-C₆-alkanol are methanol, ethanol, n-propanol, isopropanol, n-butanol, isobutanol, tert-butanol, n-pentanol, isopentanol, n-hexanol and iso-hexanol. Preferred examples includes methanol, ethanol, n-propanol and isopropanol, in particular methanol and ethanol. Most preferably, the C₁-C₆-alkanol is methanol.

The term "leaving group" is intended to mean a molecular fragment that is capable of departing from a molecule with a pair of electrons in heterolytic bond cleavage. Specific examples of leaving groups include halides, such a chloride, bromide and iodide, and sulfonate esters, such as tosylate. In a preferred embodiment of the invention the leaving group is bromide.

When used herein, the term "alcohol protection group" means a molecule that can modify, and hence temporarily mask the characteristic chemistry of, an alcohol group. Specific examples of alcohol protection groups include trimethylsilyl ether (TMS), triethylsilyl ether (TES), triisopropylsilyl ether (TIPS), tert-butyldimethylsilyl ether (TBS, TBDMS), tert-butyldiphenylsilyl ether (TBDPS), acetyl (Ac, COCH₃), benzoyl (Bz), benzyl ether (Bn), 4-methoxybenzyl ether (PMB), 2-naphthylmethyl ether (Nap), methoxymethyl acetal (MOM), 2-methoxyethoxymethyl ether (MEM), ethoxyethyl acetal (EE), methoxypropyl acetal (MOP), benzyloxymethyl acetal (BOM), tetrahydropyranyl acetal (THP), 2,2,2-trichloroethyl carbonate (Troc), methyl ether, dimethoxytrityl (DMT), methoxytrityl (MMT), methylthiomethyl ether, pivaloyl (Piv), tetrahydropyranyl (THP), triphenylmethyl (trityl, Tr), and tosyl (Ts) In a preferred embodiment of the invention the alcohol protection group is Ac, TBDMS and Ts, in particular Ac.

In the present context "Ac" means acetyl (COCH₃).

A "pharmaceutically acceptable salt" means that the salt is non-toxic and suitable for being administered to a mammal, in particular a human being. Examples of pharmaceutically acceptable salts include salts with a base, e.g. salts with an inorganic base, such as a sodium salt, a potassium salt, a calcium salt, a magnesium salt and the like, or salts with an organic base, such as a piperidine salt, a morpholine salt, a pyrrolidone salt, an arginine salt, a lysine salt and the like. In a preferred embodiment of the invention, the pharmaceutically acceptable salt is the sodium salt.

"fossil carbon percentage" means the percentage of carbon derived from "synthetic" (petrochemical) sources based on measurements of C14 according to ASTM D6866-16.

"biobased carbon percentage" means the percentage of carbon derived from "natural" sources such as plant or animal by-products/sources based on measurements of C14 according to ASTM D6866-16.

"δ¹³C value" means is an isotopic measurement of the delta notation of C13. δ¹³C values are expressed as a per mil (%) deviation, e.g. per one thousand, from an internationally accepted PDB standard (a carbonate from the Pee Dee Belemnite formation in South Carolina). δ¹³C values are determined using the following formula: ${δ}^{13} C = \frac{{\left({δ}^{13}C/{δ}^{12}C\right)}_{sample} - {\left({δ}^{13}C/{δ}^{12}C\right)}_{PDB}}{{\left({δ}^{13}C/{δ}^{12}C\right)}_{PDB}} \times 1000$

By "plant sources" are meant any source, which may be defined as a plant such as for example trees, shrubs, herbs, grasses, ferns, mosses, flowers, vegetables, weeds etc as well as compounds derived from plants such as phytosterols, and phytosterol derivatives etc.

By carbon atoms solely derived from plant sources are meant that all of the carbon atoms in the compound are derived from a plant source as defined above and not from a synthetic source or an animal source.

By carbon atoms partially derived from plant sources are meant that at least one of the carbon atoms in the compound are not derived from a plant source as defined above, but e.g. from a synthetic source or an animal source.

By "non-mammalian sources" are meant any source, which may be defined as not being a mammal.

By "C3 plants" are meant plants that does not have photosynthetic adaptations to reduce photorespiration. This includes plants such as rice, wheat, soybeans, most fruits, most vegetables and all trees.

By "C4 plants" are meant plants where the light-dependent reactions and the Calvin cycle are physically separated and where the light-dependent reactions occur in the mesophyll cells and the Calvin cycle occurs in bundle-sheath cells. This includes plants such as crabgrass, sugarcane, sorghum and corn.

Throughout this document, deoxycholic acid and DCA will be used interchangeably.

### The synthetic routes to DCA

The present inventors have provided new synthetic routes to DCA, which may be described by the following overall reaction scheme: 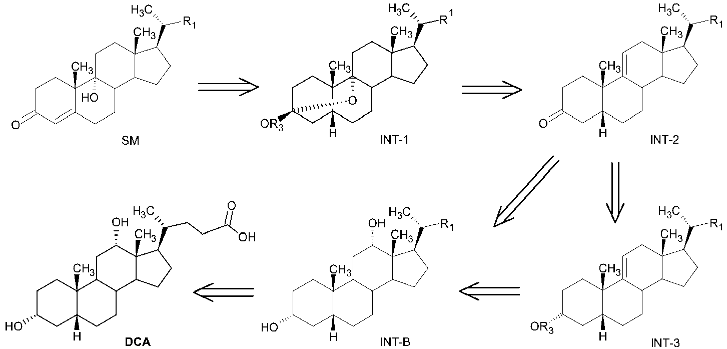 wherein
R₁ is COOR₂, CH₂OH, CH₂OP, CH₂X, CH₂CHO, CH₂-CH₂-OH, CH₂-CH₂OP, or CH₂-CH₂X or CH₂-CH₂-CHO;
R₂ is H or a linear or branched C₁-C₆-alkyl group;
P is an alcohol protection group;
R₃ either P or R₂; and
X is a halogen atom.

The individual process steps are disclosed in more detail *infra.*

### Synthetic route A

In a preferred embodiment of the invention, the process for the preparation of deoxycholic acid (DCA) or a pharmaceutically acceptable salt thereof, comprises the following steps:
i) providing a compound of the general formula **SM-a:** 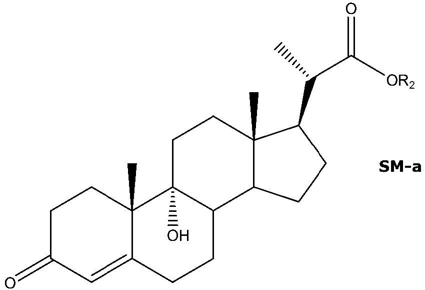
ii) reducing and optionally adding an alcohol protection group to the compound of the general formula **SM-a** to obtain an intermediate of the general formula **Int A1:** 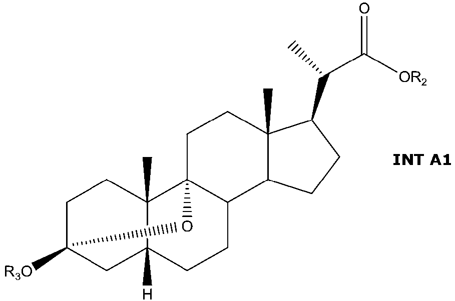
iii) converting the intermediate of the general formula **Int A1** into an intermediate of the general formula **Int A2:** 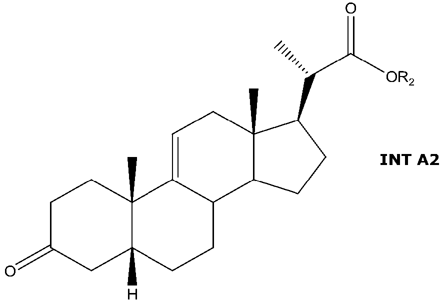
iv) reducing the intermediate of the general formula **Int A2** into an intermediate of the general formula **Int A3:** 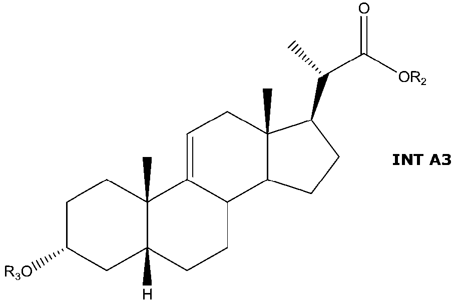
v) oxidising the intermediate of the general formula **Int A3** into an intermediate of the general formula **Int A5:** 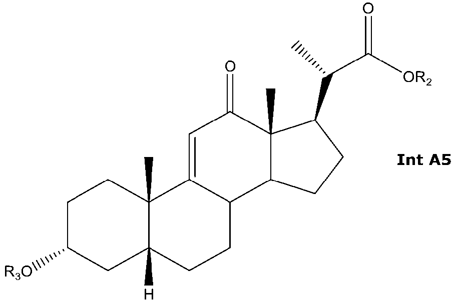
vi) reducing the intermediate of the general formula **Int A5** into an intermediate of the general formula **Int A6:** 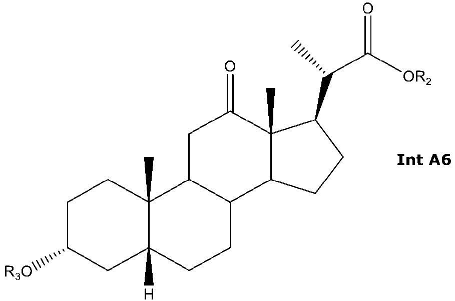
vii) reducing the intermediate of the general formula **Int A6** into an intermediate of the general formula **Int A7:** 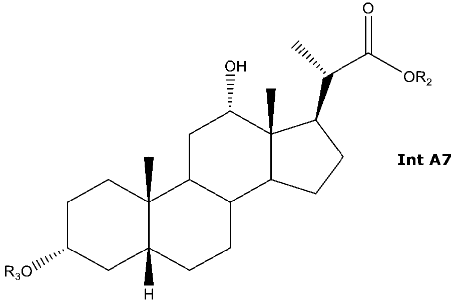
viii) reducing the compound of the general formula **Int A7** into an intermediate of the general formula **Int A8:** 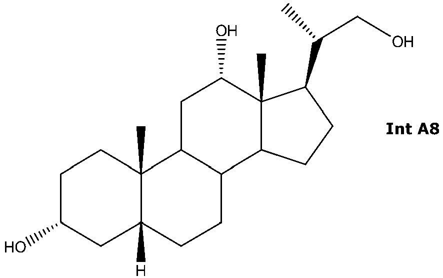
ix) elongating the carbon chain of the compound of the general formula **Int A8** to obtain deoxycholic acid (DCA): 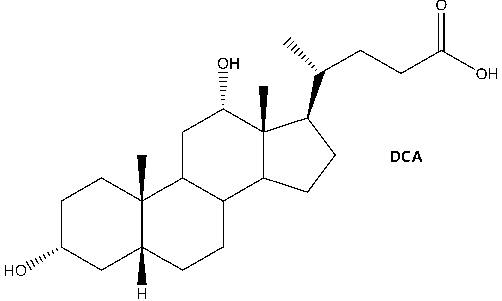
x) optionally converting deoxycholic acid to a pharmaceutically acceptable salt thereof,
wherein
R₂ is H or a linear or branched C₁-C₆-alkyl group;
R₃ is H, R₂ or an alcohol protection group.

### Step i)

The starting compound, intermediate **SM-a,** may be obtained from (or easily prepared from compounds obtained from) fermentation products of *Mycobacterium fortuitum* in the presence of an appropriate carbon source.

For example, US 4,029,549 shows the production of 9α-OH BN acid, 9α-OH BN alcohol and 9α-OH BN methyl ester by fermenting the microorganism *Mycobacterium fortuitum* NRRL B-8119 in the presence of either sitosterol (example 2) or cholesterol, stigmasterol or campesterol (example 3). The purification and isolation of 9α-OH BN acid is disclosed in example 5 of US 4,029,549.

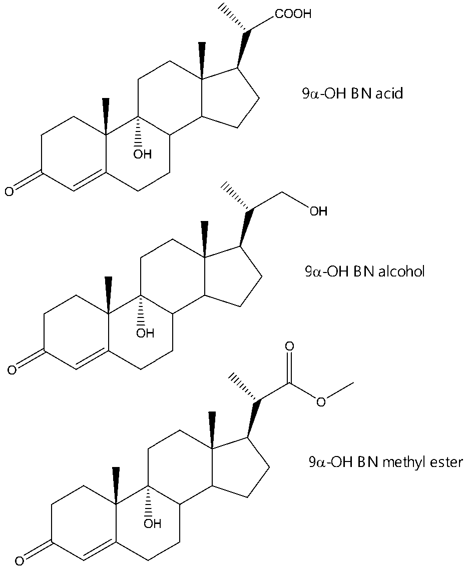

Accordingly, steps i) to vii) described in the "Synthetic route A" may be preceded by a step comprising cultivating a 9α-OH BN acid-producing microorganism in an aqueous nutrient medium under aerobic conditions in the presence of a carbon source. This applies *mutatis mutandis* to the other synthetic routes described herein, including "Synthetic route C", "Synthetic route D", "Synthetic route E" and "Synthetic route F".

The 9α-OH BN acid-producing microorganism may be selected from the group consisting of *Arthrobacter, Bacillus, Brevibacterium, Corynebacterium, Microbacterium, Nocardia, Proaminobacter, Serratia, Streptomyces* and *Mycobacterium.* In a preferred embodiment of the invention the 9α-OH BN acid-producing microorganism is *Mycobacterium,* in particular *Mycobacterium fortuitum.* In the most preferred embodiment of the invention the 9α-OH BN acid-producing microorganism is *Mycobacterium fortuitum* NRRL B-8119.

The carbon source may be a steroid, such as cholesterol, stigmasterol, campesterol and sitosterol or mixtures of all of them, preferably sitosterol.

Preferably, the carbon source may be a plant phytosterol such as sitosterol, stigmasterol, campesterol and brassicasterol or mixtures. In one embodiment, the phytosterols are mainly of soybean origin.

As will be understood, 9α-OH BN acid, 9α-OH BN alcohol and 9α-OH BN methyl ester may, if needed, easily be converted into compounds of the general formula **SM-a** by standard methods well known to the person skilled in organic chemistry.

### Step ii)

Step ii) involves reducing the compound of the general formula **SM-a** to obtain an intermediate of the general formula **Int A1.**

The reaction is typically carried out by hydrogenation of **SM-a** in the presence of palladium on charcoal (Pd/C) at a temperature of 50-90°C, preferably around 70°C, for 1-24 hours, preferably 8-16 hours. Other transition metal catalysts may also be employed, such as Ni or Rh.

If R₃ is H, the reaction is preferably carried out in a polar aprotic solvent, such as N-methylpyrrolidone, tetrahydrofuran (THF), ethylacetate (EtOAc), acetone, dimethylformamide (DMF), acetonitrile or dimethyl sulfoxide (DMSO). In a preferred embodiment the polar aprotic solvent is DMF.

If R₃ is a C₁-C₆-alkyl group the reaction is carried out in the corresponding alcohol, i.e. the solvent is an C₁-C₆-alkanol. In a preferred embodiment of the invention R₃ is methyl and the solvent is methanol.

### Step iii)

Step iii) involves converting the intermediate of the general formula **Int A1** to obtain an intermediate of the general formula **Int A2.**

The skilled person will be aware of suitable oxidising agents, and examples include chromium oxide (CrO₃) and strong acids, such as HI, HBr, HClO₄, HCl, HClO₃, H₂SO₄, HNO₃, preferably HCl or H₂SO₄, in particular H₂SO₄. The reaction is typically carried out in a non-polar solvent, such as dichloromethane (DCM), at a temperature between 0 and 90°C.

### Step iv)

Step iv) involves reducing the intermediate of the general formula **Int A2** to obtain an intermediate of the general formula **Int A3.**

The skilled person will be aware of suitable reducing agents capable of reducing a ketone to a secondary alcohol. Preferably, the reducing agent is a metal hydride, such as LiAlH₄, NaBH₄, LiBH₄ or LiAlH(OtBu)₃, in particular LiAlH(OtBu)₃.

The reaction is typically carried out in a polar aprotic solvent, such as N-methylpyrrolidone, tetrahydrofuran (THF), ethylacetate (EtOAc), acetone, dimethylformamide (DMF), acetonitrile or dimethyl sulfoxide (DMSO), in particular THF, at a temperature between 0 to 20°C.

### Step v)

Step v) involves oxidising the intermediate of the general formula **Int A3** to obtain an intermediate of the general formula **Int A5.**

The skilled person will be aware of suitable oxidising agents for performing an allylic oxidation, and a preferred example include chromium oxide (CrO₃). Other suitable oxidising agents include tert-butyl hydroperoxide (t-BuO₂H), NaOCl, SeO₂, pyridinium chlorochromate (PCC), BiCl₃ and V₂O₅. The reaction is typically carried out in a polar solvent, such as AcOH, at a temperature between 0 and 90°C.

### Step vi)

Step ii) involves reducing the intermediate of the general formula **Int A5** to obtain an intermediate of the general formula **Int A6.**

The reaction is typically carried out by hydrogenation of **Int A5** in the presence of palladium on charcoal (Pd/C) at a temperature of 50-90°C, preferably around 70°C, for 1-24 hours, preferably 8-16 hours. Other transition metal catalysts may also be employed, such as Ni or Rh.

The reaction is preferably carried out in a polar aprotic solvent, such as N-methylpyrrolidone, tetrahydrofuran (THF), ethylacetate (EtOAc), acetone, dimethylformamide (DMF), acetonitrile or dimethyl sulfoxide (DMSO). In a preferred embodiment the polar aprotic solvent is EtOAc.

### Step viii)

Step viii) involves reducing the intermediate of the general formula **Int A7** to obtain an intermediate of the general formula **Int A8.**

The skilled person will be aware of suitable reducing agents capable of reducing a carboxylic acid or an ester thereof to a primary alcohol. Preferably, the reducing agent is a metal hydride, such as LiAlH₄, NaBH₄, LiBH₄ or LiAlH(OtBu)₃, in particular LiAlH₄.

The reaction is typically carried out in a polar aprotic solvent, such as N-methylpyrrolidone, tetrahydrofuran (THF), ethylacetate (EtOAc), acetone, dimethylformamide (DMF), acetonitrile or dimethyl sulfoxide (DMSO), in particular THF, at a temperature between 0 to 50°C.

It should be noted that it is possible to elongate the carbon chain of the intermediate of the general formul **Int A7** directly to obtain an intermediate of the general formula **Int B2** in a similar way as described in step ix) *infra.* This may be done by a "Reformatsky reaction", i.e. by reacting **Int A7** with Br-CH₂-COOR₂ in the presence of Zn in a suitable solvent.

### Step ix)

Step ix) involves elongating the carbon chain of the compound of the general formula **Int A8** to obtain **DCA.**

Different synthetic routes are possible for elongating the carbon chain of **Int A8** to obtain **DCA:**
One possible route for elongating the carbon chain of **Int A8** to obtain **DCA** comprises the steps ix-a) and ix-b):
ix-a) converting the primary alcohol in the general formula **Int A8** to obtain an intermediate of the general formula **Int A9:** 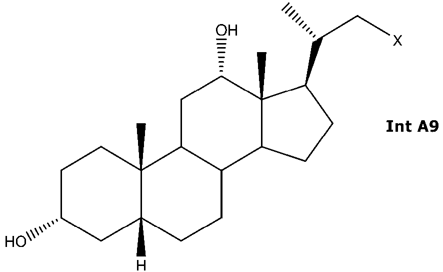
where X is OMs, OTs or halogen, preferably, Cl, Br or I, in particular Br,
optionally acylating **Int A9** with a dicarboxylic acid or an dicarboxylic acid derivative to obtain **Int A9a.** 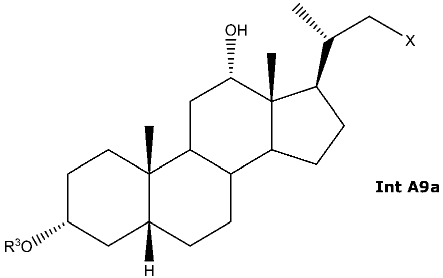
where R₃ is -CO(CH₂)ₙCOOH with n being an integer from 0 to 11 included. In a preferred embodiment n = 2 such that the dicarboxylic acid is succinic acid. Acylation of the alcohol in **Int A9** can be achieved in a number of ways. For example **Int A9** may be reacted with an acyl halide, an anhydride, an ester or condensed with a free carboxylic acid. Alternative **Int A9** may be coupled with the carboxylic acid using as suitable coupling reagent known in the art such as DCC, DIC, EDAC·HCl, HATU, TBTU, BOP, PyBOP. The coupling may be performed in the presence of base.

Optionally, the R₃ group may be further protected by e.g. an alcohol protection group or R₃ may be an alcohol protection group in order to obtain a proper elongation reaction of Int A9, Int A9a or Int A9b to obtain DCA as known by the skilled person in organic chemistry.

Optionally **Int A9a** may further be protected to obtain **Int A9b.** 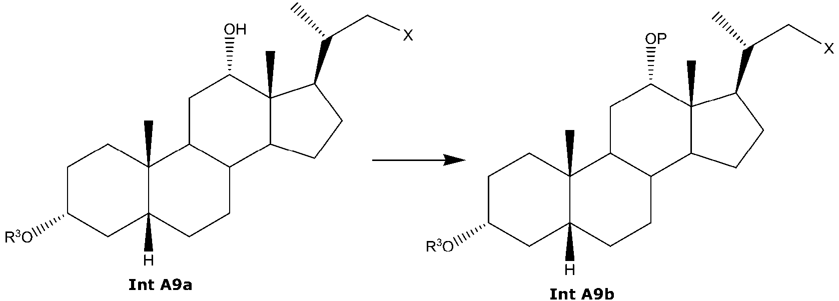 wherein P is an alcohol protecting group as previously defined.

ix-b) elongating the carbon chain of the compound of the general formula **Int A9, Int A9a or Int A9b** to obtain **DCA:**

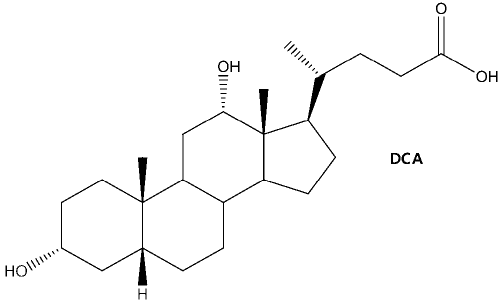

Conversion of the primary alcohol in the general formula **Int A8** into a leaving group (X) may be by means of halogenation. Halogenation of primary alcohols is well known to the person skilled in organic chemistry, and may be achieved in various ways. For example, the compound of the general formula **Int A8** may be treated with HX, where X is Cl, Br or I, preferably HBr. Alternatively, the compound of the general formula **Int A8** may be treated with CX₄ and triphenylphosphine (PPh₃), where X is Cl, Br or I, preferably Br. In a preferred embodiment of the invention **Int A9** is obtained by treating **Int A8** with CBr₄ and PPh₃.

Alternatively, the conversion of the primary alcohol in the general formula of **Int A8** into a leaving group (X) may be by means known to the person skilled in organic chemistry such as mesylating or tosylating.

Elongation of the carbon chain of **Int A9, Int A9a or Int A9b** to obtain **DCA** may be carried out using the so-called "Malonic ester synthesis" (see Morrison and Boyd, Organic Chemistry, 5th edition, 1987, pp. 1060-1063). In an embodiment of the invention **Int A9, Int A9a or Int A9b** is treated with a malonate ester, preferably diethyl malonate, in the presence of a base, preferably NaH, and subsequently acidified to obtain DCA. The malonate ester is preferably obtained from natural origin such as e.g. from the esterification of malonic acid obtained from sugar fermentation. Hereby, DCA may be obtained where all of the carbon atoms are of natural origin and solely from plant sources.

As an example, **Int A10** may be prepared by esterification with diethyl malonate.

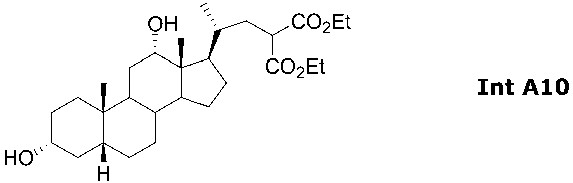

Alternatively, **Int A10** may be prepared by esterification with dimethyl malonate resulting in esterification with methyl (Me) instead of ethyl (Et).

**Int A10** may further be converted to **Int A11** under conditions being effective to produce **Int A11** and being known to the person skilled in organic chemistry for example by reacting **Int A10** with sodium hydroxide.

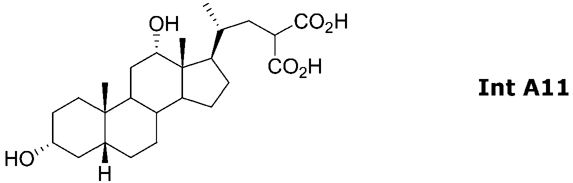

In one embodiment, the elongation of the carbon chain of **Int A9, IntA9a or Int A9b** to obtain **DCA** is defined as route P1 with the step Pi) for converting the compound of general formula **Int A9, Int A9a or Int A9b to Int A10,** the optional step Pii) for converting the compound of general formula **Int A10 to Int A11** and step Piii) for converting either the compound of general formula **Int A10 or Int A11 to DCA.**

In one embodiment the compound of formula Int A10 is derived solely from plant sources. In another embodiment, the compound og formula Int A11 is obtained solely from plant sources.

Both **Int A10** and **Int A11** may be converted into DCA. The conversion of **Int A11** into DCA may be performed by various methods as known to the person skilled in the art for example by refluxing a reaction mixture of sodium chloride with **Int A11**

Other methods for elongation of the carbon chain in **Int A9b** to obtain **DCA** may be used. In an embodiment the leaving group X in **Int A9b** may be displaced with acetonitrile (ACN) in the presense of base to obtain **Int A10b.** In another embodiment, the starting compound may be **Int A9 or Int A9a** instead of **Int A9b.**

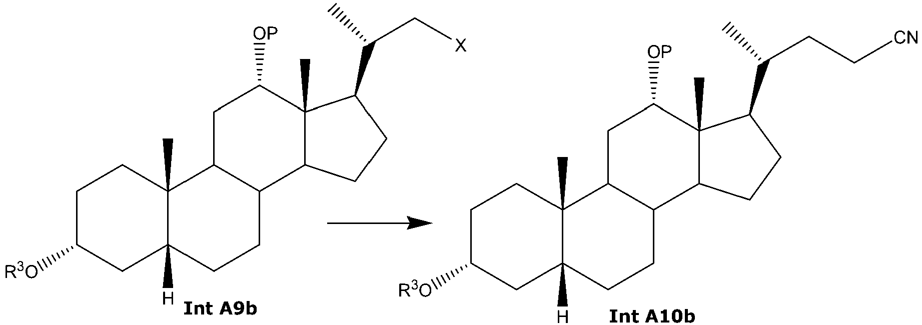

ACN (pKa = 25) can be fairly easy deprotonated due to inductive and resonance stabilization of the resulting anion by the neighbouring nitrile. Suitable bases include but are not limited to NaH, KtOBu or NaNH₂. Acetonitrile may be obtained from acetic acid as described in the Bulletin of the Tomsk Polytechnic University. 2007, vol. 310, no. 1, p. 145-148.

Alternatively, acetonitrile may be obtained as described in US 3,161,669, where a reactor was packed with 10 cubic feet of Harshaw Alumina 0104 pellets (1/4-inch size) saturated with an aqueous solution of 25 weight percent phosphoric acid. Ammonia and acetic acid were passed at a temperature of 450°C into the reaction chamber at a mole ratio of ammonia to acetic acid of 1.13:1. Contact time was 4.5 seconds. 5700 pounds of acetic acid and 1820 pounds of ammonia were fed per day. The production of acetonitrile was 3700 pounds per day or a 95% yield based on acetic acid. Reaction gases leaving the reactor were condensed in a water quench scrubber. From the scrubber 3700 pounds of acetonitrile and 4000 pounds of water were fed to a steam stripper and 3400 pounds of water was removed from the base of the stripper. The overhead drawn from the stripper at 79°C contained 3700 pounds acetonitrile and 600 pounds water. This overhead material was fed to a drying column where 600 pounds per day of water was removed by azeotropic distillation using benzene as a separating agent. The dry acetonitrile bottoms from the drying column were fed to a distillation column where low boiling point impurities were removed overhead. Acetonitrile bottoms from this column were fed to a final tripping column from which the final purified acetonitrile product was withdrawn overhead. The purified acetonitrile had an ASTM boiling range of 81.3-83.1°C, APHA color of 10, water content of 0.016 weight percent, no trace of carbonyl or benzene and total impurities as measured by chromatographic analysis of less than 0.1 weight percent.

Acetic acid is easily obtained from natural fermentation and it can be considered as a natural source such as a plant source. In one embodiment, the acetic acid may be obtained from acetone. The acetone may for example be obtained from bacterial fermentation of glucose or starch using a strain of bacteria from the class Clostridia: Clostridium acetobutylicum as e.g. described by Mark R. Wilkins and Hasan Atiye (2012): "Fermentation" (In Nurhan Turgut Dunford. Food and Industrial Bioproducts and Bioprocessing) by means of an oxidation reaction with potassium dichromate and sulphuric acid to produce acetic acid.

The nitrile in **Int A10b** may subsequently be hydrolysed into the free carboxylic acid with concomitant or subsequent removal of the alcohol protecting group P to obtain DCA.

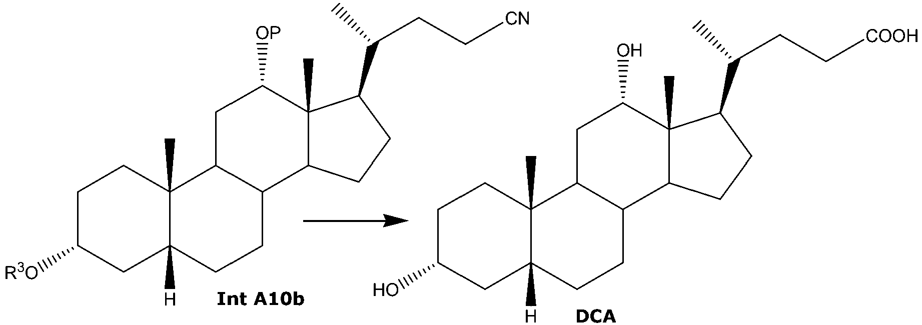

In one embodiment, the elongation of the carbon chain of **Int A9** to obtain **DCA** is defined as route P2 with the step Pi') for converting the compound of general formula **Int A9** to **Int A10b** and the step Pii') for converting the compound of general formula **Int A10b** to **DCA.**

Other equivalents for elongation of the carbon chain in **Int A9b** to obtain **DCA** include displacement of the the leaving group X in **Int A9b** with enolates formed from acetate esters (CH₃COO-R) followed by hydrolysis. Any enolate derived from acetic acid may be used such as enolates of acetates and ethyl amides. The acetic acid is preferably obtained from a fermentation process i.e. the acetic acid is of natural origin from a plant source. Hereby, DCA may be obtained where all of the carbon atoms are of natural origin and solely from plant sources.

Another possible route for elongating the carbon chain of **Int A8** to obtain **DCA** comprises the steps ix-c) to ix-e):
ix-c) oxidising the compound of the general formula **Int A8** to obtain an intermediate of the general formula **Int B1:** 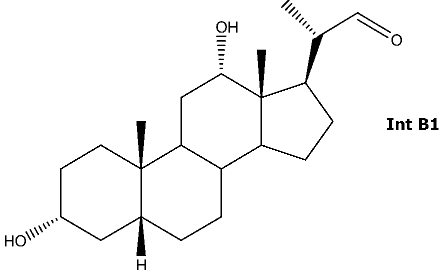
ix-d) elongating the carbon chain of the compound of the general formula **Int B1** to obtain an intermediate of the general formula **Int B2:** 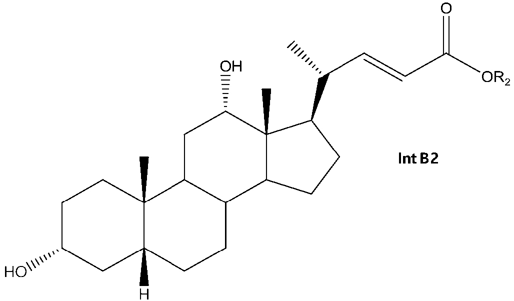 where R₂ is a linear or branched C₁-C₆-alkyl group,
ix-e) converting the compound of the general formula **Int B2** into DCA.

With respect to step ix-c), oxidation of primary alcohols into aldehydes is well known to the person skilled in organic chemistry, and may be achieved in various ways. For example by chromium-based reagents, such as Collins reagent, PDC or PCC, or by catalytic TEMPO in presence of NaOCl.

Elongation of the carbon chain of **Int B1** to **Int B2** (step ix-d)) may be carried out using the so-called "Wittig reaction" (see Morrison and Boyd, Organic Chemistry, 5th edition, 1987, pp. 920-921). Alternatively, the carbon elongation step may be performed by "Horner-Emmons olefination", by "Peterson olefination", or by a "Reformatsky reaction", i.e. by reacting **Int B1** with Br-CH₂-COOR₂ in the presence of Zn in a suitable solvent.

Conversion of **Int B2** to **DCA** (step ix-e)) may be performed by hydrogenation of **Int B2** followed by alkaline hydrolysis, or *vice versa.*

### Step x)

The optional step x) involves converting **DCA** into a pharmaceutically acceptable salt of DCA.

Examples of pharmaceutically acceptable salts include salts with a base, e.g. salts with an inorganic base, such as a sodium salt, a potassium salt, a calcium salt, a magnesium salt and the like, or salts with an organic base, such as a piperidine salt, a morpholine salt, a pyrrolidoine salt, an arginine salt, a lysine salt and the like. In a preferred embodiment of the invention, the pharmaceutically acceptable salt is the sodium salt.

In a preferred embodiment of the invention the sodium salt of DCA is obtained by reacting DCA with NaOH.

### Synthetic route A'

In another preferred embodiment of the invention, the process for the preparation of deoxycholic acid (DCA) or a pharmaceutically acceptable salt thereof, comprises the following steps:
i) providing a compound of the general formula 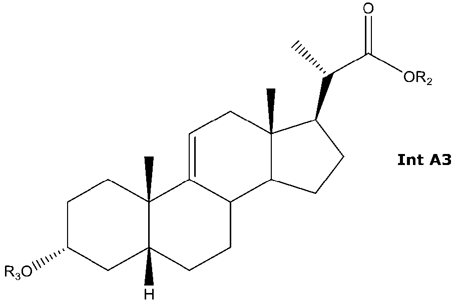
ii) oxidising the intermediate of the general formula **Int A3** into an intermediate of the general formula **Int A5:** 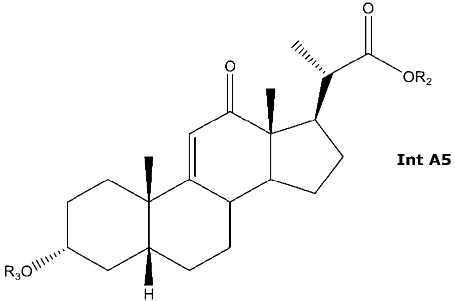
iii) reducing the intermediate of the general formula **Int A5** into an intermediate of the general formula **Int A6:** 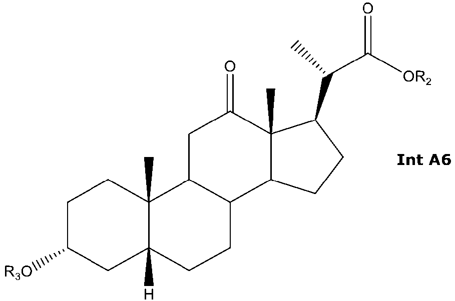
iv) reducing the intermediate of the general formula **Int A6** into an intermediate of the general formula **Int A7:** 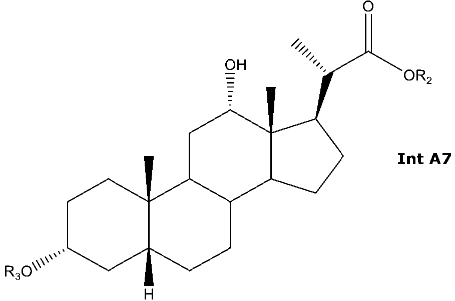
v) reducing the compound of the general formula **Int A7** into an intermediate of the general formula **Int A8:** 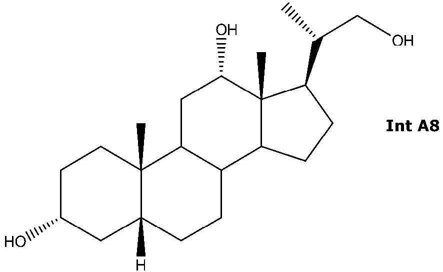
vi) elongating the carbon chain of the compound of the general formula **Int A8** to obtain deoxycholic acid (DCA): 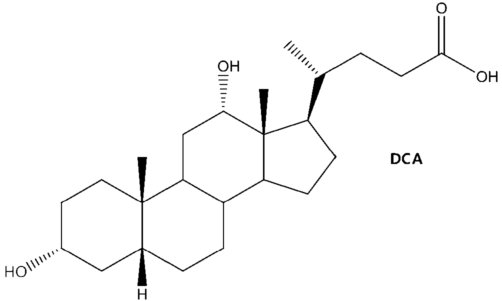
vii) optionally converting deoxycholic acid to a pharmaceutically acceptable salt thereof,
wherein
R₂ is H or a linear or branched C₁-C₆-alkyl group;
R₃ is H, R₂ or an alcohol protection group.

Steps ii) to vii) above corresponds exactly to steps v) to x) discussed in connection with "Synthetic Route A". The comments provided for steps v) to x) in connection with "Synthetic Route A" therefore apply *mutatis mutandis* to steps ii) to vii) of the "Synthetic Route A‴.

### Synthetic route C

In another interesting embodiment of the invention, the process for the preparation of deoxycholic acid (DCA) or a pharmaceutically acceptable salt thereof, comprises the following steps:
i) providing a compound of the general formula **SM-a:** 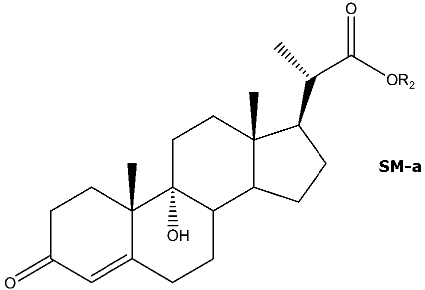
ii) reducing the compound of the general formula **SM-a** to obtain an intermediate of the general formula **Int A1:** 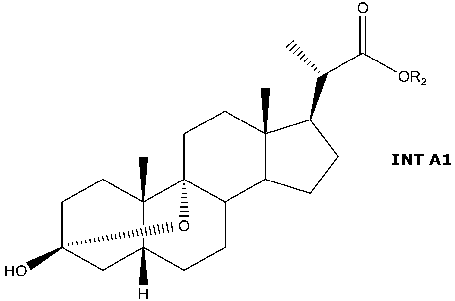
iii) converting the intermediate of the general formula **Int A1** into an intermediate of the general formula **Int A2:** 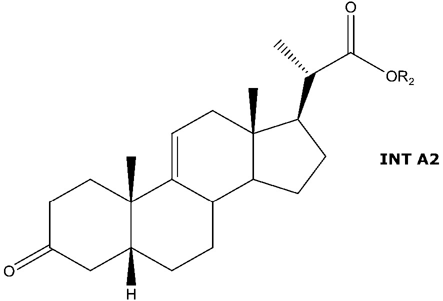
iv) oxidising the intermediate of the general formula **Int 2A** into an intermediate of the general formula **Int C1:** 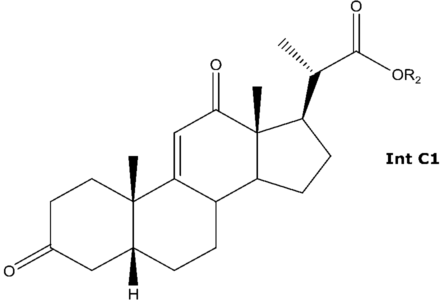
v) reducing the intermediate of the general formula **Int C1** into an intermediate of the general formula **Int C4:** 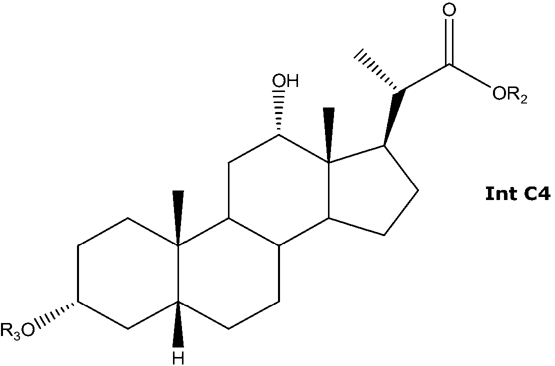
vi) reducing the compound of the general formula **Int C4** into an intermediate of the general formula **Int A8:** 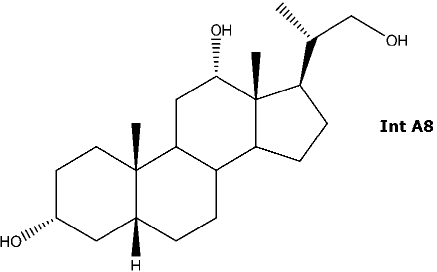
vii) elongating the carbon chain of the compound of the general formula **Int A8** to obtain deoxycholic acid (DCA): 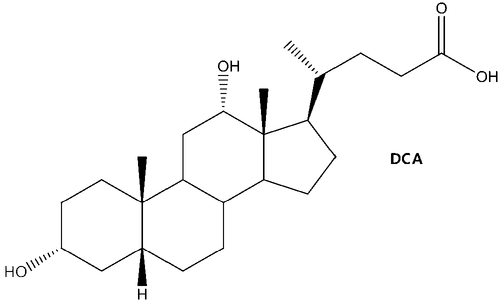
viii) optionally converting deoxycholic acid to a pharmaceutically acceptable salt thereof,
   wherein
   R₂ is H or a linear or branched C₁-C₆-alkyl group;
   R₃ is H, R₂ or an alcohol protection group.
x) optionally converting deoxycholic acid to a pharmaceutically acceptable salt thereof,
wherein
R₂ is H or a linear or branched C₁-C₆-alkyl group.

### Synthetic route D

In still another interesting embodiment of the invention, the process for the preparation of deoxycholic acid (DCA) or a pharmaceutically acceptable salt thereof, comprises the following steps:
i) providing a compound of the general formula **SM-a:** 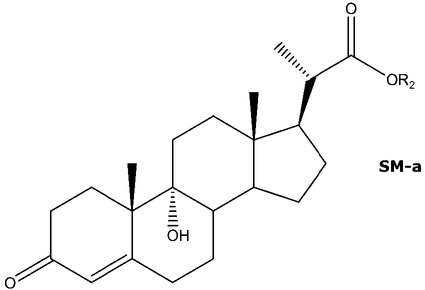
ii) reducing the compound of the general formula **SM-a** to obtain an intermediate of the general formula **Int A1:** 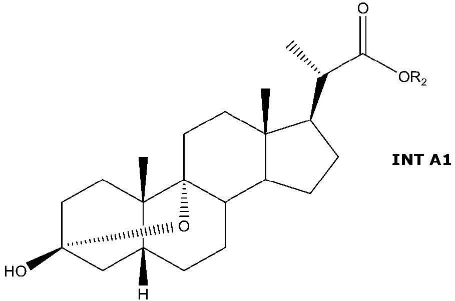
iii) converting the intermediate of the general formula **A1** into an intermediate of the general formula **D1:** 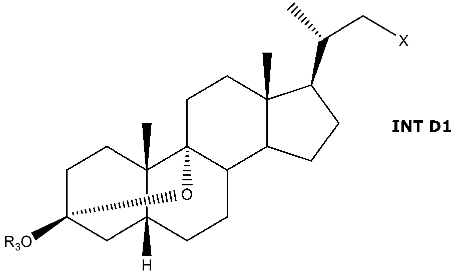
iv) oxidising the intermediate of the general formula **Int D1** into an intermediate of the general formula **Int D2:** 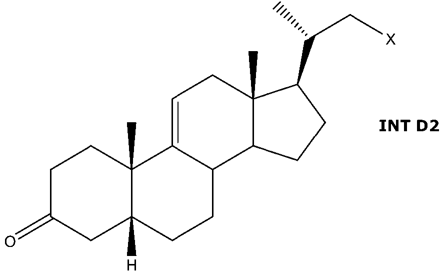
v) reducing the intermediate of the general formula **Int D2** into an intermediate of the general formula **Int D3:** 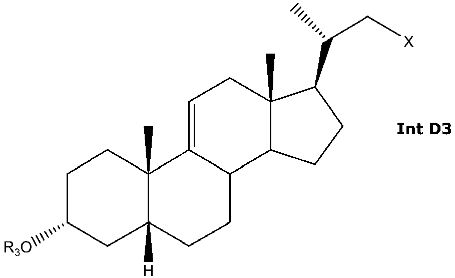
vi) oxidising the intermediate of the general formula **Int D3** into an intermediate of the general formula **Int D5:** 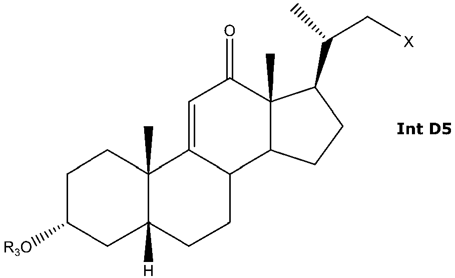
vii) reducing the intermediate of the general formula **Int D5** into an intermediate of the general formula **Int D6:** 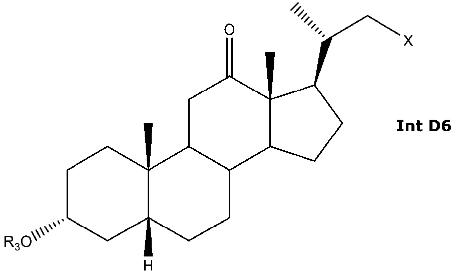
viii) reducing the intermediate of the general formula **Int D6** into an intermediate of the general formula **Int D7:** 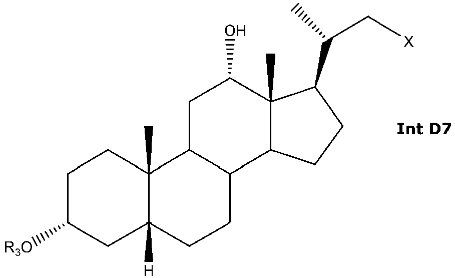
ix) hydrolysing the compound of the general formula **Int D7** into an intermediate of the general formula **Int D9:** 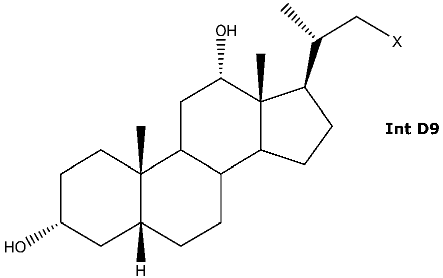
x) elongating the carbon chain of the compound of the general formula **Int D9** to obtain an deoxycholic acid (DCA): 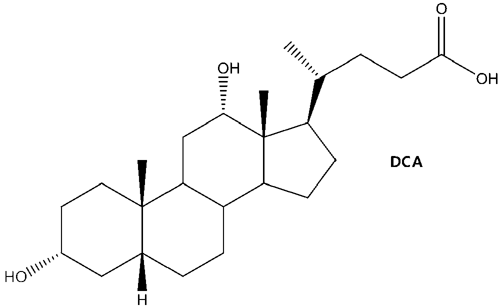
xi) optionally converting deoxycholic acid to a pharmaceutically acceptable salt thereof,
wherein
R₂ is H or a linear or branched C₁-C₆-alkyl group;
R₃ is H, R₂ or an alcohol protection group;
and X is a halogen atom.

### Synthetic route D'

In another interesting embodiment of the invention, the process for the preparation of deoxycholic acid (DCA) or a pharmaceutically acceptable salt thereof, comprises the following steps:
i) providing a compound of the general formula **Int D3:** 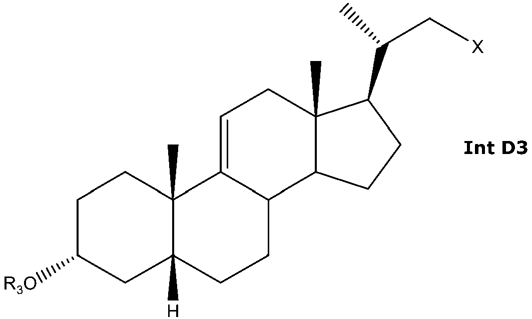
ii) oxidising the intermediate of the general formula **Int D3** into an intermediate of the general formula **Int D5:** 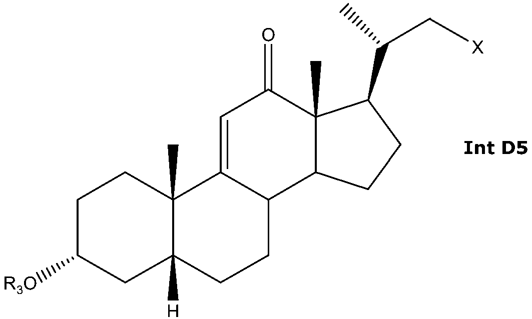
iii) reducing the intermediate of the general formula **Int D5** into an intermediate of the general formula **Int D6:** 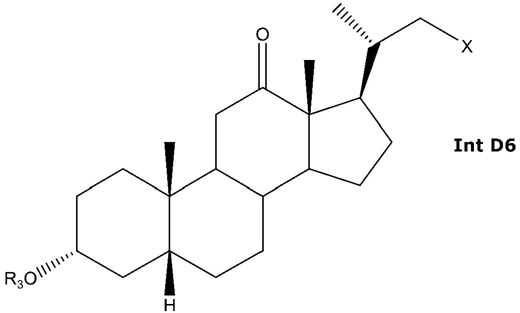
iv) reducing the intermediate of the general formula **Int D6** into an intermediate of the general formula **Int D7:** 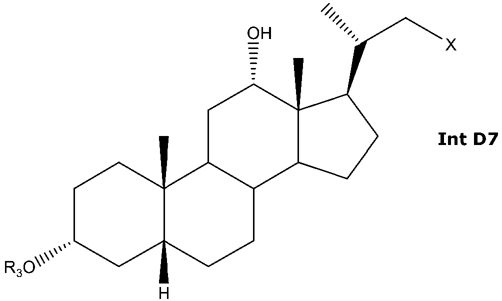
v) hydrolysing the compound of the general formula **Int D7** into an intermediate of the general formula **Int D9:** 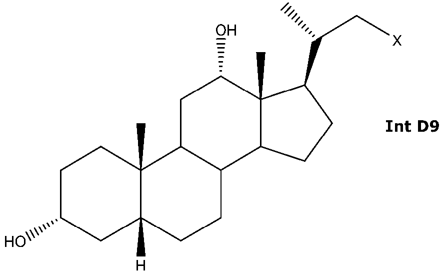
vi) elongating the carbon chain of the compound of the general formula **Int D9** to obtain an deoxycholic acid (DCA): 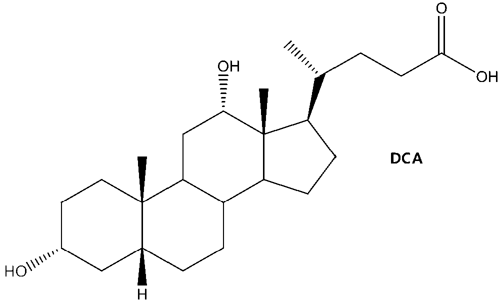
vii) optionally converting deoxycholic acid to a pharmaceutically acceptable salt thereof,
wherein
R₂ is H or a linear or branched C₁-C₆-alkyl group;
R₃ is H, R₂ or an alcohol protection group;
and X is a halogen atom.

### Synthetic route E

In yet another interesting embodiment of the invention, the process for the preparation of deoxycholic acid (DCA) or a pharmaceutically acceptable salt thereof, comprises the following steps:
i) providing a compound of the general formula **SM-a:** 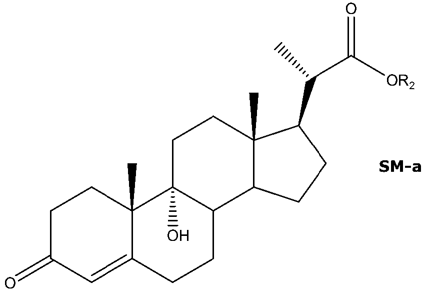
ii) reducing the compound of the general formula **SM-a** to obtain an intermediate of the general formula **SM-b:** 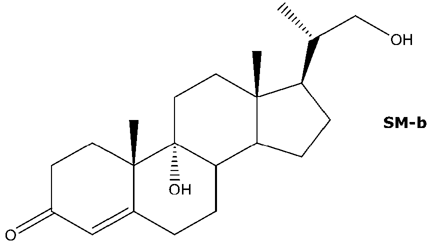
iii) protecting the alcohol group at position 22 to obtain an intermediate of the general formula **Int E1:** 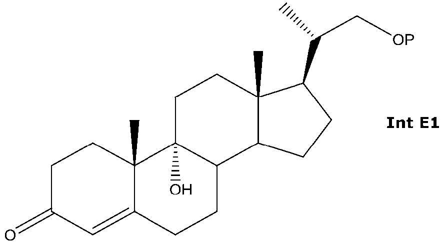
iv) converting the compound of the general formula **Int E1** to obtain an intermediate of the general formula **Int E2:** 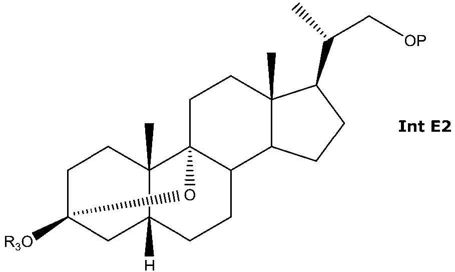
v) dehydration of the intermediate of the general formula **Int E2** into an intermediate of the general formula **Int E3:** 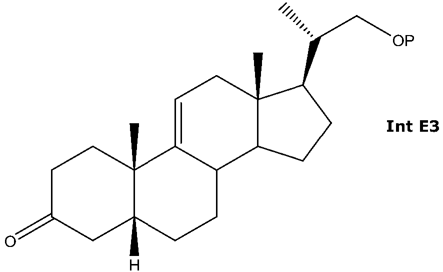
vi) reducing the intermediate of the general formula **Int E3** into an intermediate of the general formula **Int E5:** 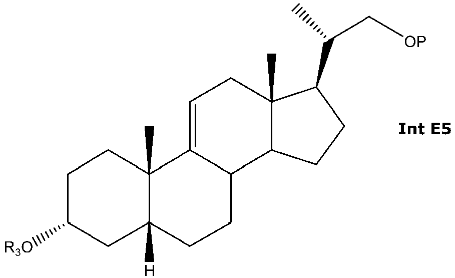
vii) oxidising the intermediate of the general formula **Int E5** into an intermediate of the general formula **Int E6:** 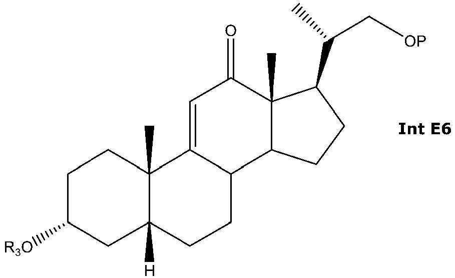
viii) reducing the intermediate of the general formula **Int E6** into an intermediate of the general formula **Int E7:** 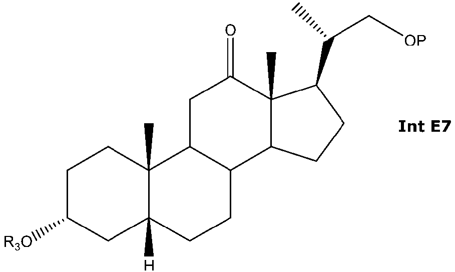
ix) reducing the intermediate of the general formula **Int E7** into an intermediate of the general formula **Int E9** 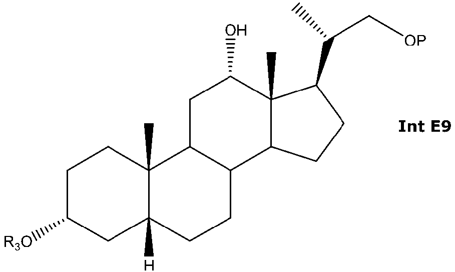
x) elongating the carbon chain of the compound of the general formula **Int E9** to obtain an deoxycholic acid (DCA): 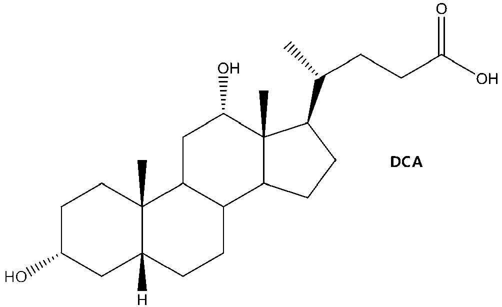
x) optionally converting deoxycholic acid to a pharmaceutically acceptable salt thereof,
wherein
R₂ is H or a linear or branched C₁-C₆-alkyl group;
R₃ is H, R₂ or an alcohol protection group; and
P is an alcohol protection group.

### Synthetic route E'

In another interesting embodiment of the invention, the process for the preparation of deoxycholic acid (DCA) or a pharmaceutically acceptable salt thereof, comprises the following steps:
i) providing a compound of the general formula **Int E3:** 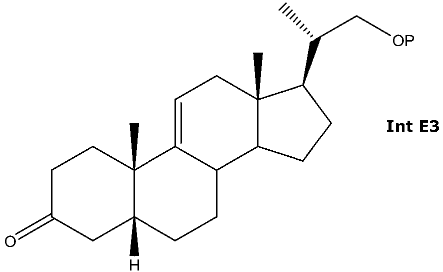
ii) reducing the intermediate of the general formula **Int E3** into an intermediate of the general formula **Int E5:** 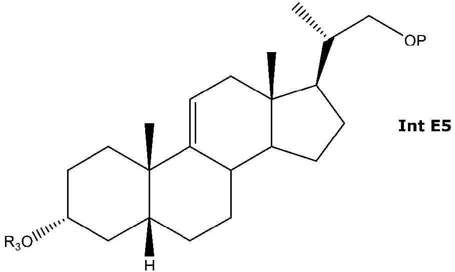
iii) oxidising the intermediate of the general formula **Int E5** into an intermediate of the general formula **Int E6:** 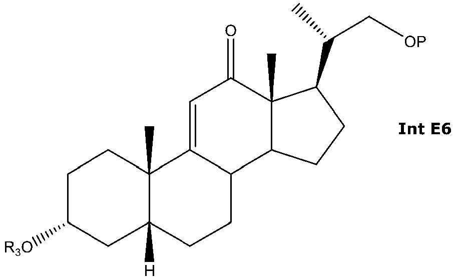
iv) reducing the intermediate of the general formula **Int E6** into an intermediate of the general formula **Int E7:** 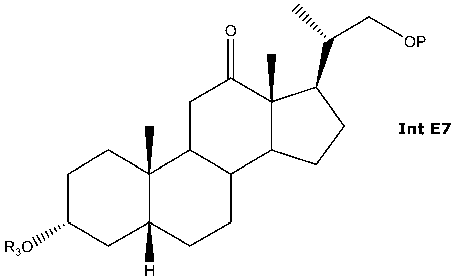
v) reducing the intermediate of the general formula **Int E7** into an intermediate of the general formula **Int E9** 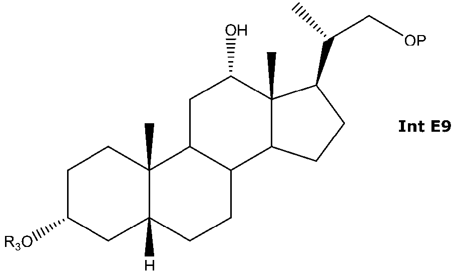
vi) elongating the carbon chain of the compound of the general formula **Int E9** to obtain an deoxycholic acid (DCA): 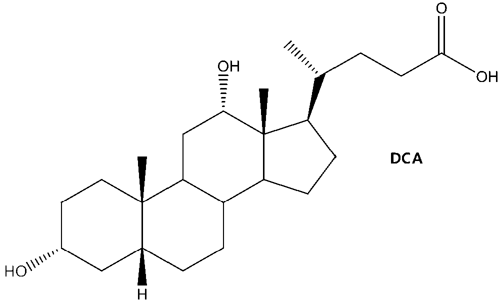
vii) optionally converting deoxycholic acid to a pharmaceutically acceptable salt thereof,
wherein
R₂ is H or a linear or branched C₁-C₆-alkyl group;
R₃ is H, R₂ or an alcohol protection group; and
P is an alcohol protection group.

### Synthetic route F

In still another interesting embodiment of the invention, the process for the preparation of deoxycholic acid (DCA) or a pharmaceutically acceptable salt thereof, comprises the following steps:
i) providing a compound of the general formula **SM-a:** 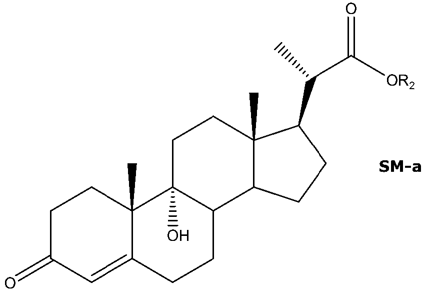
ii) reducing the compound of the general formula **SM-a** to obtain an intermediate of the general formula **SM-b:** 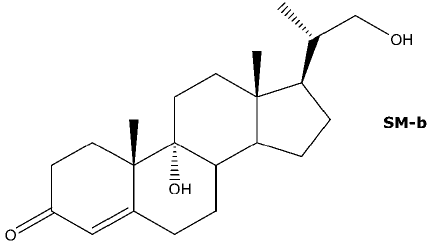
iii) protecting the alcohol group at position 22 to obtain an intermediate of the general formula **Int E1:** 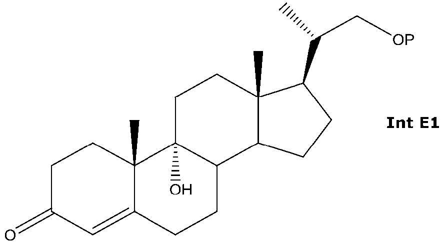
iv) converting the compound of the general formula **Int E1** into an intermediate of the general formula **Int F2:** 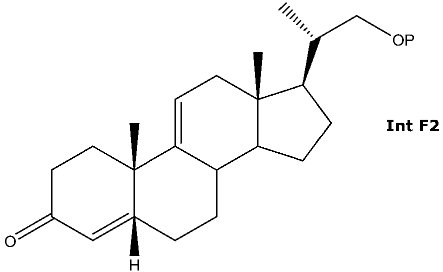
v) reducing the intermediate of the general formula **Int F2** into an intermediate of the general formula **Int E3:** 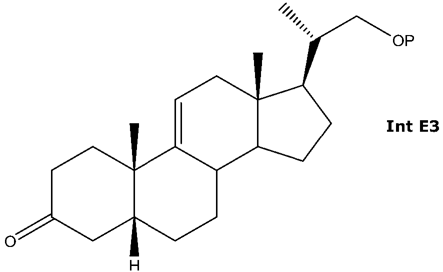
vi) reducing the intermediate of the general formula **Int E3** into an intermediate of the general formula **Int E5:** 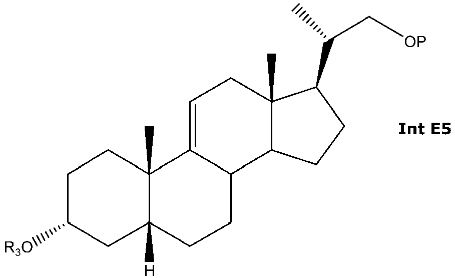
vii) oxidising the intermediate of the general formula **Int E5** into an intermediate of the general formula **Int E6:** 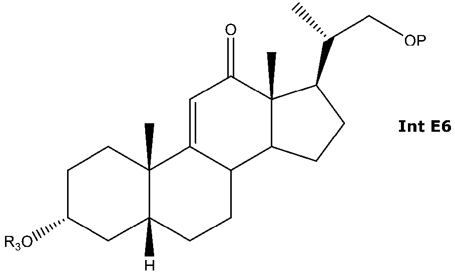
viii) reducing the intermediate of the general formula **Int E6** into an intermediate of the general formula **Int E7:** 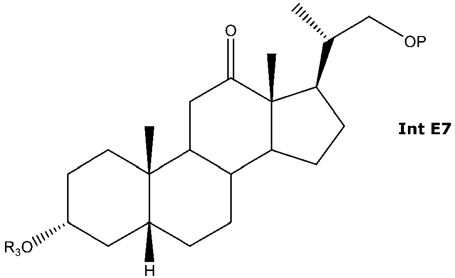
ix) reducing the intermediate of the general formula **Int E7** into an intermediate of the general formula **Int E9** 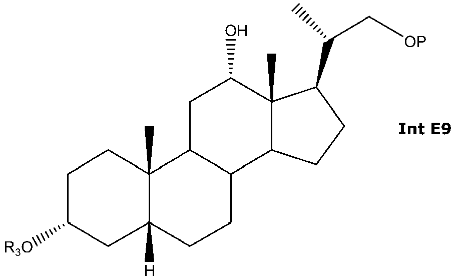
x) elongating the carbon chain of the compound of the general formula **Int E9** to obtain an deoxycholic acid (DCA): 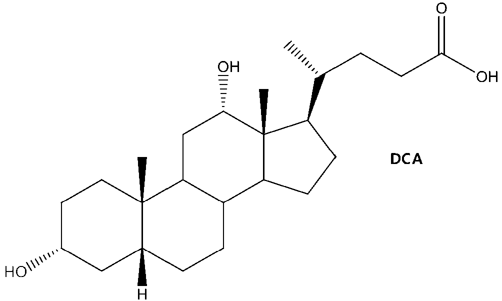
x) optionally converting deoxycholic acid to a pharmaceutically acceptable salt thereof,
wherein
R₂ is H or a linear or branched C₁-C₆-alkyl group;
R₃ is H, R₂ or an alcohol protection group; and
P is an alcohol protection group.

### The intermediate compounds

### The starting compound - the intermediate of the general formula SM

In a further aspect, the present invention relates to a compound of the general formula **SM** 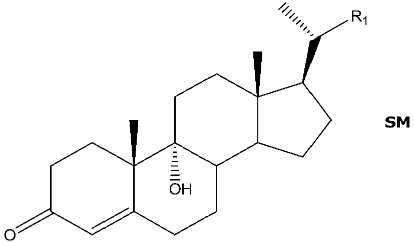 wherein
R₁ is COOR₂, CH₂OP, CH₂X, CH₂CHO, CH₂-CH₂-OH, CH₂-CH₂OP, CH₂-CH₂X or CH₂-CH₂-CHO;
R₂ is a linear or branched C₁-C₆-alkyl group with the proviso that R₂ is not CH₃;
P is an alcohol protection group with the proviso that P is not Ac; and
X is a halogen atom.

In a preferred embodiment of the invention, R₁ is COOR₂ or CH₂X where R₂ is selected from the group consisting of ethyl, n-propyl and iso-propyl, in particular ethyl, and X is selected from the group consisting of Cl, Br and I, in particular Br.

Accordingly, in one particular interesting embodiment of the invention R₁ is COOC₂H₅, and in another particular interesting embodiment of the invention R₂ is CH₂Br.

Such compounds can be obtained from (or easily prepared from compounds obtained from) fermentation products of *Mycobacterium fortuitum* in the presence of an appropriate carbon source.

For example, US 4,029,549 shows the production of 9α-OH BN acid, 9α-OH BN alcohol and 9α-OH BN methyl ester by fermenting the microorganism *Mycobacterium fortuitum* NRRL B-8119 in the presence of either sitosterol (example 2) or cholesterol, stigmasterol or campesterol (example 3). The purification and isolation of 9α-OH BN acid is disclosed in example 5 of US 4,029,549.

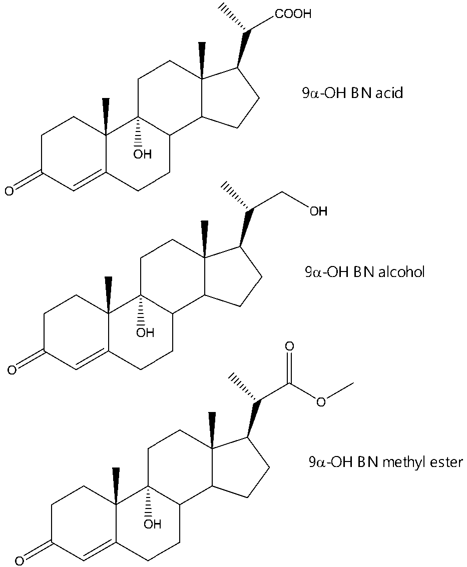

Accordingly, steps I) to VI) described herein may be preceded by a step comprising cultivating a 9α-OH BN acid-producing microorganism in an aqueous nutrient medium under aerobic conditions in the presence of a carbon source.

The 9α-OH BN acid-producing microorganism may be selected from the group consisting of *Arthrobacter, Bacillus, Brevibacterium, Corynebacterium, Microbacterium, Nocardia, Proaminobacter, Serratia, Streptomyces* and *Mycobacterium.* In a preferred embodiment of the invention the 9α-OH BN acid-producing microorganism is *Mycobacterium,* in particular *Mycobacterium fortuitum.* In the most preferred embodiment of the invention the 9α-OH BN acid-producing microorganism is *Mycobacterium fortuitum* NRRL B-8119.

The carbon source may be a steroid, such as cholesterol, stigmasterol, campesterol and sitosterol, preferably sitosterol.

As will be understood, 9α-OH BN acid, 9α-OH BN alcohol and 9α-OH BN methyl ester may, if needed, easily be converted into compounds of the general formula **SM** by standard methods well known to the person skilled in organic chemistry.

### The intermediate of the general formula INT 1

In a still further aspect, the present invention relates to a compound of the general formula **INT 1** 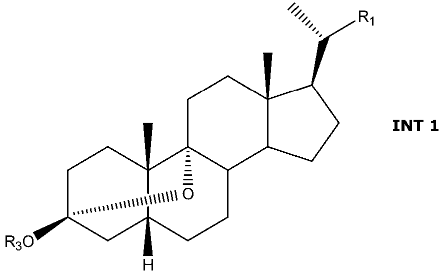 wherein
R₁ is COOR₂, CH₂OH, CH₂OP, CH₂X, CH₂CHO, CH₂-CH₂-OH, CH₂-CH₂OP, or CH₂-CH₂X or CH₂-CH₂-CHO;
R₂ is H or a linear or branched C₁-C₆-alkyl group;
P is an alcohol protection group;
R₃ either P or R₂; and
X is a halogen atom.

In a preferred embodiment of the invention, R₁ is COOR₂, CH₂X, CH₂OH or CH₂OP where R₂ is H or selected from the group consisting of methyl, ethyl, n-propyl and iso-propyl, in particular H or methyl, and X is selected from the group consisting of Cl, Br and I, in particular Br.

In a more preferred embodiment of the invention R₁ is COOR₂, CH₂X, CH₂OH or CH₂OP where R₂ is H or methyl, and X is Br.

In an even more preferred embodiment of the invention, R₁ is COOR₂ where R₂ is H or selected from the group consisting of methyl, ethyl, n-propyl and iso-propyl, in particular H or methyl, and X is selected from the group consisting of Cl, Br and I, in particular Br.

Accordingly, in one particular interesting embodiment of the invention R₁ is COOH or COOCH₃ and R₃ is H or CH₃CO. Specific examples include embodiments where R₁ is COOH and R₃ is H, where R₁ is COOCH₃ and R₃ is H, where R₁ is COOH and R₃ is CH₃CO, and where R₁ is COOCH₃ and R₃ is CH₃CO.

In another highly preferred embodiment of the invention, R₁ is CH₂OH and R₃ is either H or CH₃CO.

In a further highly preferred embodiment of the invention, R₁ is CH₂X and R₃ is either H or CH₃CO, and X is selected from the group consisting of Cl, Br and I, in particular Br. Specific examples include embodiments where R₁ is CH₂Br and R₃ is H, and where R₁ is CH₂Br and R₃ is CH₃CO.

In a still further highly preferred embodiment of the invention R₁ is CH₂OP and R₃ is either H or CH₃CO, wherein P is selected from the group consisting of trimethylsilyl ether (TMS), triethylsilyl ether (TES), triisopropylsilyl ether (TIPS), tert-butyldimethylsilyl ether (TBS, TBDMS), tert-butyldiphenylsilyl ether (TBDPS), acetyl (Ac, COCH₃), benzoyl (Bz), benzyl ether (Bn), 4-methoxybenzyl ether (PMB), 2-naphthylmethyl ether (Nap), methoxymethyl acetal (MOM), 2-methoxyethoxy-methyl ether (MEM), ethoxyethyl acetal (EE), methoxypropyl acetal (MOP), benzyloxymethyl acetal (BOM), tetrahydropyranyl acetal (THP), 2,2,2-trichloro-ethyl carbonate (Troc), methyl ether, dimethoxytrityl (DMT), methoxytrityl (MMT), methylthiomethyl ether, pivaloyl (Piv), tetrahydropyranyl (THP), triphenylmethyl (trityl, Tr), and tosyl (Ts), in particular Ac, TBDMS and Ts. Thus, specific embodiments include examples where R₁ is CH₂OAc and R₃ is H, where R₁ is CH₂OAc and R₃ is CH₃CO, where R₁ is CH₂OTBDMS and R₃ is H, where R₁ is CH₂OTBDMS and R₃ is CH₃CO, where R₁ is CH₂OTs and R₃ is H, and where R₁ is CH₂OTs and R₃ is CH₃CO.

Compounds of the general formula **INT** 1 may easily be prepared by reducing compounds of the general formula **SM** by methods well known to the person skilled in organic chemistry, as described herein.

### The intermediate of the general formula INT 2

In a still further aspect, the present invention relates to a compound of the general formula **INT 2** 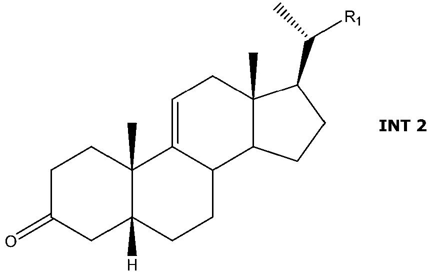 wherein
R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
R₂ is H or a linear or branched C₁-C₆-alkyl group;
P is an alcohol protection group; and
X is a halogen atom.

In a preferred embodiment of the invention, R₁ is COOR₂, CH₂X, CH₂OH or CH₂OP where R₂ is H or selected from the group consisting of methyl, ethyl, n-propyl and iso-propyl, in particular methyl, and X is selected from the group consisting of Cl, Br and I, in particular Br.

In a more preferred embodiment of the invention R₁ is COOR₂, CH₂X, CH₂OH or CH₂OP where R₂ is methyl, and X is Br.

Accordingly, in one particular interesting embodiment of the invention R₁ is COOCH₃. In another particular interesting embodiment of the invention R₁ is CH₂Br. In a further particular interesting embodiment of the invention R₁ is CH₂OH.

In a further highly preferred embodiment of the invention R₁ is CH₂OP, wherein P is selected from the group consisting of trimethylsilyl ether (TMS), triethylsilyl ether (TES), triisopropylsilyl ether (TIPS), tert-butyldimethylsilyl ether (TBS, TBDMS), tert-butyldiphenylsilyl ether (TBDPS), acetyl (Ac, COCH₃), benzoyl (Bz), benzyl ether (Bn), 4-methoxybenzyl ether (PMB), 2-naphthylmethyl ether (Nap), methoxymethyl acetal (MOM), 2-methoxyethoxy-methyl ether (MEM), ethoxyethyl acetal (EE), methoxypropyl acetal (MOP), benzyloxymethyl acetal (BOM), tetrahydropyranyl acetal (THP), 2,2,2-trichloro-ethyl carbonate (Troc), methyl ether, dimethoxytrityl (DMT), methoxytrityl (MMT), methylthiomethyl ether, pivaloyl (Piv), tetrahydropyranyl (THP), triphenylmethyl (trityl, Tr), and tosyl (Ts), in particular Ac, TBDMS and Ts. Thus, specific embodiments include examples where R₁ is CH₂OAc, where R₁ is CH₂OTBDMS and where R₁ is CH₂OTs.

Compounds of the general formula **INT 2** may easily be prepared by oxidising compounds of the general formula **INT 1** by methods well known to the person skilled in organic chemistry, as described herein.

### The intermediate of the general formula INT 3

In an even further aspect, the present invention relates to a compound of the general formula **INT 3** 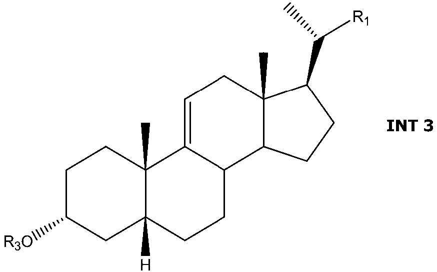 wherein
R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
R₂ is H or a linear or branched C₁-C₆-alkyl group;
P is an alcohol protection group;
R₃ is either P or R₂; and
X is a halogen atom;
with the proviso that R₁ is not CH₂-CH₂-OH when R₃ is H; R₁ is not CH₂-CH₂OAc when R₃ is Ac; and R₁ is not COOCH₃ when R₃ is Ac.

In a preferred embodiment of the invention, R₁ is COOR₂, CH₂X, CH₂OH or CH₂OP where R₂ is selected from the group consisting of ethyl, n-propyl and iso-propyl, in particular ethyl, and X is selected from the group consisting of Cl, Br and I, in particular Br.

In an interesting embodiment of the invention R₁ is COOCH₃ and R₃ is H.

In another interesting embodiment of the invention R₁ is CH₂X, wherein X is selected from the group consisting of Cl, Br and I, and R₃ is H or Ac. Particular examples are where X is Br and R₃ is H, and where X is Br and R₃ is Ac.

In another interesting embodiment of the invention R₁ is CH₂OP and R₃ is either H or CH₃CO, wherein P is selected from the group consisting of trimethylsilyl ether (TMS), triethylsilyl ether (TES), triisopropylsilyl ether (TIPS), tert-butyldimethylsilyl ether (TBS, TBDMS), tert-butyldiphenylsilyl ether (TBDPS), acetyl (Ac, COCH₃), benzoyl (Bz), benzyl ether (Bn), 4-methoxybenzyl ether (PMB), 2-naphthylmethyl ether (Nap), methoxymethyl acetal (MOM), 2-methoxyethoxy-methyl ether (MEM), ethoxyethyl acetal (EE), methoxypropyl acetal (MOP), benzyloxymethyl acetal (BOM), tetrahydropyranyl acetal (THP), 2,2,2-trichloro-ethyl carbonate (Troc), methyl ether, dimethoxytrityl (DMT), methoxytrityl (MMT), methylthiomethyl ether, pivaloyl (Piv), tetrahydropyranyl (THP), triphenylmethyl (trityl, Tr), and tosyl (Ts), in particular Ac, TBDMS and Ts. Thus, specific embodiments include examples where R₁ is CH₂OAc and R₃ is H, where R₁ is CH₂OAc and R₃ is CH₃CO, where R₁ is CH₂OTBDMS and R₃ is H, where R₁ is CH₂OTBDMS and R₃ is CH₃CO, where R₁ is CH₂OTs and R₃ is H, and where R₁ is CH₂OTs and R₃ is CH₃CO.

Compounds of the general formula **INT** 3 may easily be prepared by reducing compounds of the general formula **INT** 2 by methods well known to the person skilled in organic chemistry, as described herein.

As will be understood, the intermediates disclosed herein can be used for the preparation of DCA and pharmaceutically acceptable salts thereof. Since the synthetic routes described herein allow for introduction of an -OH group in position 12, it is contemplated that the same intermediates will also be suitable for preparing other bile acids, which include an -OH group in position 7. Specific examples of such bile salts include cholic acid, glycocholic acid, taurocholic acid, or a pharmaceutically acceptable salt thereof.

### Biobased DCA from plant sources

The DCA prepared from the processes as described above may preferably be a biobased DCA, where the atoms of the DCA originates from plant sources. This may be obtained by providing the compound of formula SM-a from plant sources and amending and elongating this compound by use of compounds obtained only from plant sources. In one embodiment, the compounds used for modifying the compound of formula SM-a into DCA come from waste products obtained from the processing of plant products such as polysaccharides from sugar fermentation.

In one embodiment, the carbon atoms of DCA or a pharmaceutically acceptable salt of DCA originates solely from plant sources. Thus, all of the carbon atoms of the DCA or the pharmaceutically acceptable salt of DCA are derived from plants.

In another embodiment, the carbon atoms of DCA or a pharmaceutically acceptable salt of DCA originates partially from plant sources being C3 plants. In a further embodiment, the carbon atoms of DCA or a pharmaceutically acceptable salt of DCA originates primarily from plant sources being C3 plants. In a further embodiment, the carbon atoms of DCA or a pharmaceutically acceptable salt of DCA originates solely from plant sources being C3 plants i.e. all of the carbon atoms of the DCA or the pharmaceutically acceptable salt of DCA is derived from C3 plants.

In another embodiment, the carbon atoms of DCA or a pharmaceutically acceptable salt of DCA originates partially from phytosterols or phytosterol derivatives.

In a further embodiment, the carbon atoms of DCA or a pharmaceutically acceptable salt of DCA originates partially from phytosterols, phytosterol derivatives and C3 plants. In a further embodiment, the carbon atoms of DCA or a pharmaceutically acceptable salt of DCA originates primarily from phytosterols,phytosterol derivatives and C3 plants. In a further embodiment, the carbon atoms of DCA or a pharmaceutically acceptable salt of DCA originates solely from phytosterols, phytosterol derivatives and C3 plants i.e. all of the carbon atoms of the DCA or the pharmaceutically acceptable salt of DCA is derived from phytosterols, phytosterol derivatives and C3 plants.

In another embodiment, the carbon atoms of DCA or a pharmaceutically acceptable salt of DCA originates partially from non-mammalian sources. In a further embodiment, the carbon atoms of DCA or a pharmaceutically acceptable salt of DCA originates primarily from non-mammalian sources. In a further embodiment, the carbon atoms of DCA or a pharmaceutically acceptable salt of DCA originates solely from non-mammalian sources i.e. all of the carbon atoms of the DCA or the pharmaceutically acceptable salt of DCA is not derived from mammals.

The origin of a compound may be identified based on the content of C14 and C13 in the compound. Carbon atoms do naturally occur in three different isotopes: C12, C13 and C14. C12 makes up 99%, while C13 makes up 1% and C14 occurs in trace amounts. C12 and C13 are both stable, while C14 is a radioactive isotope of carbon.

C14 can be used to demonstrate the percentage of carbon atoms which originates from "natural" (plant or animal by-product) sources i.e. biobased carbons or "synthetic" (petrochemical) sources i.e. fossil carbons. Thus, the fossil carbon percentage is the percentage of carbon atoms deriving from "synthetic" sources out of the total number of carbon atoms, while the biobased carbon percentage is the percentage of carbon atoms deriving from "natural" sources out of the total number of carbon atoms. Consequently, the sum of the fossil carbon percentage and the biobased carbon percentage will be 100%.

The differentiation of C14 into "natural" or "synthetic" sources defined by percentages is a common way of characterising as compared with ppt-measurements as also described in US 8,242,294 B2. Furthermore, measurements using an ASTM D6866 method of the C14 content would reveal the C14 content only in percentage and not in ppt.

In one aspect of the invention, a biobased DCA would be a DCA molecule where at least 90% of the carbon atoms is derived from natural sources. In a further embodiment, a biobased DCA would be a DCA molecule where at least 95% of the carbon atoms are derived from natural sources. In a further embodiment, a biobased DCA would be a DCA molecule where at least 97% of the carbon atoms are derived from natural sources. In a further embodiment, a biobased DCA is a DCA molecule where at least 99% of the carbon atoms are derived from natural sources. In a further embodiment, a biobased DCA is a DCA molecule were essentially all carbon atoms are derived from natural sources. In a further embodiment, a biobased DCA is a DCA molecule where all carbon atoms are derived from natural sources.

In a further aspect of the invention, a biobased DCA from a plant source would be a DCA molecule where at least 90% of the carbon atoms is derived from natural sources being plants. In a further aspect of the invention, a biobased DCA from a plant source would be a DCA molecule where at least 95% of the carbon atoms is derived from natural sources being plants. In a further aspect of the invention, a biobased DCA from a plant source would be a DCA molecule where at least 97% of the carbon atoms is derived from natural sources being plants. In a further aspect of the invention, a biobased DCA from a plant source would be a DCA molecule where at least 99% of the carbon atoms is derived from natural sources being plants. In a further aspect of the invention, a biobased DCA from a plant source would be a DCA molecule where essentially all carbon atoms is derived from natural sources being plants. In a further aspect of the invention, a biobased DCA from a plant source would be a DCA molecule where all carbon atoms is derived from natural sources being plants.

In a further aspect of the invention, a biobased DCA from a non-mammalian source would be a DCA molecule where at least 90% of the carbon atoms is derived from natural sources being non-mammalian. In a further aspect of the invention, a biobased DCA from a non-mammalian source would be a DCA molecule where at least 95% of the carbon atoms is derived from natural sources being non-mammalian. In a further aspect of the invention, a biobased DCA from a non-mammalian source would be a DCA molecule where at least 97% of the carbon atoms is derived from natural sources being non-mammalian. In a further aspect of the invention, a biobased DCA from a non-mammalian source would be a DCA molecule where at least 99% of the carbon atoms is derived from natural sources being non-mammalian. In a further aspect of the invention, a biobased DCA from a non-mammalian source would be a DCA molecule where essentially all carbon atoms is derived from natural sources being non-mammalian. In a further aspect of the invention, a biobased DCA from a non-mammalian source would be a DCA molecule where all carbon atoms is derived from natural sources being non-mammalian.

One way to distinguish between the "natural" and "synthetic" sources is to use the % Biobased Carbon Content determined according to ASTM D6866-16 Method B (AMS). Other standards have be developed but the analytical procedures for measuring radiocarbon content using the different standards are identical. The only difference is the reporting format. Results are usually reported using the standardized terminology "% biobased carbon". Only ASTM D6866 uses the term "% biogenic carbon" when the result represents all carbon present (Total Carbon) rather than just the organic carbon (Total Organic Carbon).

A value of 100% biobased or biogenic carbon would indicate that 100% of the carbon came from plants or animal by-products (biomass) living in the natural environment and a value of 0% would mean that all of the carbon was derived from petrochemicals, coal and other fossil sources. A value between 0-100% would indicate a mixture of fossil carbon and biobased carbon. The higher the value, the greater the proportion of biobased carbons i.e. naturally sourced components in the material.

In one embodiment, the fossil carbon percentage in the deoxycholic acid is less than 10 percent. In a further embodiment, the fossil carbon percentage in the deoxycholic acid is less than 9 percent. In a further embodiment, the fossil carbon percentage in the deoxycholic acid is less than 8 percent. In a further embodiment, the fossil carbon percentage in the deoxycholic acid is less than 7 percent. In a further embodiment, the fossil carbon percentage in the deoxycholic acid is less than 6 percent. In a further embodiment, the fossil carbon percentage in the deoxycholic acid is less than 5 percent. In a further embodiment, the fossil carbon percentage in the deoxycholic acid is less than 4 percent. In a further embodiment, the fossil carbon percentage in the deoxycholic acid is less than 3 percent. In a further embodiment, the fossil carbon percentage in the deoxycholic acid is less than 2 percent. In a further embodiment, the fossil carbon percentage in the deoxycholic acid is less than 1 percent. In a further embodiment, the fossil carbon percentage in the deoxycholic acid is less than 0.1 percent. In a further embodiment, the fossil carbon percentage in the deoxycholic acid is less than 0.01 percent.

In one embodiment, the fossil carbon percentage in the pharmaceutically acceptable salt of the deoxycholic acid is less than 10 percent. In a further embodiment, the fossil carbon percentage in the pharmaceutically acceptable salt of the deoxycholic acid is less than 9 percent. In a further embodiment, the fossil carbon percentage in the pharmaceutically acceptable salt of the deoxycholic acid is less than 8 percent. In a further embodiment, the fossil carbon percentage in the pharmaceutically acceptable salt of the deoxycholic acid is less than 7 percent. In a further embodiment, the fossil carbon percentage in the pharmaceutically acceptable salt of the deoxycholic acid is less than 6 percent. In a further embodiment, the fossil carbon percentage in the pharmaceutically acceptable salt of the deoxycholic acid is less than 5 percent. In a further embodiment, the fossil carbon percentage in the pharmaceutically acceptable salt of the deoxycholic acid is less than 4 percent. In a further embodiment, the fossil carbon percentage in the pharmaceutically acceptable salt of the deoxycholic acid is less than 3 percent. In a further embodiment, the fossil carbon percentage in the pharmaceutically acceptable salt of the deoxycholic acid is less than 2 percent. In a further embodiment, the fossil carbon percentage in the pharmaceutically acceptable salt of the deoxycholic acid is less than 1 percent. In a further embodiment, the fossil carbon percentage in the pharmaceutically acceptable salt of the deoxycholic acid is less than 0.1 percent. In a further embodiment, the fossil carbon percentage in the pharmaceutically acceptable salt of the deoxycholic acid is less than 0.01 percent.

The amount of C14 may also be used for the characterization of the deoxycholic acid and the pharmaceutically acceptable salt thereof. This measurement may be performed using standard techniques and possibly a gas ionization detector.

The origin of the carbon atoms may be even further differentiated by measurement of the δ¹³C value as disclosed e.g. in US 8,076,156 and "Stable Isotope Ratios as biomarkers of diet for health research" by D.M. O'Brien, Annual Reviews (www.annualreviews.org), 2015. The δ-value appears as the C13 is measured in relation to a standard being Pee Dee Belemnite based on a Cretaceous marine fossil, which had an anomalously high C13.

Biochemical reactions discriminate against ¹³C, why the concentration of ¹²C is increased in biological materials. In this manner, different sources such as plant versus animal may be distinguished using the pure compounds as reference values as described in Application Note 30276 from Thermo Scientific: "Detection of Squalene and Squalane Origin with Flash Elemental Analyzer and Delta V Isotope Ratio Mass Spectrometer" by Guibert et al. (2013).

The δ¹³C values may also differ among plants due to their different photosynthethic physiology. This may be observed by C3 plants such as wheat, rice, beans, most fruits and vegetables exhibit a higher δ¹³C value than C4 plants such as corn, sugar cane and sorghum ("Stable Isotope Ratios as biomarkers of diet for health research" by D.M. O'Brien, Annual Reviews (www.annualreviews.org), 2015).

In one embodiment, the deoxycholic acid shows a δ¹³C value that is different from the δ¹³C value of deoxycholic acid obtained from animal sources. In a further embodiment, the deoxycholic acid shows a δ¹³C value that is different from the δ¹³C value of deoxycholic acid obtained from mammal sources. The animal sources may be cows and sheep.

In one embodiment, the deoxycholic acid or a pharmaceutically acceptable salt thereof has a mean δ¹³C value in the range from -20‰ to - 40‰. In a further embodiment, the deoxycholic acid or a pharmaceutically acceptable salt thereof has a mean δ¹³C value in the range from -21‰ to -35‰. In a further embodiment, the deoxycholic acid or a pharmaceutically acceptable salt thereof has a mean δ¹³C value in the range from -20‰ to - 32‰. In a further embodiment, the deoxycholic acid or a pharmaceutically acceptable salt thereof has a mean δ¹³C value in the range from - 25‰ to -28‰. In a further embodiment, the deoxycholic acid or a pharmaceutically acceptable salt thereof has a mean δ¹³C value around - 26‰.

In one embodiment, the deoxycholic acid or a pharmaceutically acceptable salt thereof has a mean δ¹³C value different from a mean δ¹³C value of around -12‰. In a further embodiment, the deoxycholic acid or a pharmaceutically acceptable salt thereof has a mean δ¹³C value different from a mean δ¹³C value in the range from -10‰ to -14‰.

In one embodiment, the deoxycholic acid or the pharmaceutically acceptable salt thereof does not comprise impurities of animal origin. In a further embodiment, the deoxycholic acid or the pharmaceutically acceptable salt thereof does not comprise further cholic acids.

In one embodiment, the deoxycholic acid or the pharmaceutically acceptable salt thereof is derived solely from plant sources, comprises a fossil carbon percentage of less than 3 percent and has a mean δ¹³C value different from the mean δ¹³C value of deoxycholic acid obtained from animal sources.

In one embodiment, the deoxycholic acid or the pharmaceutically acceptable salt thereof is derived solely from plant sources, comprises a fossil carbon percentage of less than 1 percent and has a mean δ¹³C value different from the mean δ¹³C value of deoxycholic acid obtained from animal sources.

In one embodiment, the deoxycholic acid or the pharmaceutically acceptable salt thereof is derived solely from plant sources, comprises a fossil carbon percentage of less than 0.1 percent and has a mean δ¹³C value different from the mean δ¹³C value of deoxycholic acid obtained from animal sources.

In one embodiment, the deoxycholic acid or the pharmaceutically acceptable salt thereof is derived solely from plant sources, comprises a fossil carbon percentage of less than 0.01 percent and has a mean δ¹³C value different from the mean δ¹³C value of deoxycholic acid obtained from animal sources.

In one embodiment, the deoxycholic acid or the pharmaceutically acceptable salt thereof is derived from phytosterols or phytosterol derivatives, comprises a fossil carbon percentage of less than 3 percent and has a mean δ¹³C value in the range from -21‰ to - 35‰.

In one embodiment, the deoxycholic acid or the pharmaceutically acceptable salt thereof is derived from phytosterols or phytosterol derivatives, comprises a fossil carbon percentage of less than 1 percent and has a mean δ¹³C value in the range from -21‰ to - 35‰.

In one embodiment, the deoxycholic acid or the pharmaceutically acceptable salt thereof is derived solely from phytosterols or phytosterol derivatives, comprises a fossil carbon percentage of less than 0.1 percent and has a mean δ¹³C value in the range from -21‰ to - 35‰.

In one embodiment, the deoxycholic acid or the pharmaceutically acceptable salt thereof is derived solely from phytosterols or phytosterol derivatives, comprises a fossil carbon percentage of less than 0.01 percent and has a mean δ¹³C value in the range from -21‰ to - 35‰.

In one embodiment, the deoxycholic acid or the pharmaceutically acceptable salt thereof is derived from phytosterols or phytosterol derivatives, comprises a fossil carbon percentage of less than 3 percent and has a mean δ¹³C value different from the mean δ¹³C value of deoxycholic acid obtained from animal sources.

In one embodiment, the deoxycholic acid or the pharmaceutically acceptable salt thereof is derived from phytosterols or phytosterol derivatives, comprises a fossil carbon percentage of less than 1 percent and has a mean δ¹³C value different from the mean δ¹³C value of deoxycholic acid obtained from animal sources.

In one embodiment, the deoxycholic acid or the pharmaceutically acceptable salt thereof is derived solely from phytosterols or phytosterol derivatives, comprises a fossil carbon percentage of less than 0.1 percent and has a mean δ¹³C value different from the mean δ¹³C value of deoxycholic acid obtained from animal sources.

In one embodiment, the deoxycholic acid or the pharmaceutically acceptable salt thereof is derived solely from phytosterols or phytosterol derivatives, comprises a fossil carbon percentage of less than 0.01 percent and has a mean δ¹³C value different from the mean δ¹³C value of deoxycholic acid obtained from animal sources.

In one embodiment, the deoxycholic acid or the pharmaceutically acceptable salt thereof is derived solely from plant sources, comprises a fossil carbon percentage of less than 3 percent and has a mean δ¹³C value in the range from -21‰ to - 35‰.

In one embodiment, the deoxycholic acid or the pharmaceutically acceptable salt thereof is derived solely from plant sources, comprises a fossil carbon percentage of less than 1 percent and has a mean δ¹³C value different from the mean δ¹³C value in the range from -21‰ to - 35‰.

In one embodiment, the deoxycholic acid or the pharmaceutically acceptable salt thereof is derived solely from plant sources, comprises a fossil carbon percentage of less than 0.1 percent and has a mean δ¹³C value in the range from -21‰ to - 35‰.

In one embodiment, the deoxycholic acid or the pharmaceutically acceptable salt thereof is derived solely from plant sources, comprises a fossil carbon percentage of less than 0.01 percent and has a mean δ¹³C value different from the mean δ¹³C value in the range from -21‰ to - 35‰.

In one embodiment, the deoxycholic acid or the pharmaceutically acceptable salt thereof is derived from phytosterols or phytosterol derivatives, comprises a fossil carbon percentage of less than 1 percent, has a mean δ¹³C value different from the mean δ¹³C value of deoxycholic acid obtained from animal sources and does not comprise impurities of animal origin.

In one embodiment, the deoxycholic acid or the pharmaceutically acceptable salt thereof is derived solely from phytosterols or phytosterol derivatives, comprises a fossil carbon percentage of less than 0.1 percent, has a mean δ¹³C value different from the mean δ¹³C value of deoxycholic acid obtained from animal sources and does not comprise impurities of animal origin.

In one embodiment, the deoxycholic acid or the pharmaceutically acceptable salt thereof is derived solely from plant sources, comprises a fossil carbon percentage of less than 1 percent, has a mean δ¹³C value different from the mean δ¹³C value in the range from -21‰ to - 35‰ and does not comprise impurities of animal origin.

In one embodiment, the deoxycholic acid or the pharmaceutically acceptable salt thereof is derived solely from plant sources, comprises a fossil carbon percentage of less than 0.1 percent, has a mean δ¹³C value in the range from -21‰ to - 35‰ and does not comprise impurities of animal origin.

In one embodiment, the deoxycholic acid or the pharmaceutically acceptable salt thereof is derived from phytosterols or phytosterol derivatives, comprises a fossil carbon percentage of less than 1 percent, has a mean δ¹³C value different from the mean δ¹³C value of deoxycholic acid obtained from animal sources and does not comprise further cholic acids.

In one embodiment, the deoxycholic acid or the pharmaceutically acceptable salt thereof is derived solely from phytosterols or phytosterol derivatives, comprises a fossil carbon percentage of less than 0.1 percent, has a mean δ¹³C value different from the mean δ¹³C value of deoxycholic acid obtained from animal sources and does not comprise further cholic acids.

In one embodiment, the deoxycholic acid or the pharmaceutically acceptable salt thereof is derived solely from plant sources, comprises a fossil carbon percentage of less than 1 percent, has a mean δ¹³C value different from the mean δ¹³C value in the range from -21‰ to - 35‰ and does not comprise further cholic acids.

In one embodiment, the deoxycholic acid or the pharmaceutically acceptable salt thereof is derived solely from plant sources, comprises a fossil carbon percentage of less than 0.1 percent, has a mean δ¹³C value in the range from -210‰ to - 35‰ and does not comprise further cholic acids.

In a still further embodiment, the deoxycholic acid or the pharmaceutically acceptable salt thereof may be further distinguished from deoxycholic acid originating from an animal source. The deoxycholic acid or the pharmaceutically acceptable salt thereof may be distinguished by difference in other naturally occurring isotopes such as hydrogen isotopes, which pattern may be different from DCA derived from animal sources and DCA synthezised partially or solely from plant sources.

In a further embodiment, the DCA derived from animal sources and the DCA synthesized partially or solely from plant sources may be distinguished by other applicable marker(s).

A potential applicable marker could be characterization using PCR (polymerase chain reaction) for example by detecting possible impurities in DCA obtained from animal origin.

### Examples

### Example 1

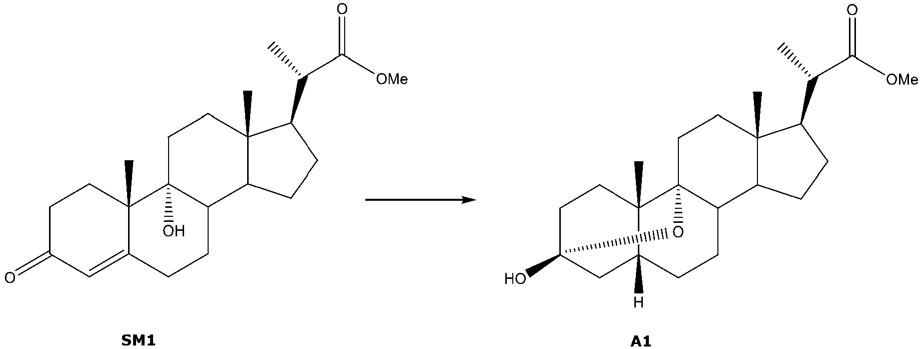

40 g of compound **SM1** (106.80 mmol) was suspended in 150 ml of DMF, then 2.77 g of dry Pd/C 10% was added. The reaction mixture was stirred at 70°C and hydrogenated (3.5 atm) overnight. The mixture was filtered through Celite^{®}. Then, the mixture was poured over water forming a precipitate. The precipitate was filtered off as a white solid, washed with water and dried under vacuum, thereby yielding 39.3 g of compound **A1.**

¹H NMR (400 MHz, CDCl₃): δ 3.56 (s, 3H); 2.30 (m, 1H); 1.10 (d, 3H); 0.87 (s, 3H); 0.62 (s, 3H).

### Example 2

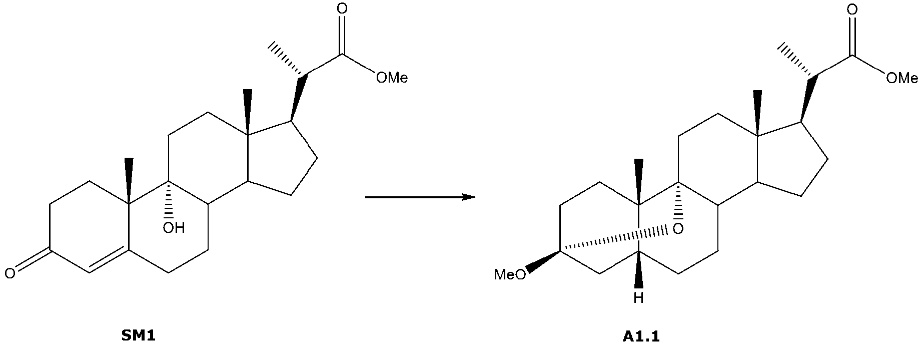

20 g of compound **SM1** (53.40 mmol) was suspended in 150 ml of MeOH, then 1.4 g of dry Pd/C 10% was added. The reaction mixture was stirred at 70°C and hydrogenated (1.0 atm) overnight. 1.0 g of p-TsOH (10% molar, 5.3 mmol) was added and stirred for 8 h. The mixture was filtered through Celite^{®}. The solvent was evaporated under vacuum. The solid was recrystallized in 60 ml of EtOH. The solid was filtered off and dried under vacuum, yielding 18.8 g of compound **A1.1.**

### Example 3

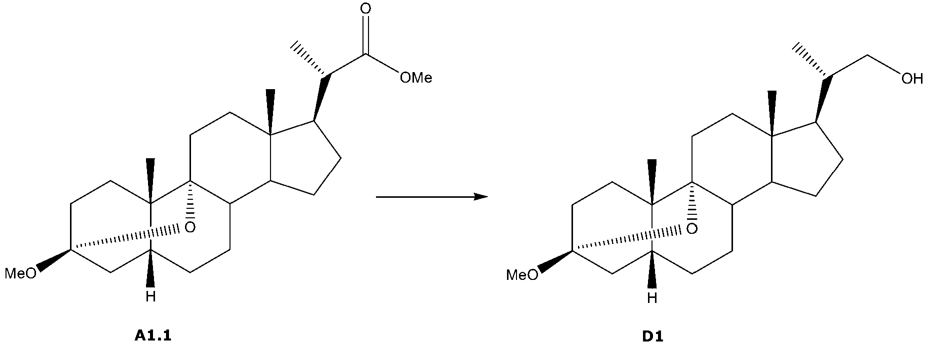

LiAliH₄ (1.88 g, 49.63 mmol, 1.3 eq.) and THF (20 ml) were mixed in an inert atmosphere. A mixture of **A1.1** (14.0 g) and 40 ml of THF was added dropwise. The mixture was stirred overnight at room temperature until the reaction was completed. The mixture was then cooled at 0-5°C and was quenched by dropwise addition of an aqueous solution of Na₂SO₄•10H₂O (16.20 g) and THF (50 ml). The precipitate was filtered off, the solvent was evaporated under reduced pressure. The solid was recrystallized in 150 ml EtOH. The solid was filtered off and dried under vacuum, thereby yielding 10.15 g of **D1.**

### Example 4

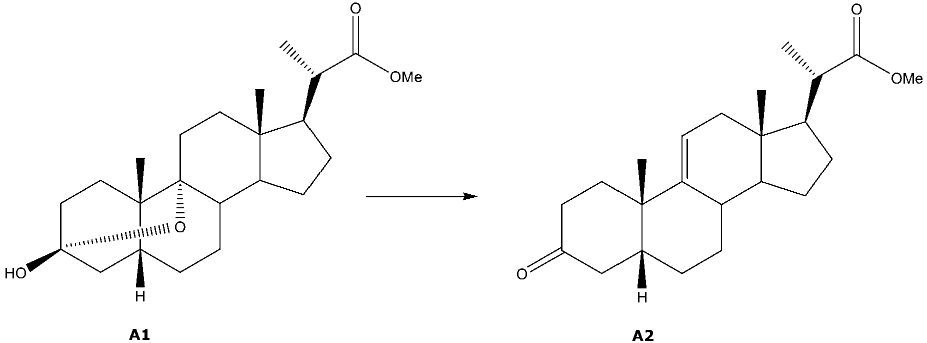

39, 3 g of **A1** (104.37 mmol) was dissolved in DCM (100 ml) and stirred at room temperature. Sulphuric acid (9.29 g, 0.9 eqv) was added at 10°C, then the mixture is stirred overnight. The reaction mixture was worked up by washing with water and NaHCO₃ and he organic layer was separated. The organic layer was concentrated followed by column chromatography to yield 28.8 g of **A2.**

¹H NMR (400 MHz, CDCl₃): δ 5.50 (s, 1H); 3.57 (s, 3H); 1.15 (d, 3H); 1.09 (s, 3H); 0.59 (s, 3H).

### Example 5

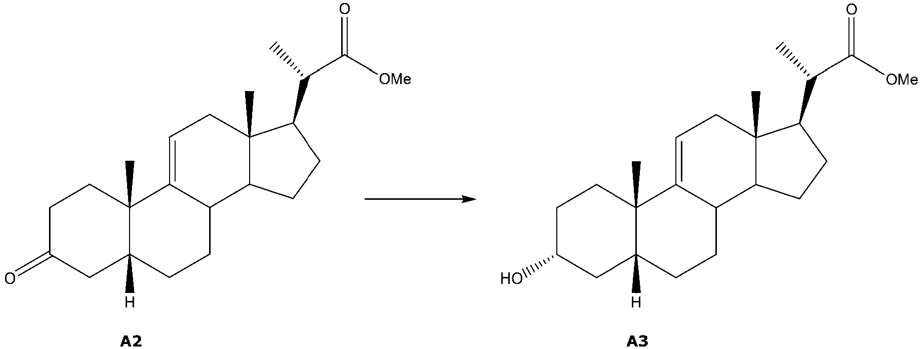

28,6 g of **A2** (79.87 mmol) was dissolved and stirred in THF (100 ml) under inert atmosphere. The solution was cooled at 0-5°C and LiAlH(OtBu)₃ (22.34 g, 1.1 eqv) was added slowly (exothermic reaction). The mixture was stirred at room temperature until the reaction was complete. The mixture was cooled at 0-5°C and was hydrolyzed slowly with a solution of 1M HCl. The aqueous phase was extracted with EtOAc and the organic phase is washed with a solution of NaHCO₃. The solvent was evaporated under reduced pressure, yielding 27.62 g of **A3.** ¹H NMR (400 MHz, CDCl₃): δ 0.59 (s, 3H); 1.05 (s, 3H); 1.18 (d, 3H); 2.43 (m, 2H); 3.65 (s, 3H); 3.65 (m, 1H); 5.32 (dd, 1H).

¹H NMR (400 MHz, CDCl₃): δ 5.29 (s, 1H); 3.57 (s, 3H); 3.39 (m, 1H); 1.11 (d, 3H); 1.01 (s, 3H); 0.55 (s, 3H).

### Example 6

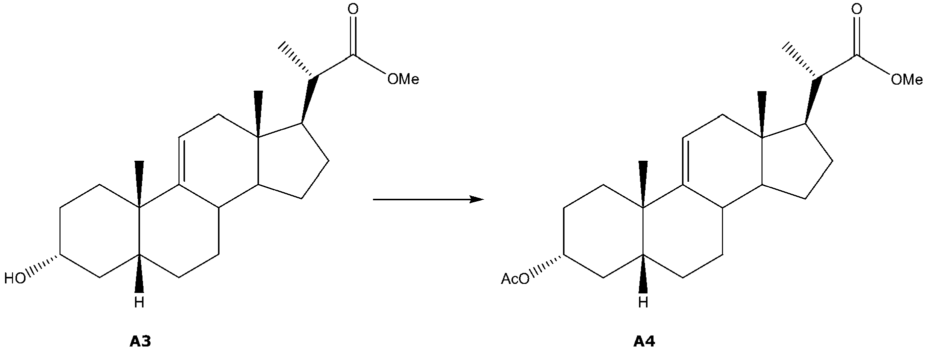

**A3** 27.62 g (76.61 mmol) (10 g) was dissolved in DCM (70 ml) at room temperature. Then, Triethylamine 50 ml (6.66 eqv), Acetic anhydride 8.85 g (3.33 eq) and DMAP (3.40 g) were added, keeping the temperature below 10°C. The mixture stirred until reaction was complete. The organic phase was concentrated under reduced pressure and the solid was suspended in 60 mL of DCM and then washed with a solution of 1M HCl. The solvent was evaporated under reduced pressure thereby yielding 32.29 g of **A4.** ¹H NMR (400 MHz, CDCl₃): δ 0.58 (s, 3H); 1.04 (s, 3H); 1.16 (d, 3H); 1.99 (s, 3H); 2.41 (m, 2H); 3.63 (s, 3H); 4.71 (m, 1H); 5.31 (dd, 1H).

¹H NMR (400 MHz, CDCl₃): δ 5.31 (s, 1H); 3.60 (s, 3H); 2.00 (m, 3H); 1.17 (d, 3H); 0.59 (s, 3H).

### Example 7

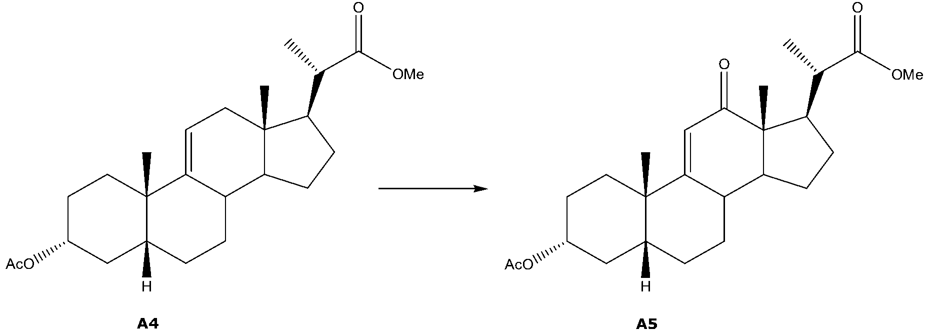

27 g of compound **A4** (64.5 mmol) was suspended in 300 ml of AcOH and then anhydrous CrO₃ (27.73 g, 4.30 eqv) was added. The suspension was heated at 60°C. The reaction mixture was stirred for 0.5 h until the reaction was complete. Then, the mixture was poured over 250 mL of water and a precipitate was formed. The organic phase was washed with water. The operation was repeated two more times. The organic phase were concentrated until an oily residue was obtained. The residue was purified on silica gel yielding 13.05 g of pure **A5.**

¹H NMR (400 MHz, CDCl₃): δ 0.84 (s, 3H); 1.17 (s, 3H); 1.30 (d, 3H); 1.98 (s, 3H); 3.63 (s, 3H); 4.72 (m, 1H); 5.72 (s, 1H).

### Example 8

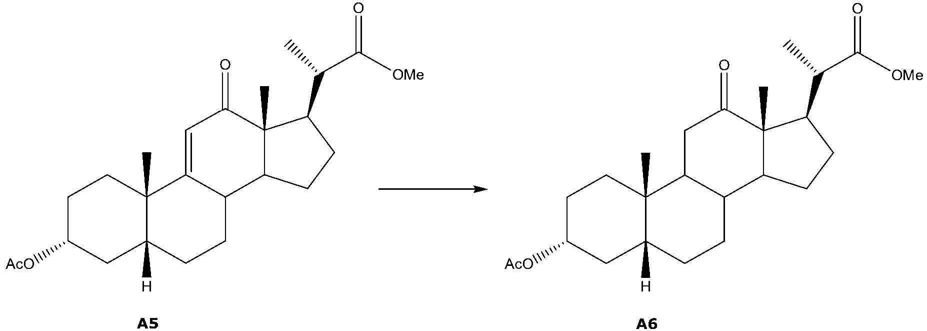

11 g of **A5** (28.31 mmol) was dissolved in 65 ml of AcOEt followed by addition of 2.75 g dry Pd/C 10% (25% weight). The reaction mixture was stirred at 70°C and hydrogenated (4.1 atm) overnight. The mixture was filtered through Celite^{®} and the solvent was evaporated under vacuum, thereby yielding 11.02 g of **A6** (a white solid. ¹H NMR (400 MHz, CDCl₃): δ 0.97 (s, 3H); 1.00 (s, 3H); 1.15 (d, 3H); 2.00 (s, 3H); 2.46 (m, 2H); 3.63 (s, 3H); 4.68 (m, 1H).

### Example 9

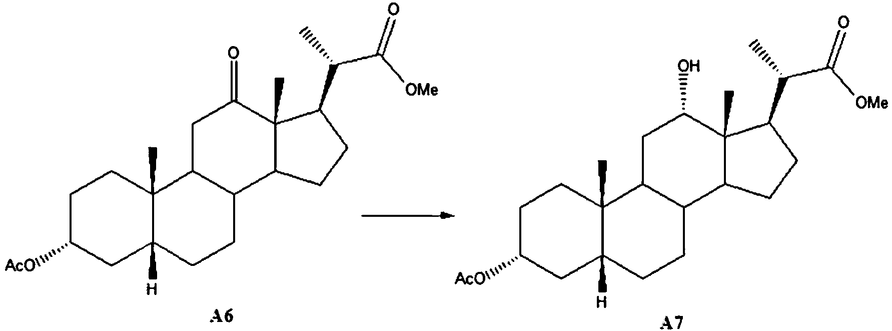

11.02 g of **A6** (26.30 mmol) was dissolved and stirred in THF (40 ml) under an inert atmosphere. The solution was cooled at 0-5°C. LiAlH(OtBu)₃ (1.5 eqv, 10.0 g, 39.45 mmol) was added dropwise (exothermic reaction). The mixture was stirred at room temperature until the reaction was complete. The mixture was cooled at 0-5°C and was then quenched by adding an aqueous solution of 1M HCl. The aqueous phase was extracted with EtOAc and the organic phase was washed with a solution of NaHCO₃. The solvent was evaporated under reduced pressure, thereby yielding 11.16 g of **A7.** ¹H NMR (400 MHz, CDCl₃): δ 0.66 (s, 3H); 0.89 (s, 3H); 1.20 (d, 3H); 2.00 (s, 3H); 3.62 (s, 3H); 3.92 (m, 1H); 4.72 (m, 1H).

### Example 10

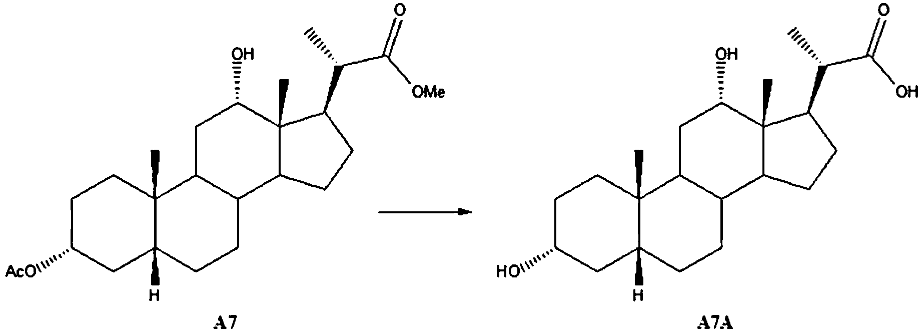

3.00 g of **A7** (7.13 mmol) was dissolved and stirred in a mixture of THF (30 ml) and MeOH (30 ml) under an inert atmosphere at room temperature. LiOH (4M, 30 ml) was added. The solution was heated at 60°C. The mixture was stirred until the reaction was complete (6 h). The mixture was cooled at room temperature. The solvent was evaporated and was quenched by adding an aqueous solution of HCl 2N until acidic pH. The precipitate was filtered off as a palid yellow solid, washed with water and EtOAc, and then dried under vacuum yileding 2.53 g of **A7A.**

### Example 11

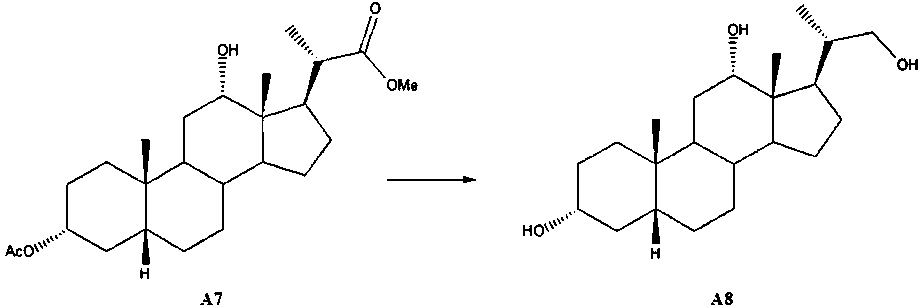

0.3 g of **A7** (0.71 mmol) was dissolved and stirred in dry THF (7 ml) under an inert atmosphere. The solution was cooled at 0-5°C. LiAlH₄ (0.06 g, 1.49 mmol) was added dropwise (exothermic reaction). The mixture was stirred at room temperature until the reaction was completed. The mixture was cooled at 0-5°C and was quenched by addition Na₂SO₄•10H₂O. The precipitate was filtered off and washed with THF. The solvent was evaporated under reduced pressure and the solid obtained was washed with EtOAc, thereby yielding 0.198 g of **A8.**

¹H NMR (400 MHz, CDCl₃): δ 3.95 (t, J = 2.8 Hz, 1H), 3.56 (dd, J = 10.5, 3.4 Hz, 1H), 3.50 (td, J = 11.1, 5.5 Hz, 1H), 3.21 (dd, J = 10.5, 7.7 Hz, 1H), 1.95 - 1.70 (m, 6H), 1.66 - 1.22 (m, 16H), 1.19 - 1.10 (m, 2H), 1.07 (d, J = 6.6 Hz, 3H), 1.02 - 0.94 (m, 1H), 0.92 (s, 3H), 0.72 (s, 3H).

### Example 12

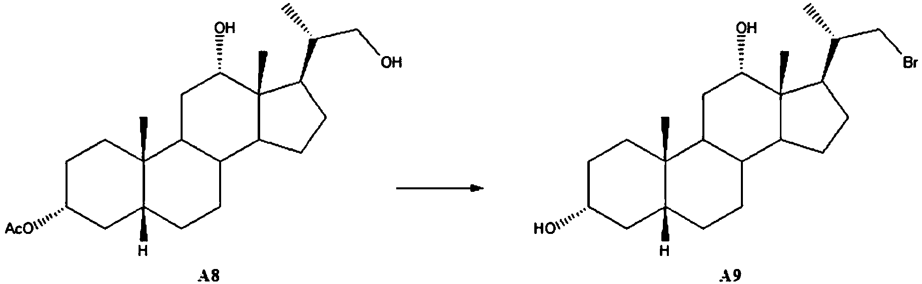

0.025 g of **A8** (0.078 mmol) was dissolved and stirred in DCM (2 ml). CBr₄ (2.4 eqv, 0.062 g, 0.09 mmol) and triphenylphosphine (PPh₃, 2.5 eqv, 0.051 g, 2.5 mmol) was added. The solution was heated under reflux. The mixture was stirred until the reaction was completed. The mixture was cooled at room temperature. The residue was purified on silica gel yielding 0.05 g of **A9.**

¹H NMR (400 MHz, CDCl₃): δ 3.93 (t, J = 2.8 Hz, 1H), 3.63 - 3.52 (m, 1H), 3.48 (dt, J = 5.9, 2.9 Hz, 1H), 3.29 (dd, J = 9.7, 6.5 Hz, 1H), 1.90 - 1.15 (m, 23H), 1.11 (d, J = 6.5 Hz, 3H), 0.94 (ddd, J = 12.6, 9.9, 2.5 Hz, 1H), 0.87 (s, 3H), 0.67 (s, 3H).

### Example 13

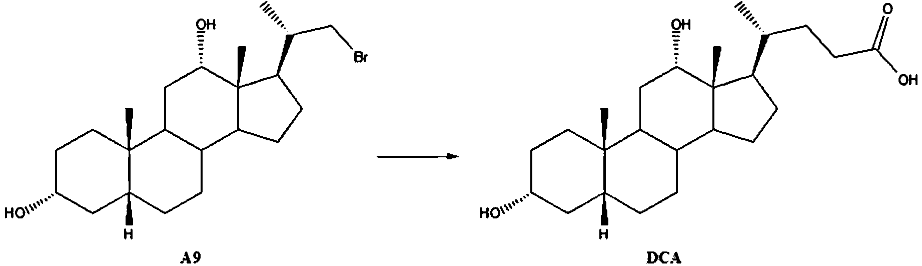

0.5 g of NaH 60% (12.5 mmol) was dissolved and stirred in dry DMF (10 ml) under an inert atmosphere. Diethyl malonate (2.0 g, 12.48 mmol) dissolved in 3.0 ml of DMF was added dropwise. The solution was heated and stirred until the mixture was turned clear. The mixture was cooled at 40°C. **A9** (5.12 g, 12.4 mmol) dissolved in 3.0 ml of DMF was added. The solution was heated at 60°C. The mixture was quenched by addition of water (15 ml). The aqueous phase was extracted with EtOAc. The solvent was evaporated under reduced pressure and the residue was suspended in an aqueous solution of KOH 2.8 M (10.0 ml). The solution was heated under reflux. Water (10 ml) was added and the organic solvent was evaporated under reduced pressure. The aqueous phase was acidified by adding 2N HCl and was extracted with EtOAc. The solvent was evaporated under reduced pressure and the residue was suspended in a mixture of dioxane (5 ml) and 12N HCl (10 ml). The mixture was heated under reflux for 24 hours. The mixture was cooled at room temperature and was extracted with EtOAc. The organic phases were mixed and evaporated under reduced pressure. The residue was purified on silica gel thereby yielding 3.1 g of **DCA.**

### Example 14

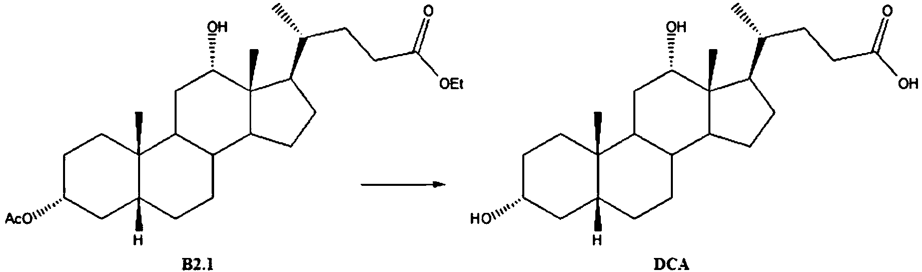

0.42 g of **B2.1** (0.92 mmol) was dissolved and stirred in THF (8 ml). Water (8 ml) was added and stirred at room temperature. A solution of LiOH 4M (2.0 ml) was added. The mixture was heated at 50°C and stirred overnight. The mixture was poured over water. The aqueous phase was extracted with EtOAc. The organic phase was washed with an aqueous solution of 2N HCl. The aqueous phase was extracted with EtOAc and the combined organic layers were evaporated under reduced pressure, thereby yielding 0.38 g of **DCA.** ¹H NMR (400 MHz, CDCl₃): δ 3.94 (t, J = 2.7 Hz, 1H), 3.58 - 3.45 (m, 1H), 2.41 - 2.11 (m, 2H), 1.99 - 1.71 (m, 7H), 1.67 - 1.04 (m, 19H), 0,99 (d, J = 6,4 Hz ,3H), 0.92 (s, 3H), 0.70 (s, 3H).

### Example 15

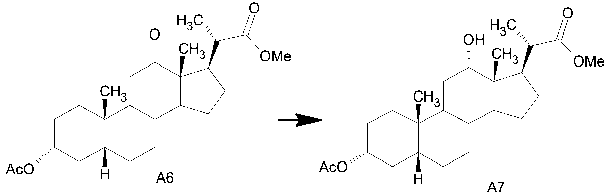

10.5 g of A6 (24.0 mmol) were dissolved and stirred in THF (137 ml) under inert atmosphere. The solution was cooled at -40°C. LiAl(OtBu)₃H (1.1 eq, 6.6 g, 26.0 mmol) was added dropwise (exothermic reaction). The mixture was stirred at -20°C until the reaction was complete. The solvent was evaporated under reduced pressure then the mixture was cooled at 0/5°C. The solid was filtered off, washed with water and dried, affording 10.55 g of compound A7.

### Example 16

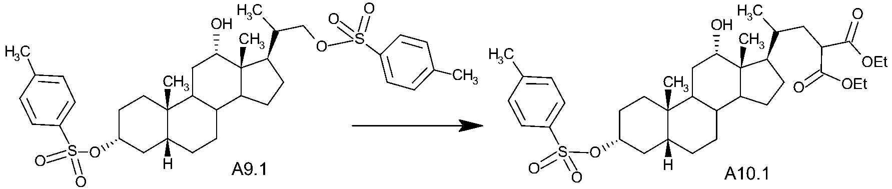

1.6 g of NaH 60 % (39.5 mmol) were dissolved and stirred in dry DMF (0.5 ml) under inert atmosphere. Diethyl malonate (6.2 ml, 4.02 mmol) dissolved in 0.5 ml of DMF were added dropwise. A suspension of the steroid intermediate (6.1 g, 9.8 mmol) in 1.0 ml DMF were added dropwise. The solution was heated and was stirred at 60°C. The mixture was quenched by adding water (15 ml). The aqueous phase was extracted with EtOAc. The solvent was evaporated under reduced pressure. The residue was purified by chromatography on silica gel.

### Example 17

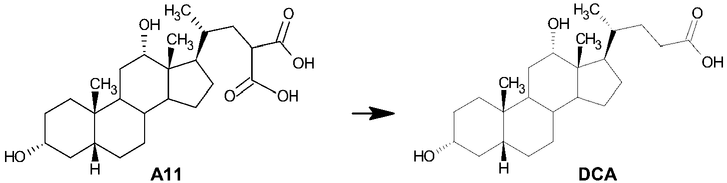

A11 (1.8 g, 4.12 mmol) were suspended in 108 ml of Xiylene. The suspension was heated under reflux. The mixture was slowly cooled at 20/25°C. Water (54 ml) and EtOAc (270 ml) were added. The aqueous phase was cooled at 10°C and acidified by adding HCl 2N. The mixture was stirred and the solid was filtered off, washed with water and dried, affording 1.04 g of Deoxycholic acid.

### Example 18

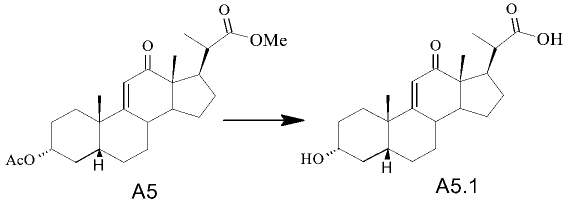

4.00 g of **A5** (9.6 mmol) was dissolved and stirred in MeOH (150 ml) under an inert atmosphere at room temperature. NaOH 20% (40 ml, 22 mmoles) was added. The solution was heated at reflux. The mixture was stirred until the reaction was complete (3 h). The mixture was cooled at room temperature. The solvent was evaporated and was quenched by adding an aqueous solution of HCl 6N until acidic pH. The precipitate was filtered off as a solid, washed with water and EtOH, and then dried under vacuum yielding 3.4 g (95%) of **A5.1.**

¹H-RMN (400 MHz, DMSO-d6) δ=0,57 (s, 3H); 0,81 (s, 3H); 1,09 (d, J= 6 Hz, 3H); 3,71 (m, 1H).

### Example 19

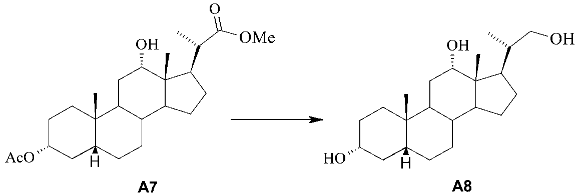

3.0 g of **A7** (7.14 mmol) was dissolved and stirred in THF (75 ml) under inert atmosphere. The solution was cooled at 0-5°C and LiAlH₄ (1.1 g, 28.6 mmol) was added slowly (exothermic reaction). The mixture was stirred at room temperature then heated under reflux until the reaction was complete. The mixture was cooled at room temperature and was hydrolyzed slowly with a solution of water (1.1 ml), NaOH (20%) 1.1 ml and water (3.3 ml). The white solid obtained was filtered off and was washed THF (150 ml). The organic phase was dried with anhydrous Magnesium sulfate. The solvent was evaporated under reduced pressure, yielding 2.3 g (92%) of **A8.**

### Example 20

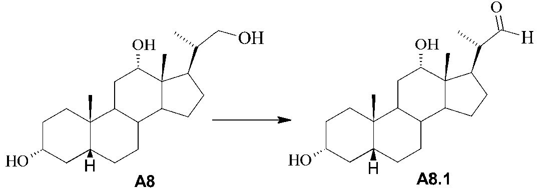

0.2 g of compound **A8** (0.57 mmol) was suspended in a mixture of DCM (6.0 ml) and ACN (6.0 ml) under inert atmosphere and then Dess-Martin Periodinane reagent (0.24 g, 0.57 mmol) and 4-methylmorpholine 4-oxide (11 mg, 0.07 mmol) were added. The suspension was stirred at room temperature until the reaction was complete. Then, a solution of Na₂S₂O₃ (1M, 50 mL) was added. The aqueous phase was extracted with DCM (3 x 50 ml) and then was washed with brine. The organic phase were concentrated until a solid was obtained, yielding 0.25 g of **A8.1.**

¹H NMR (400 MHz, CDCl₃): δ 9.7 (d, 1H); 9,5 (d, 1H); 3.6 (m, 1H); 3.4 (bs, 1H); 2.3 (qd, J= 9 Hz, 1H).

### Example 21

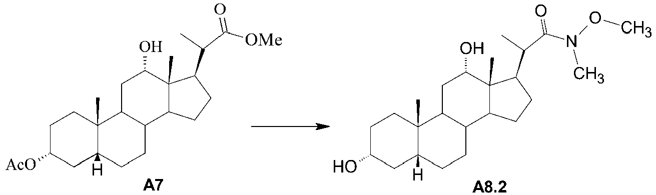

2.00 g of **A7** (4.76 mmol) was dissolved and stirred in MeOH (100 ml) under inert atmosphere at room temperature. NaOH 20% (20 ml) was added. The solution was heated at 80°C. The mixture was stirred until the reaction was complete (3 h). The mixture was cooled at room temperature. The solvent was evaporated and the mixture was quenched by adding an aqueous solution of HCl 6N until acidic pH. The precipitate was filtered off as a solid, washed with water and MeOH, and then dried under vacuum yielding 1.4 g (80%) of acid intermediate.

9.0 g of intermediate acid compound (25 mmol), DCC (6.2 g, 30 mmol), DMAP (3.7 g, 30 mmol) and *N*,O-dimethylhydroxylamine hydrochloride (4.9 g, 50 mmol) were dissolved in DCM (250 ml) under inert atmosphere. Et₃N (10 ml) was added and the suspension was stirred at room temperature until the reaction was complete. Then, the organic phase was washed with brine. The organic phase was concentrated until a solid was obtained. The solid was purified by column chromatography (AcOEt/Heptane), yielding 5.9 g of **A8.2.**

¹H NMR (400 MHz, CDCl₃): δ 3.9 (bs, 1H); 3.66 (s, 3H); 3.61 (m, 1H); 3.15 (s, 3H); 2.4 (q, J= 9 Hz, 1H).

### Example 22

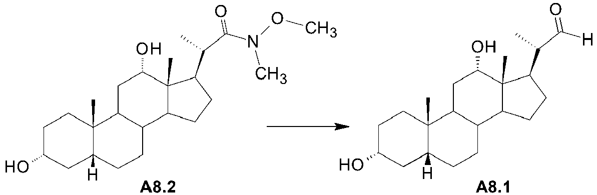

2.5 g of **A8.2** (6.14 mmol) was dissolved and stirred in THF (25 ml) under inert atmosphere. The solution was cooled at 0-5°C and was added slowly (exothermic reaction) to a solution of LiAlH₄ (0.36 g, 9.2 mmol) in THF (75 ml). The mixture was stirred at 0-5°C until the reaction was complete. The mixture was hydrolyzed slowly with a solution of water (0.25 ml), NaOH (20%) 0.25 ml and water (0.75 ml). The white solid obtained was filtered off and was washed THF (100 ml). The organic phase were concentrated and was purified by column chromatography (AcOEt/Heptane), yield 85% of **A8.2.**

### Example 23

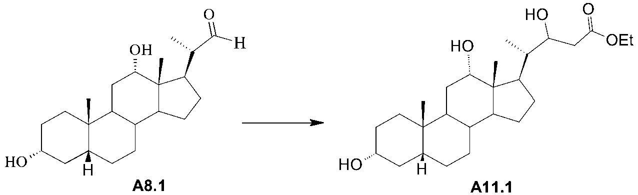

0.26 g of Zn (4.1 mmol) was suspended in THF under inert atmosphere. Trimethylchlorosilane (0.1 mmol) were added. The suspension was stirred at heated under reflux 1 hour. Then, a solution of 0.2 g of **A8.1** (0.57 mmol) and Ethyl Bromoacetate 0.3 ml (2.85 mmol) in THF (20 ml) were added. The mixture was stirred at heated under reflux until the reaction was complete. The mixture was cooled at room temperature. The solvent was evaporated and the mixture was quenched by adding an aqueous saturated solution of NH₄Cl (50 ml). EtOAc (75 ml) was added. The organic phase was washed with an aqueous saturated solution of NaCl. The organic phase were concentrated and the solid was purified by column chromatography (EtOAc/Heptane).

¹H NMR (400 MHz, CDCl₃): δ 4.35 (dt, J= 9 Hz, J= 3 Hz, 1H); 4.55 (q, J= 7.5 Hz, 2H); 3.35 (m, 1H); 2.7 (dd, J= 17 Hz, J= 9 Hz, 1H); 2.75 (dd, J= 17 Hz, J= 3 Hz, 1H).

### Example 24

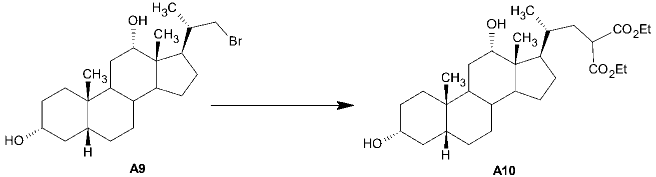

0.41 g of EtONa (0.60 mmol) was dissolved and stirred in EtOH (2 ml) under an inert atmosphere and was cooled at 0°C. Diethyl malonate (0.97 g, 0.60 mmol) was added to the mixture. The mixture was heated at room temperature and **A9** (0.20 g, 0.48 mmol) were added. The solution was heated at 90°C. The mixture was quenched by addition of water (5 ml). The aqueous phase was extracted with EtOAc. The organic phases were evaporated under reduced pressure. The residue was purified on silica gel thereby yielding 0.12 g of **A10.**

### Example 25

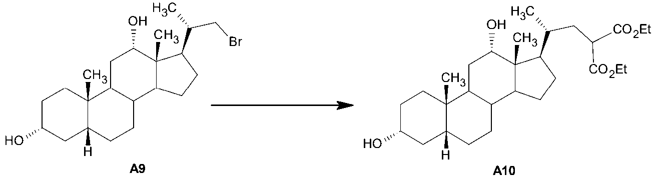

0.15 g of NaH 60% (3.87 mmol) was dissolved and stirred in DMF (2 ml) under an inert atmosphere and was cooled at 0°C. Diethyl malonate (0.59 ml, 3.87 mmol) was added to the mixture. The mixture was heated at room temperature and **A9** (0.40 g, 0.96 mmol) were added. The solution was heated at 60°C. The mixture was poured into a solution of NaCl 20% (30 ml). The aqueous phase was extracted with EtOAc. The organic phases were evaporated under reduced pressure. The residue was purified on silica gel thereby yielding 0.76 g of **A10.**

### Example 26

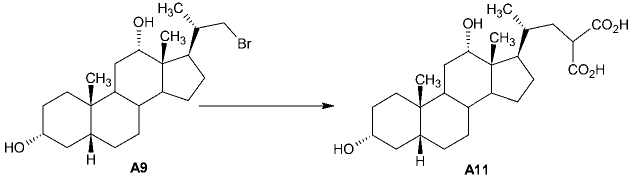

5.86 g of NaH 60% (146.4 mmol) was dissolved and stirred in dry DMF (75 ml) under an inert atmosphere cooled at 0°C. Diethyl malonate (23.4 g, 146.4 mmol) was added dropwise. The solution was stirred until the mixture was turned clear. **A9** (15.13 g, 36.6 mmol) dissolved in 75.0 ml of DMF was added. The solution was heated at 60°C. The mixture was quenched with a solution of NaCl 20% (1200 ml). The aqueous phase was extracted with EtOAc. The solvent was concentrated under reduced pressure and the mixture was cooled at room temperature and stirred until solid was precipitated. The solid was filtered off and dried, yielding 25.3 g of **A11.**

¹H NMR (400 MHz, CDCl₃): δ 3.95-3.93 (m, 1H), 3.57 - 3.46 (m, 1H), 3.39 (dd, J = 11.1, 3.5 Hz, 1H), 2.12 (t, J = 11.4 Hz, 1H), 1.96 - 1.04 (m, 25H), 1.00 (d, J = 7.5 Hz, 3H), 0.91 (s, 3H), 0.68 (s, 3H).

### Example 27

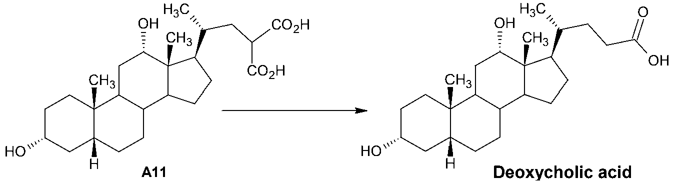

**A11** (3.0 g, 0.76 mmol) were suspended in 90.0 ml of NaCl 20%. The suspension was heated at reflux for 60 hours. The mixture was cooled to room temperature. The solid was filtered off and dried, yielding 2.48 g of **DCA.**

### Example 28

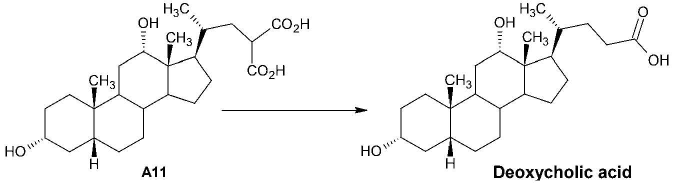

A11 (0.3 g, 0.076 mmol) were suspended in 9.0 ml of a solution of aqueous NaH₂PO₃ (pH 4.55). The suspension was heated at reflux for 70 hours. The mixture was cooled at room temperature. The solid was filtered off and dried, yielding 0.22 g of **DCA.**

### Example 29

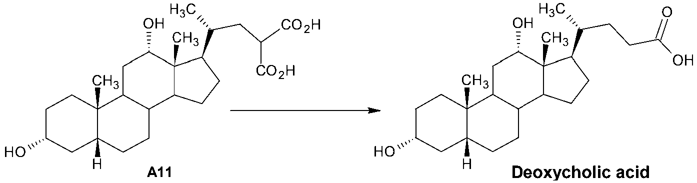

**A11** (0.3 g, 0.076 mmol) were suspended in 9.0 ml of water in a pressure vessel and was closed. The suspension was heated at reflux for 80 hours. The mixture was cooled to room temperature. The solid was filtered off and dried, yielding 0.23 g of **DCA.**

### Example 30

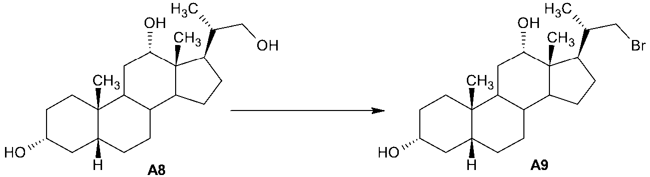

0.05 g of **A8** (0.14 mmol) was dissolved and stirred in ACN (1 ml), the mixture is cooled at 0°C. A solution of PPh₃Br₂ (0.105 g, 0.24 mmol) in ACN (1 ml) was added dropwise. The mixture was stirred until the reaction was completed. The mixture was cooled at room temperature. The residue was purified on silica gel yielding 0.04 g of **A9.**

### Example 31

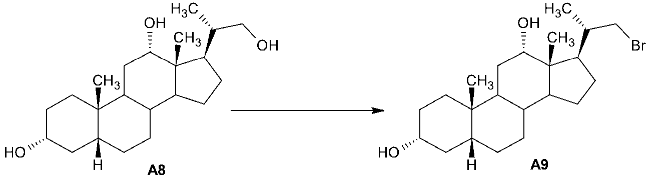

0.05 g of **A8** (0.14 mmol) was dissolved and stirred in DCM (2 ml), under an inert atmosphere and cooled to -40°C. 0.4 ml of a solution of TPP (0.077 g, 1.75 eq) in DCM (8 ml) was added dropwise. 0.4 ml of a solution of Br₂ (0.04 g, 1.75 eq) in DCM (8 ml) was added dropwise. The mixture was stirred until the reaction was completed. The mixture was allowed to reach room temperature. The residue was purified on silica gel yielding 0.05 g of **A9.**

### Example 32

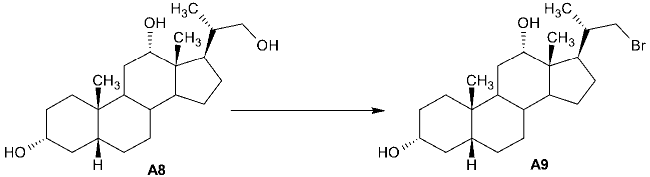

0.05 g of **A8** (0.14 mmol) was dissolved and stirred in ACN (1 ml), and cooled to 0°C. A solution of PPh₃Br₂ (0.105 g, 0.24 mmol) in ACN (1 ml) was added dropwise. The mixture was stirred until the reaction was completed. The mixture was warmed to room temperature. The residue was purified on silica gel yielding 0.04 g of **A9.**

### Example 33

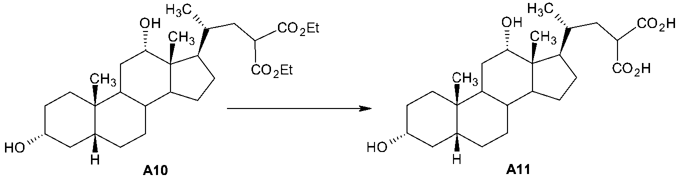

5.0 g of **A10** (10.15 mmol) was dissolved in EtOH (25 ml) and stirred at room temperature. NaOH 4M (40 ml) was added and the mixture was stirred. The organic solvent was concentrated under reduced pressure. Water (30 ml) wad added dropwise and a solid was obtained. The aqueous phase was washed with DCM (150 ml). The aqueous phase was acidified with HCl 2 N (until pH 1). The mixture was stirred at room temperature and a solid was obtained. The solid was filtered off and dried, yielding 3.5 g of **A11.**

### Example 34

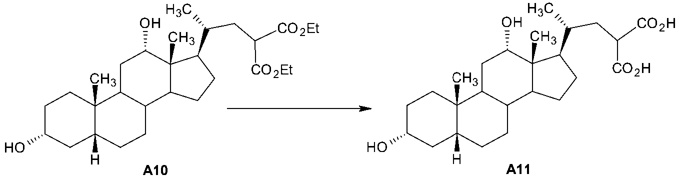

5.0 g of **A10** (10.15 mmol) was dissolved in EtOH (25 ml) and stirred at room temperature. LiOH 4M (40 ml) was added and the mixture was stirred at 40°C until the reaction was completed. The organic solvent was concentrated under reduced pressure. Water (500 ml) and DCM (150 ml) was added. The aqueous phase was separated and was acidified with HCl 2 N (until pH 1). The mixture was stirred at room temperature and a solid was obtained. The solid was filtered off and dried, yielding 4.3 g of **A11.**

### Example 35

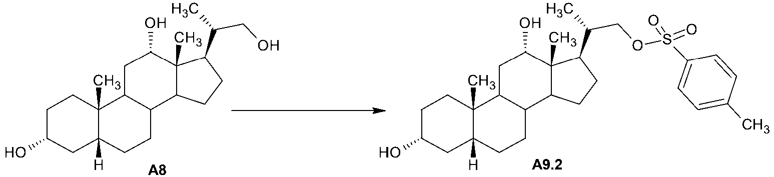

0.05 g of **A8** (0.14 mmol) was dissolved and stirred in DCM (1 ml), the mixture is cooled at 0°C. TsCl (0.05 g, 0.28 mmol) and DMAP (0.03 g, 0.28 mmol) were added. The suspension was stirred until the reaction was completed. The mixture was allowed to reach room temperature. The residue was purified on silica gel yielding 0.04 g of **A9.2.**

### Example 36: Preparation of diethyl malonic ester

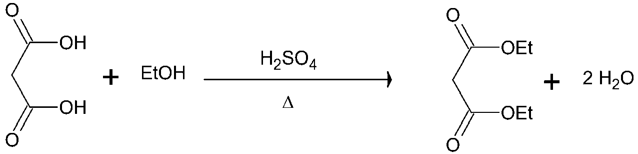

In a three-neck 250 mL round-bottom flask, malonic acid (20 g, 0.192 mol), absolute ethanol (70 mL, 1.2 mol) and concentrated sulphuric acid (0.8 mL, 0.08 mmol) were introduced. The round-bottom flask was equipped with a condenser to perform an azeotropic distillation at atmospheric pressure. The amount of ethanol that is removed through distillation is replaced with an ethanol vapor stream that was originated in a separated 500 mL round-bottom flask used as vaporizer. The ethanol/water vapors are evaporated and condensed at a rate of 50 mL/h.

The reaction conditions were unaltered during 7h. The reaction was followed through the quantification of the water amount detected by Karl-Fischer titration. The reaction is finished when the amount of water detected is similar to the original content of the absolute ethanol. The reaction was cooled to room temperature and 100 mL of a solution of sodium hydroxide 5% w/v was added (sodium carbonate can be used according the literature). The aqueous phase was extracted with methylene chloride (3 x 50 mL) and the joined organic phases were washed with 50 mL of water. The organic solution was rotoevaporated leading to 18.43 g (115 mmol) of ethyl malonate (60% molar yield). The water content of the final product is 0.06% and the absence of malonic acid was checked by the use of GC.

The IPC can be performed by the use of GC adding enough triethylamine to reach a neutral pH. The following GC method was used:
In-house GC method:
GC instrument: Agilent.
Injector: Split/Splitless
Column: HP-FFAP, length: 30 m, inner diameter: 0.32 mm, film: 0.25 µm.
Stationary phase: Nitroterephthalic acid modified polyethylene glycol.
Injection: Automatic/Manual
Detector: FID
Gas flow:
   - Nitrogen: Carrier (9 psi, 60 KPa)
   - Air: 300-400 mL/min
   - Hydrogen: 30-40 mL/min
   - Split-flow: 28 mL/min
   - Auxiliary gas (N2): 6.25 mL/min
   - Septum purge: 1.8 mL/min
GC temperature: Injector (250 °C); Detector (300 °C), Range: 3; Column temperature: 80 °C during 3 min, increasing temperature at a rate of 20 °C/min until 220 °C, hold during 1 minute.
   Injection volume: 1 µL
   Chromatogram time: 10 minutes

### Example 37: Preparation of biobased DCA from plant sources

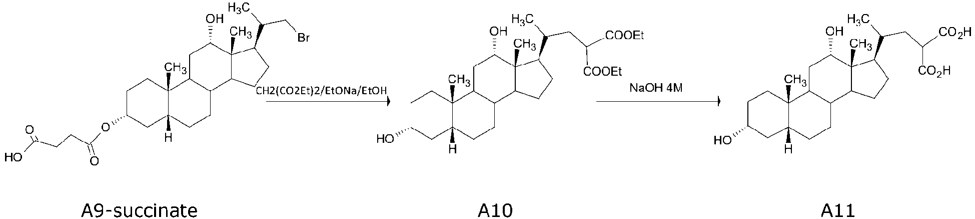

In a three-neck 250 mL round-bottom flask under inert atmosphere, 49 mL of absolute ethanol were introduced. It was followed by addition of sodium ethoxide (4.6 g, 68.2 mmol). Diethyl malonate was added dropwise (10.92 g, 10.4 ml, 68.2 mmol). The solution (or light suspension) was stirred during 30 min followed by the addition of A9-succinate (10.9 g, 68.2 mmol) and 35 mL of absolute ethanol. The suspension was heated under reflux overnight.

The in-process control (IPC) was performed after 7.5h using TLC silica plate and a mixture of toluene/acetonitrile 1/1. The reaction was finished and cooled down to 30 °C. A solution of sodium hydroxide 4 M was added dropwise during 15 min without reaching 50 °C. The mixture was heated at 40 °C during 2h. Alternatively, the reaction can be performed stirring overnight at room temperature.

Once the reaction is over, ethanol was distilled off at 150 mbar and 40 °C. Then, 350 mL of water were added. A formation of a white solid might be observed. In such a case these solids need to be removed through filtration under vacuum and washing the cake with 20 mL of water. The aqueous phase containing the product as a salt was washed three times with methylene chloride (210 mL). The removal of the total amount of methylene chloride is a critical parameter and therefore, a distillation at 150 mbar and 25 °C was performed. The solution was cooled down to 0/5 °C and 147 mL of a solution of sulphuric acid 2N were added dropwise during 10 minutes originating a white solid. The final pH is 1.7-2.0. The suspension was heated until 75 °C with a rate of 5 °C/10min. A 60/65 °C, a change in the solid morphology was noticed. Conditions were maintained during 20 minutes and the suspension was cooled down to 15 °C lasting one hour. The suspension was held at 15 °C during 30 minutes followed by filtration under vacuum. The cake was washed twice with 70 mL of water. The final yield of both steps is 73.7% molar.

The final product can be dried at 50 °C in a forced air drying oven overnight.

### DCA Synthesis:

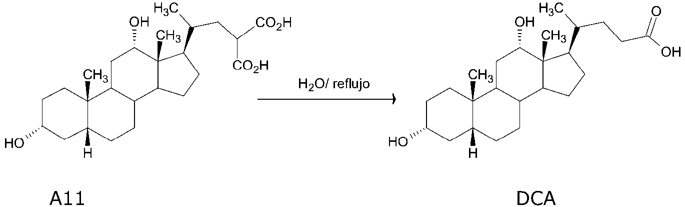

In a three-neck 250 mL round-bottom flask, 4.4 g of A11 (10 mmol) were suspended in 130 mL of water. The suspension was heated at reflux over 3 days.

Once the starting material has been turned into the final product, the suspension was cooled down to room temperature and filtered under vacuum. The wet cake was washed three times with 20 mL of water. The product was dried overnight at 50 °C in a forced air drying oven. Final yields are from 90 to 99% molar.

Alternatively, this reaction can be performed faster at higher temperature (130 °C) using as solvents *N*-methylpyrrolidone, *N,N*'-dimethylacetamide, *N,N'-*dimethylformamide, xylenes or diethyleneglycol monomethyl ether. With these solvents, the reaction is done between 1 and 3 hours. Then, 40 V of water can be added to precipitate the product. A TGA experiment performed over A11 suggested that the reaction can be carried out until 200 °C without other thermal events being involved.

Purification of the wet cake was done using 25 volumes of methylene chloride followed by addition of methanol until dissolution of the cake (usually 4-5 volumes). The solvent was removed by distillation at atmospheric pressure. 25 Volumes of methylene chloride were added again followed by distillation. This procedure was repeated three times. Finally, the removal of organic solvents requires suspension of the cake in 30 volumes of water, heating at reflux during 5/10 minutes at least, and cooling down to room temperature followed by filtration. The wet cake was washed twice with 5 volumes of water. The yield in the purification is 90%.

### Example 38: Analysis of biobased content of DCA from plant sources

The % Biobased Carbon Content was determined according to ASTM D6866-16 Method B (AMS) on the DCA product from Example 37. The result was obtained using the radiocarbon isotope (also known as Carbon-14, C14 or 14C), a naturally occurring isotope of carbon that is radioactive and decays in such a way that there is none left after about 45,000 years following the death of a plant or animal. An industrial application was also developed to determine if consumer products and CO₂ emissions were sourced from plants/biomass or from materials such as petroleum or coal (fossil-based). By 2003 there was growing demand for a standardized methodology for applying Carbon-14 testing within the regulatory environment. The first of these standards was ASTM D6866-04, which was written with the assistance of Beta Analytic. Since ASTM was largely viewed as a US standard, European stakeholders soon began demanding an equivalent CEN standard while global stakeholders called for ISO standardization.

The analytical procedures for measuring radiocarbon content using the different standards are identical. The only difference is the reporting format. Results are usually reported using the standardized terminology "% biobased carbon". Only ASTM D6866 uses the term "% biogenic carbon" when the result represents all carbon present (Total Carbon) rather than just the organic carbon (Total Organic Carbon).

The result was obtained by measuring the ratio of radiocarbon in the material relative to a National Institute of Standards and Technology (NIST) modern reference standard (SRM 4990C). This ratio was calculated as a percentage and is reported as percent modern carbon (pMC). The value obtained relative to the NIST standard is normalized to the year 1950 AD so an adjustment was required to calculate a carbon source value relative to today. This factor is listed on the report sheet as the terminology "REF".

A value of 100% biobased or biogenic carbon would indicate that 100% of the carbon came from plants or animal by-products (biomass) living in the natural environment and a value of 0% would mean that all of the carbon was derived from petrochemicals, coal and other fossil sources. A value between 0-100% would indicate a mixture. The higher the value, the greater the proportion of naturally sourced components in the material.

The analytical measurement is cited as "percent modern carbon (pMC)". This is the percentage of C14 measured in the sample relative to a modern reference standard (NIST 4990C). The "% Biobased Carbon" is calculated from pMC by applying a small adjustment factor for C14 in carbon dioxide in air today. $" % Biobased Carbon " = pMC / 1.010$

Reported results are accredited to ISO/IEC 17025:2005 Testing Accreditation PJLA #59423 standards and all chemistry was performed at Beta Analytic, Inc in Miami, Florida.

| | |
|---|---|
| *Result:* | 100 % Biobased Carbon Content (as a fractionof total organic carbon) |
| *Percent modern carbon (pMC):* | 103.39 +/- 0.29 pMC |
| *Atmospheric adjustment factor (REF):* | 101.0; = pMC/1.010 |

### Example 39: Analysis if δ¹³C content of DCA from animal, synthetic and plant sources

DCA samples being of animal origin and DCA having a phytosterol origin i.e. derived from plant sources were measured using IRMS (isotope ratio mass spectrometry) and according to the method described in in US 8,076,156 and "Stable Isotope Ratios as biomarkers of diet for health research" by D.M. O'Brien, Annual Reviews (www.annualreviews.org), 2015. The δ-value appears as the C13 is measured in relation to a standard being Pee Dee Belemnite based on a Cretaceous marine fossil, which had an anomalously high C13.

Values are expressed as a per mil (%) deviation, e.g. per one thousand, from an internationally accepted PDB standard (a carbonate from the Pee Dee Belemnite formation in South Carolina).

| **Sample No.** | **DCA** | **δ¹³C PDB ‰** (± 0.1‰) |
|---|---|---|
| 1 | Animal origin | -12 |
| 2 | Animal origin | -12.6 |
| 3 | Animal origin | -12.7 |
| 4 | Animal origin | -12.6 |
| 5 | Animal origin | -13.4 |
| 6 | Animal origin | -13.2 |
| 7 | Plant source (phytosterol) | -26.6 |
| 8 | Plant source (phytosterol) | -27.1 |
| 9 | Plant source (phytosterol) | -27.2 |
| 10 | Plant source (phytosterol) | -29.7 |
| 11 | Combined plant source and synthetic source | -29.8 |
| 12 | Combined plant source and synthetic source | -29.7 |
| 13 | Combined plant source and synthetic source | -29.8 |
| 14 | Combined plant source and synthetic source | -29.8 |
| 15 | Combined plant source and synthetic source | -29.8 |

The results described in the table above demonstrate results obtained by measurement on separate samples. The DCA samples measures upon are as follows:
Animal origin is DCA purified from animal sources i.e. DCA as traditionally obtained.
Plant source (phytosterol) is DCA produced starting from phytosterol from plants and produced according to the present invention, where the additional carbon atoms added are derived solely from plant sources.
Combined plant source and synthetic source: The DCA contains two carbon atoms from a synthetic source (fossil derived starting material) and the remaining carbon atoms from plant sources being produced from a phytosterol starting material. The DCA is prepared as described herein but using diethyl/dimethyl malonate, acetic acid and acetonitrile from a synthetic source.

These results show that the origin of the DCA may be differentiated by whether it is obtained from animals or partially or solely from plant sources.

### Example 40: Measurement of Biobased Carbon Content and δ¹³C content of DCA

The biobased carbon content measured as 14C was measured in DCA samples from different sources according to the method as described in Example 38 providing the following measurements: ${" % Biobased Carbon " = pMC / 1.010}_{¯}$
Percent modern carbon (pMC)
Atmospheric adjustment factor (REF).

Reported results are accredited to ISO/IEC 17025:2005 Testing Accreditation PJLA #59423 standards and all chemistry was performed at Beta Analytic, Inc in Miami, Florida.

The same samples were measured for their δ¹³C content as described in Example 39.

The tested DCA were obtained from the following sources:
Animal origin is DCA purified from animal sources i.e. DCA as traditionally obtained.
Plant source (phytosterol) is DCA produced starting from phytosterol from plants and produced according to the present invention, where the additional carbon atoms added are derived solely from plant sources.
Combined plant source and synthetic source: The DCA contains two carbon atoms from a synthetic source (fossil derived starting material) and the remaining carbon atoms from plant sources being produced from a phytosterol starting material. The DCA is prepared as described herein but using diethyl/dimethyl malonate, acetic acid and acetonitrile from a synthetic source.

| **Sample No** | **DCA** | **%Biobased** (± 3%) | **pMC** | **REF** | δ**13C** (±‰) |
|---|---|---|---|---|---|
| 3 | From animal origin | 100% | 104.85+/- 0.31 | 101.0 | -12.1 |
| 9 | From plant source (phytosterol) | 100% | 103.39+/- 0.29 | 101.0 | -27.1 |
| 10 | From plant source (phytosterol) | 100% | 104.71+/- 0.24 | 100.5 | -29.7 |
| 15 | Combined plant source and synthetic source | 95% | 95.95 +/-0.23 | 100.5 | -29.8 |

The table illustrates that DCA derived from phytosterol and solely from plant sources holds a biobased level of 100% and a δ13C of -27.1‰ or -29.7‰. DCA from animal origin is also 100% biobased but in contrast the δ¹³C is considerably higher i.e. -12.1‰.

When combining DCA from plant source of the starting material and synthetic source reagent the biobased level decreases due to the lower level from the synthetic source but δ¹³C is around the same level as when the DCA is obtained from plant sources.

Accordingly, the measurement of both the % of biobased carbon atoms and the level of δ13C enables one to differentiate between DCA from different sources.

### Example 41: Measurement of protein content using Bradford assay

The Bradford assay (Bradford MM.: A a rapid and sensitive method for the quantitation of microgram quantities of protein utilizing the principle of protein-dye binding. Anal Biochem 1976; 72:248-54) is a fast and accurate method recommended for general use, especially used to determine the total protein concentration of a sample. The Bradford assay has become the preferred method for quantifying protein in many laboratories. The Bradford assay relies on the binding of the dye Coomassie Blue G250 to protein and the observation that the absorbance maximum for an acidic solution of Coomassie Brilliant Blue G-250 shifts from 465 nm to 595 nm when binding to protein occurs. The assay is useful since the extinction coefficient of a dye-albumin complex solution is constant over a 10-fold concentration range.

### Method:

The presence of proteins was determined by Bradford, a colorimetric protein assay. This method involves the binding of Coomassie Brilliant Blue G-250 dye to proteins. Under acidic conditions, the dye is predominantly in the doubly protonated red cationic form (Amax= 470 nm). However, when the dye binds to protein, it is converted to a stable unprotonated blue form (Amax= 595 nm). Therefore, the appearance of blue color, measured with a spectrophotometer, indicates the presence of proteins.

### Sample preparation:

To a 1 g of sample was added 1 mL of water. The mixture was shacked and sonicated for 20 minutes. Then, the mixture was settled for 10 minutes. The upper liquid was analysed by Bradford test.

Bovine serum albumin (BSA), a serum albumin protein derived from cows, often used as a protein concentration standard, was spiked with known amount of 2.5 µg (10 µL of a matrix solution of 0.25 µg/µL) as a positive control in some of the problem sample.

The calibrated curve was carried out by means of different concentration of BSA on the range from 0.5 to 10 µg.

### Bradford assay:

The assay was done according the standard procedure (Bradford, 1976) using different amounts of the problem samples. 3 measurements were done with every sample.
i) 1 µL of sample
ii) 5 µL of sample
iii) Positive control: 5 µL of sample plus 2.5 mg of BSA.

Bradford method results for determination of presence/absence of proteins:

| **Sample No** | **DCA** | **Result** |
|---|---|---|
| 14 | Combined plant source and synthetic source | Negative |
| 15 | Combined plant source and synthetic source | Negative |
| 10 | From plant source (phytosterol) | Negative |
| 16 | From animal origin | Negative |

Negative means that the potential amount of proteins, if there are something, would in any event be below the *de minimis* threshold of detection of the apparatus.

The tested DCA were obtained from the following sources:
Animal origin is DCA purified from animal sources i.e. DCA as traditionally obtained.
Plant source (phytosterol) is DCA produced starting from phytosterol from plants and produced according to the present invention, where the additional carbon atoms added are derived solely from plant sources.
Combined plant source and synthetic source: The DCA contains two carbon atoms from a synthetic source (fossil derived starting material) and the remaining carbon atoms from plant sources being produced from a phytosterol starting material. The DCA is prepared as described herein but using diethyl/dimethyl malonate, acetic acid and acetonitrile from a synthetic source.

The Bradford method is a common technique to quantify proteins. However, the Bradford method does not detect any proteins in any of the DCA samples either being of animal origin, from plant source and derived according to this invention or as a DCA being combined plant source and synthetic source. Thus, the Bradford method cannot be used for distinguishing DCA from different sources like differentiating on the level of 14C and δ13C as described herein.

### Embodiments of the invention

**A.** A process for the preparation of deoxycholic acid (DCA) or a pharmaceutically acceptable salt thereof, comprising the following steps:
   I) providing a compound of the general formula **SM:** 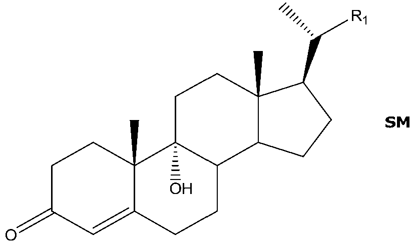
   II) reducing the compound of the general formula **SM** to obtain an intermediate of the general formula **INT 1:** 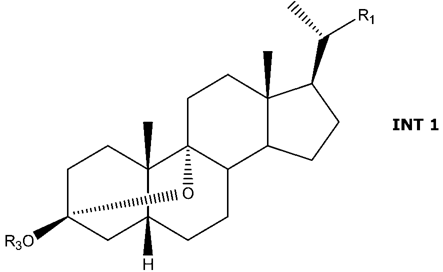
   III) converting the intermediate of the general formula **INT 1** into an intermediate of the general formula **INT 2:** 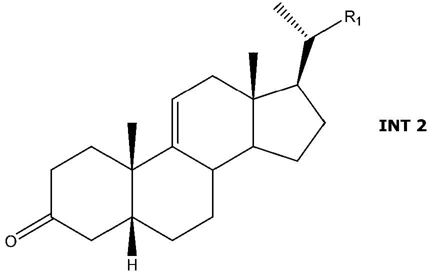
   IVa) reducing the intermediate of the general formula **INT 2** into an intermediate of the general formula **INT 3:** 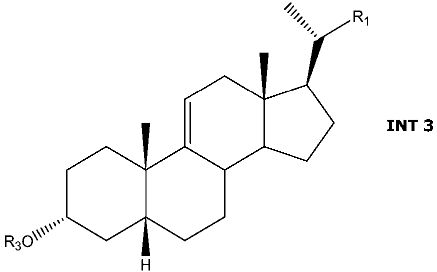 followed by converting the intermediate of the general formula **INT 3** into an intermediate of the general formula **INT B:** 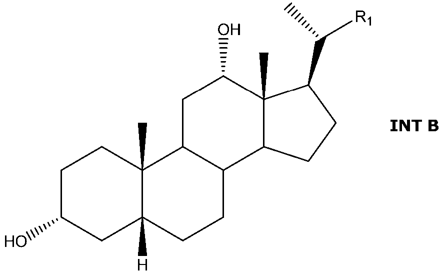 or
   IVb) converting the intermediate of the general formula **INT 2** into an intermediate with the general formula **INT B:** 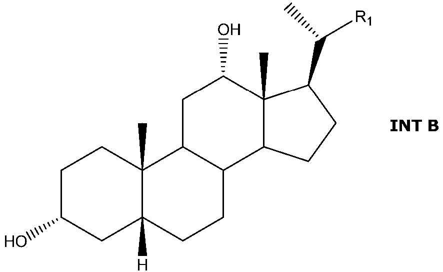
   V) converting the intermediate of the general formula **INT B** into deoxycholic acid (DCA): 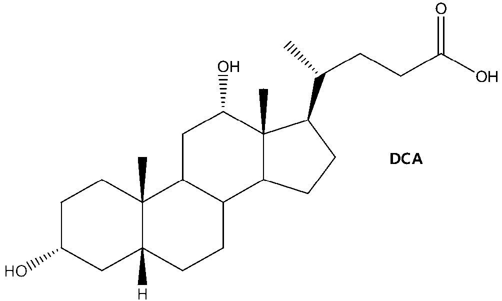
   VI) optionally converting deoxycholic acid to a pharmaceutically acceptable salt thereof,
      wherein
      R₁ is COOR₂, CH₂OH, CH₂OP, CH₂X, CH₂CHO, CH₂-CH₂-OH, CH₂-CH₂OP, or CH₂-CH₂X or CH₂-CH₂-CHO;
      R₂ is H or a linear or branched C₁-C₆-alkyl group;
      P is an alcohol protection group;
      R₃ either P or R₂; and
      X is a halogen atom.
**B.** The process according to embodiment A, comprising the following steps:
   i) providing a compound of the general formula **SM-a:** 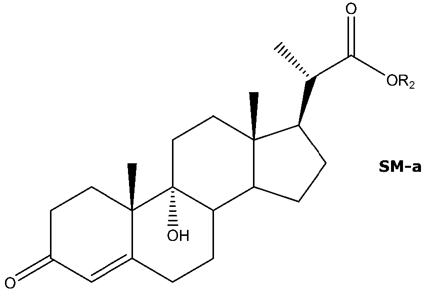
   ii) reducing the compound of the general formula **SM-a** to obtain an intermediate of the general formula **Int A1:** 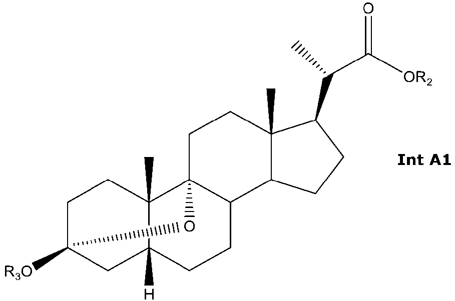
   iii) converting the intermediate of the general formula **Int A1** into an intermediate of the general formula **Int A2:** 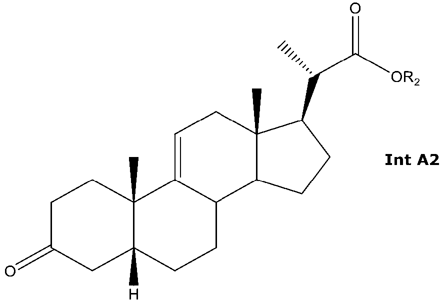
   iv) reducing the intermediate of the general formula **Int A2** into an intermediate of the general formula **Int A3:** 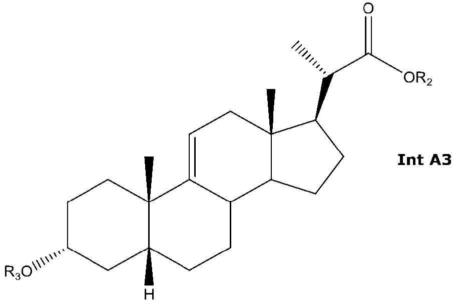
   v) oxidising the intermediate of the general formula **Int A3** into an intermediate of the general formula **Int A5:** 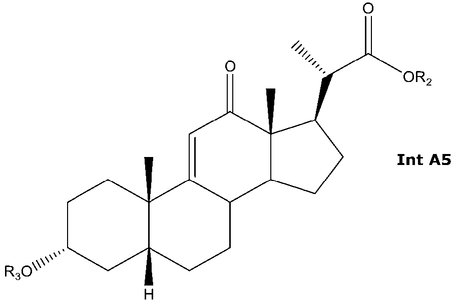
   vi) reducing the intermediate of the general formula **Int A5** into an intermediate of the general formula **Int A6:** 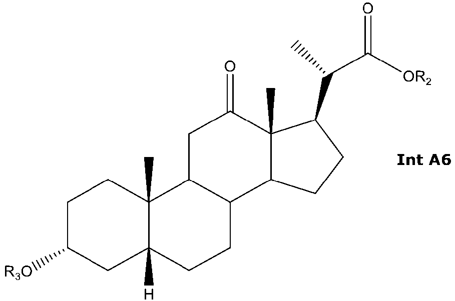
   vii) reducing the intermediate of the general formula **Int A6** into an intermediate of the general formula **Int A7:** 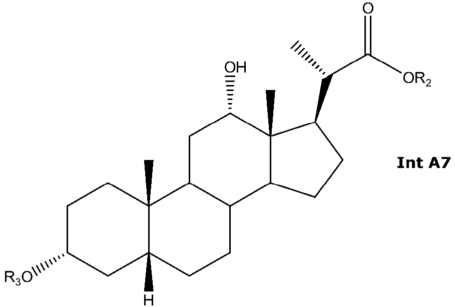
   viii) reducing the compound of the general formula **Int A7** into an intermediate of the general formula **Int A8:** 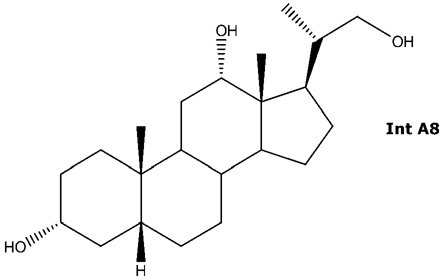
   ix) elongating the carbon chain of the compound of the general formula **Int A8** to obtain deoxycholic acid (DCA): 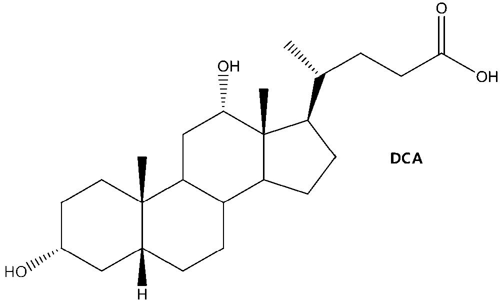
   x) optionally converting deoxycholic acid to a pharmaceutically acceptable salt thereof,
      wherein
      R₂ is H or a linear or branched C₁-C₆-alkyl group;
      R₃ is H, R₂ or an alcohol protection group.
**C.** The process according to embodiment A or B, wherein R₂ is selected from the group consisting of methyl, ethyl, n-propyl and isopropyl.
**D.** The process according to embodiment C, wherein R₂ is methyl or ethyl.
**E.** The process according to embodiment D, wherein R₂ is methyl.
**F.** The process according to any of the preceding embodiments, wherein R₃ is selected from the group consisting of trimethylsilyl ether (TMS), triethylsilyl ether (TES), triisopropylsilyl ether (TIPS), tert-butyldimethylsilyl ether (TBS, TBDMS), tert-butyldiphenylsilyl ether (TBDPS), acettyl (Ac, COCH₃), benzoyl (Bz), benzyl ether (Bn), 4-methoxybenzyl ether (PMB), 2-naphthylmethyl ether (Nap), methoxymethyl acetal (MOM), 2-methoxyethoxy-methyl ether (MEM), ethoxyethyl acetal (EE), methoxypropyl acetal (MOP), benzyloxymethyl acetal (BOM), tetrahydropyranyl acetal (THP), 2,2,2-trichloro-ethyl carbonate (Troc), methyl ether, dimethoxytrityl (DMT), methoxytrityl (MMT), methylthiomethyl ether, pivaloyl (Piv), tetrahydropyranyl (THP), triphenylmethyl (trityl, Tr), and tosyl (Ts).
**G.** The process according to embodiment F, wherein R₃ is selected from the group consisting of Ac, TBDMS and Ts.
**H.** The process according to embodiment G, wherein R₃ is Ac.
**I.** The process according to embodiment A or B, wherein R₂ is methyl and R₃ is Ac.
**J.** The process according to embodiment J, wherein R₃ is R₂, and R₂ is as defined in any of embodiments B-D.
**K.** A process for the preparation of deoxycholic acid (DCA) or a pharmaceutically acceptable salt thereof, comprising the following steps:
   I) providing a compound of the general formula **INT 3:** 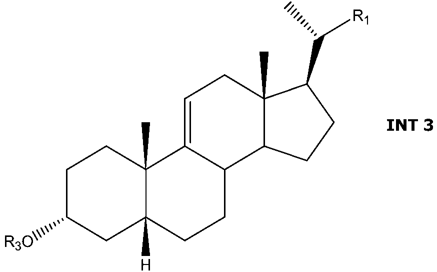
   II) converting the intermediate of the general formula **INT 3** into an intermediate of the general formula **INT B:** 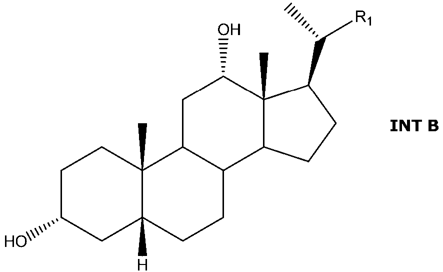
   III) converting the intermediate of the general formula **INT B** into deoxycholic acid (DCA): 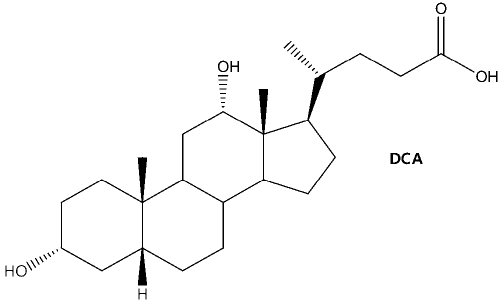
   IV) optionally converting deoxycholic acid to a pharmaceutically acceptable salt thereof,
      wherein
      R₁ is COOR₂, CH₂OH, CH₂OP, CH₂X, CH₂CHO, CH₂-CH₂-OH, CH₂-CH₂OP, or CH₂-CH₂X or CH₂-CH₂-CHO;
      R₂ is H or a linear or branched C₁-C₆-alkyl group;
      P is an alcohol protection group;
      R₃ either P or R₂; and
      X is a halogen atom.
**L.** The process according to embodiment K, wherein **INT 3** is provided from **INT 2** as defined in step IVa) of embodiment A.
**M.** The process according to embodiment L, wherein **INT 2** is provided from **INT** 1 as defined in step III) of embodiment A.
**N.** The process according to embodiment M, wherein INT 1 is obtained from SM as defined in step II) of embodiment A.
**O.** The process according to embodiment K, comprising the following steps:
   i) providing a compound of the general formula **Int A3:** 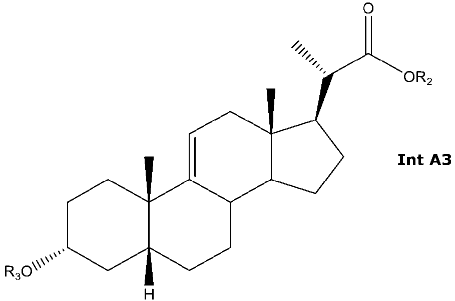
   ii) oxidising the intermediate of the general formula **Int A3** into an intermediate of the general formula **Int A5:** 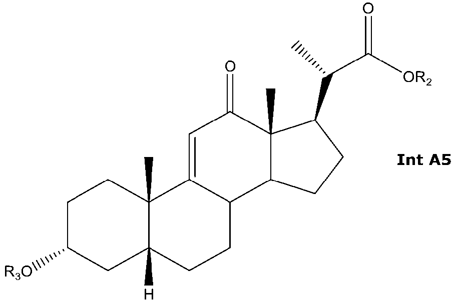
   iii) reducing the intermediate of the general formula **Int A5** into an intermediate of the general formula **Int A6:** 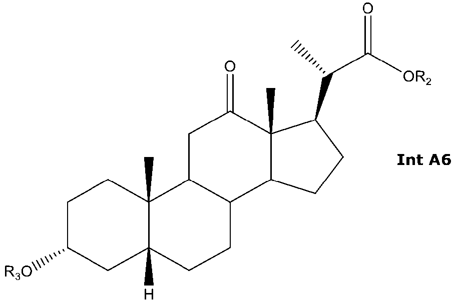
   iv) reducing the intermediate of the general formula **Int A6** into an intermediate of the general formula **Int A7:** 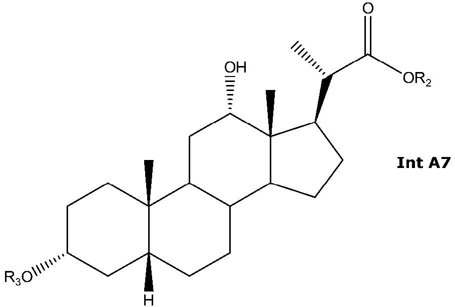
   v) reducing the compound of the general formula **Int A7** into an intermediate of the general formula **Int A8:** 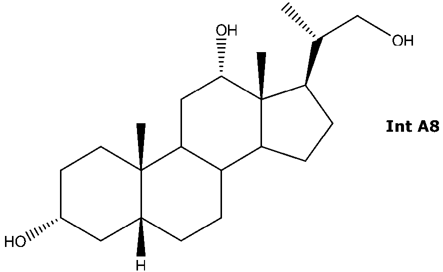
   vi) elongating the carbon chain of the compound of the general formula **Int A8** to obtain deoxycholic acid (DCA): 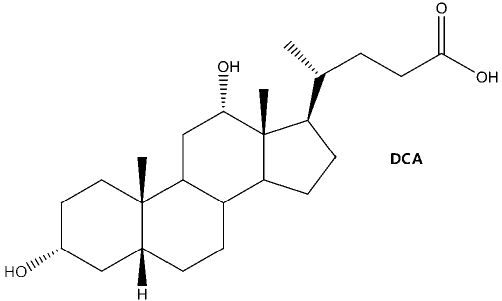
   vii) optionally converting deoxycholic acid to a pharmaceutically acceptable salt thereof,
      wherein
      R₂ is H or a linear or branched C₁-C₆-alkyl group;
      R₃ is H, R₂ or an alcohol protection group.
**P.** The process according to embodiment O, wherein **INT A3** is provided from **INT A2** as defined in step iv) of embodiment B.
**Q.** The process according to embodiment P, wherein **INT A2** is provided from **INT A1** as defined in step iii) of embodiment B.
**R.** The process according to embodiment Q, wherein **INT A1** is provided from **SM-a** as defined in step ii) of embodiment B.
**S.** The process according to any of embodiments K-R, wherein R₂ and R₃ are a defined in any of embodiments B-J.
**T.** A compound of the general formula I 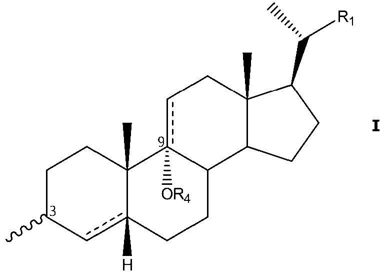 wherein
   R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
   R₂ is H or a linear or branched C₁-C₆-alkyl group;
   P is an alcohol protection group;
   X is a halogen atom;
   -̅ -̅ -̅ -̅ -̅ -̅ is either a C-C bond or a C=C bond;
   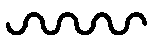 is either =O or 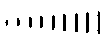 OR₃ where R₃ is either P or R₂;
   OR₄ is either OH or R₄ is the C3 carbon in the A ring; and
   with the proviso that formula **I** is not 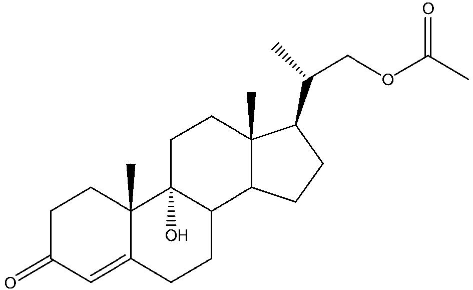 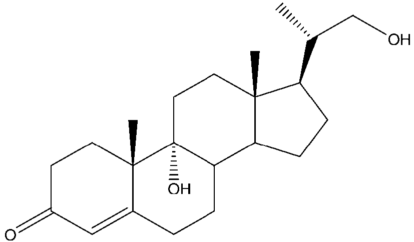 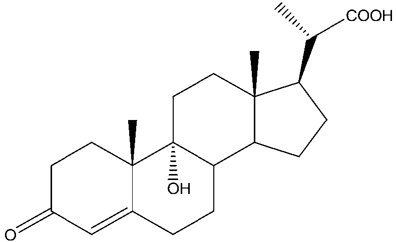 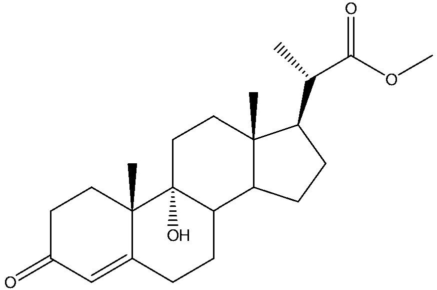 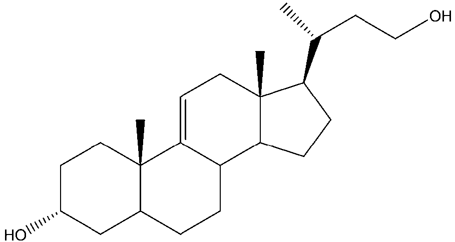 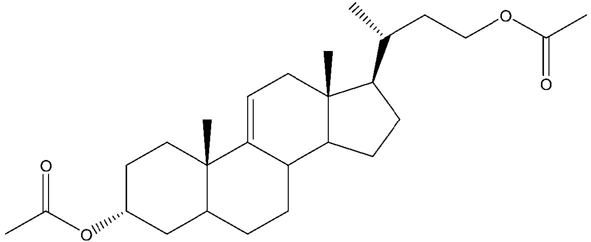 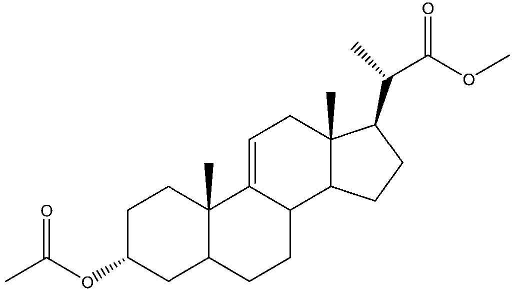
**T1.** The compound of embodiment T, wherein
   R₁ is COOR₂, CH₂OP, CH₂X, CH₂CHO, CH₂-CH₂-OH, CH₂-CH₂OP or CH₂-CH₂X;
      P is an alcohol protection group with the proviso that P is not Ac or Pv;
   with the proviso that formula **I** is not 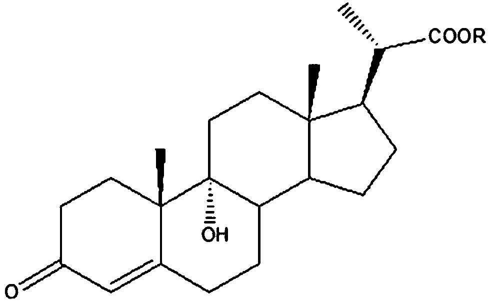
   Wherein R is H or Me 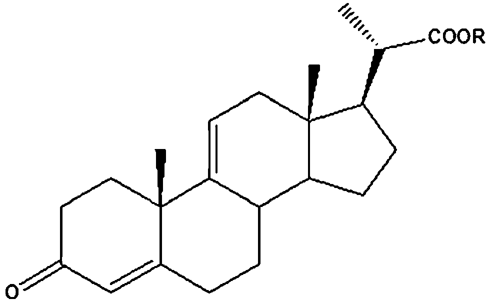
   Wherein R is H or Me 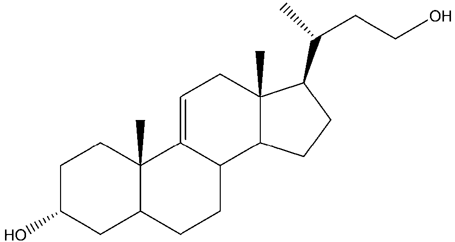 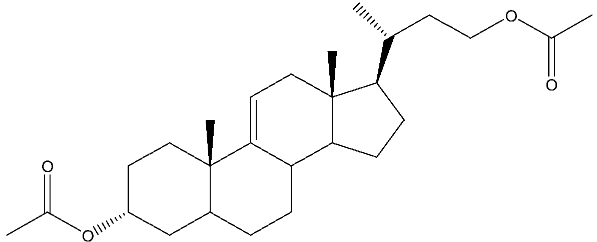 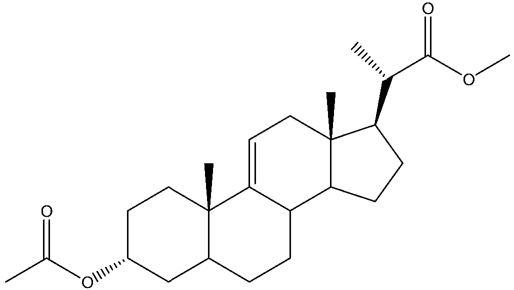
**U.** The compound according to embodiment T or T1 having the general formula **SM** 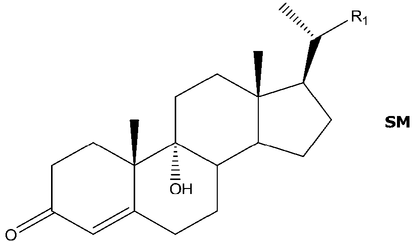 wherein
   R₁ is COOR₂, CH₂OP, CH₂X, CH₂CHO, CH₂-CH₂-OH, CH₂-CH₂OP, or CH₂-CH₂X;
   R₂ is a linear or branched C₁-C₆-alkyl group with the proviso that R₂ is not CH₃;
   P is an alcohol protection group with the proviso that P is not Ac; and
   X is a halogen atom.
**V.** The compound according to embodiment T or T1 having the general formula 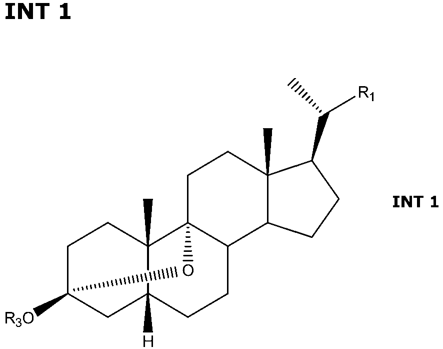 wherein
   R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
   R₂ is H or a linear or branched C₁-C₆-alkyl group;
   P is an alcohol protection group;
   R₃ is either P or R₂; and
   X is a halogen atom.
**W.** The compound according to embodiment T or T1 having the general formula 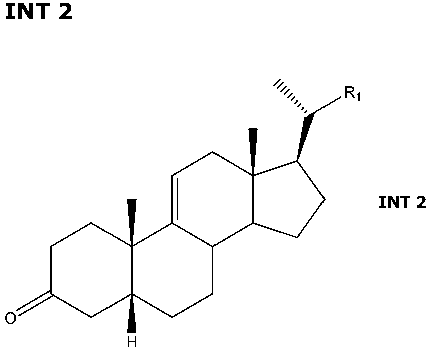 wherein
   R₁ is COOR₂, CH₂OP, CH₂X, CH₂CHO, CH₂-CH₂-OH, CH₂-CH₂OP or CH₂-CH₂X;
   R₂ is H or a linear or branched C₁-C₆-alkyl group;
   P is an alcohol protection group; and
   X is a halogen atom.
**X.** The compound according to embodiment T or T1 having the general formula 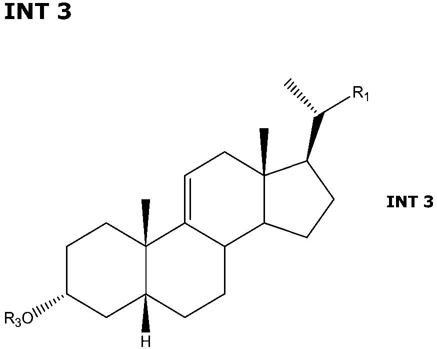 wherein
   R₁ is COOR₂, CH₂OP, CH₂X, CH₂CHO, CH₂-CH₂-OH, CH₂-CH₂OP or CH₂-CH₂X;
   R₂ is H or a linear or branched C₁-C₆-alkyl group;
   P is an alcohol protection group;
   R₃ is either P or R₂; and
   X is a halogen atom.
**Y.** The compound according to any of embodiments U-X, wherein R₁ is COOR₂, , CH₂OP or CH₂X, where
   R₂ is H or a linear or branched C₁-C₆-alkyl group;
   P is an alcohol protection group;
   R₃ is either P or R₂; and
   X is a halogen atom.
**Z.** The compound according to any of embodiments U-Y, wherein R₂ is selected from the group consisting of methyl, ethyl, n-propyl and isopropyl.
**AA.** The compound according to embodiment Z, wherein R₂ is methyl or ethyl.
**AB.** The compound according to embodiment AA, wherein R₂ is methyl.
**AC.** The compound according to embodiment AA, wherein R₂ is ethyl.
**AD.** The compound according to any of embodiments U-Y, wherein R₂ is H.
**AE.** The compound according to any of embodiments U-Y, wherein X is selected from the group consisting of Cl, Br and I.
**AF.** The compound according to embodiment AE, wherein X is Br.
**AG.** The compound according to any of embodiments U-Y, wherein P is selected from the group consisting of trimethylsilyl ether (TMS), triethylsilyl ether (TES), triisopropylsilyl ether (TIPS), tert-butyldimethylsilyl ether (TBS, TBDMS), tert-butyldiphenylsilyl ether (TBDPS), acettyl (Ac, COCH₃), benzoyl (Bz), benzyl ether (Bn), 4-methoxybenzyl ether (PMB), 2-naphthylmethyl ether (Nap), methoxymethyl acetal (MOM), 2-methoxyethoxy-methyl ether (MEM), ethoxyethyl acetal (EE), methoxypropyl acetal (MOP), benzyloxymethyl acetal (BOM), tetrahydropyranyl acetal (THP), 2,2,2-trichloro-ethyl carbonate (Troc), methyl ether, dimethoxytrityl (DMT), methoxytrityl (MMT), methylthiomethyl ether, pivaloyl (Piv), tetrahydropyranyl (THP), triphenylmethyl (trityl, Tr), and tosyl (Ts).
**AH.** The compound according to embodiment AG, wherein P is selected from the group consisting of Ac, TBDMS and Ts.
**AI.** The compound according to any of embodiments U-AH, wherein R₃ is H.
**AJ.** The compound according to any of embodiments U-AH, wherein R₃ is R₂, and R₂ is as defined in any of embodiments Z-AC.
**AK.** The compound according to any of embodiments U-AH, wherein R₃ is P, and P is as defined in embodiment AG or AH.
**AL.** The compound according to embodiment AK, wherein R₃ is Ac.
**AM.** Use of a compound of the general formula I 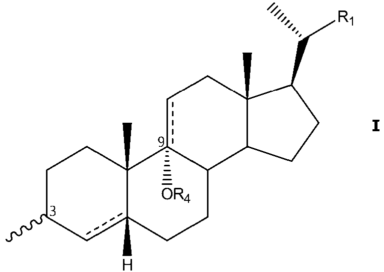 wherein
   R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
   R₂ is H or a linear or branched C₁-C₆-alkyl group;
   P is an alcohol protection group;
   X is a halogen atom;
   -̅ -̅ -̅ -̅ -̅ -̅ is either a C-C bond or a C=C bond;
   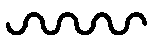 is either =O or 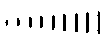 OR₃ where R₃ is either P or R₂; and
   OR₄ is either OH or R₄ is the C3 carbon in the A ring;
   for the preparation of deoxycholic acid (DCA), cholic acid, glycocholic acid, taurocholic acid, or a pharmaceutically acceptable salt thereof.
**AM1.** Use of a compound of the general formula I 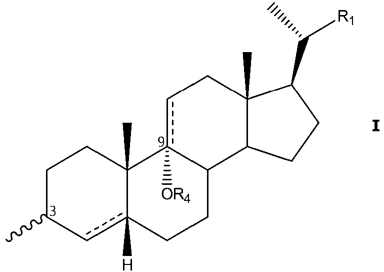 wherein
   R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
   R₂ is H or a linear or branched C₁-C₆-alkyl group;
   P is an alcohol protection group;
   X is a halogen atom;
   -̅ -̅ -̅ -̅ -̅ -̅ is either a C-C bond or a C=C bond;
   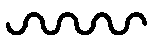 is either =O or 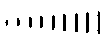 OR₃ where R₃ is either P or R₂; and
   OR₄ is either OH or R₄ is the C3 carbon in the A ring;
   for the preparation of a compound of the general formula II or a pharmaceutically acceptable salt thereof. 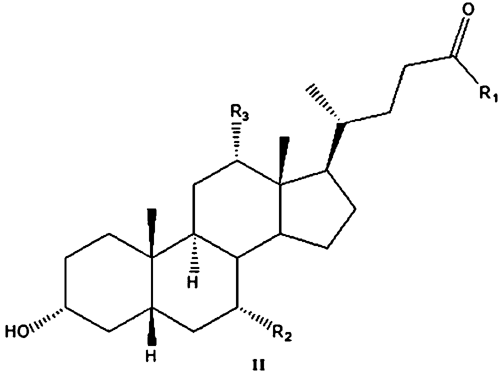
   wherein
   R₁ is OH, NHCH₂CH₂SO₃H or NHCH₂COOH;
   R₂ and R₃ is H or OH.
**AN.** Use according to claim AM1, wherein the compound of the general formula II is deoxycholic acid (DCA), cholic acid, glycocholic acid, taurocholic acid, or a pharmaceutically acceptable salt thereof.
**AO.** Use according to embodiment AM-AN, wherein the compound of the general formula I is as defined in any of embodiments U-AL.
**BA.** A deoxycholic acid or a pharmaceutically acceptable salt thereof of formula (DCA): 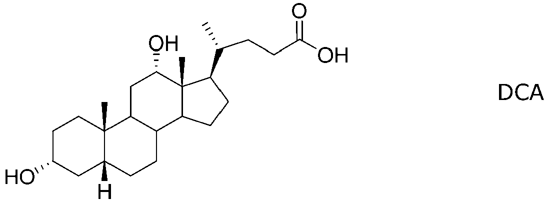 wherein the deoxycholic acid or the pharmaceutically acceptable salt thereof comprises a fossil carbon percentage of less than 10 percent and wherein the carbon atoms of the deoxycholic acid or the pharmaceutically acceptable salt thereof are derived at least partially from plant sources.
**BB.** The deoxycholic acid or a pharmaceutically acceptable salt thereof according to embodiment BA, wherein the fossil carbon percentage is less than 8 percent, such as less than 5 percent, like less than 3 percent, such as less than 1 percent, like less than 0.1 percent, such as less than 0.01 percent.
**BC.** The deoxycholic acid or a pharmaceutically acceptable salt thereof according to any of the embodiments BA-BB, wherein the carbon atoms of the deoxycholic acid or the pharmaceutically acceptable salt thereof are derived solely from plant sources.
**BD.** A deoxycholic acid or a pharmaceutically acceptable salt thereof of formula (DCA): 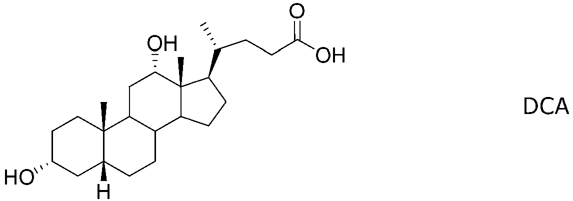 wherein the deoxycholic acid has a mean δ¹³C value different from the mean δ¹³C value of deoxycholic acid obtained from animal sources, preferably obtained from mammal sources.
**BE.** The deoxycholic acid or a pharmaceutically acceptable salt thereof according to embodiment BD, wherein the carbon atoms of the deoxycholic acid or the pharmaceutically acceptable salt thereof is derived solely or partially from plant sources.
**BF.** The deoxycholic acid or a pharmaceutically acceptable salt thereof according to any of the embodiments BD-BE, wherein the carbon atoms of the deoxycholic acid or the pharmaceutically acceptable salt thereof is derived solely or partially from phytosterols or phytosterol derivatives.
**BG.** The deoxycholic acid or a pharmaceutically acceptable salt thereof according to any of the embodiments BD-BF, wherein the deoxycholic acid has a mean δ¹³C value in the range from -20‰ to - 40‰, such as from -21‰ to -35‰, like from -20‰ to -32‰, such as from - 25‰ to -28‰, like around - 26‰.
**BH.** The deoxycholic acid or a pharmaceutically acceptable salt thereof according to any of the embodiments BD-BG, wherein the plant source is partially or solely from C3 plants.
**BI.** The deoxycholic acid or a pharmaceutically acceptable salt thereof according to any of the embodiments BD-BH, wherein the deoxycholic acid or a pharmaceutically acceptable salt thereof further comprises a fossil carbon percentage less than 10 percent, like less than 8 percent, such as less than 5 percent, like less than 3 percent, such as less than 1 percent, like less than 0.1 percent, such as less than 0.01 percent.
**BJ.** The deoxycholic acid or a pharmaceutically acceptable salt thereof according to any of the embodiments BD-BI, wherein the deoxycholic acid or a pharmaceutically acceptable salt thereof comprises a fossil carbon percentage less than 1 percent, has carbon atoms derived solely from plant sources, and has a mean δ¹³C value different from the mean δ¹³C value of deoxycholic acid obtained from animal sources.
**BK.** A deoxycholic acid or a pharmaceutically acceptable salt thereof of formula (DCA): 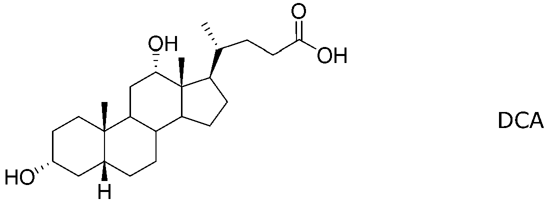 obtained by
   i) providing an intermediate of the general formula **Int A8:** 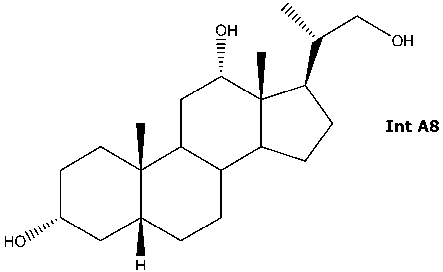 and
   ii) elongating the carbon chain of the compound of the general formula **Int A8** to obtain deoxycholic acid (DCA): 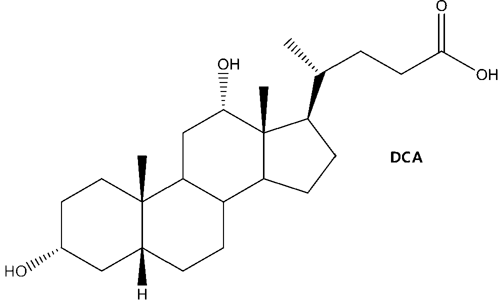 wherein
      R₂ is H or a linear or branched C₁-C₆-alkyl group;
      R₃ is H, R₂ or an alcohol protection group; and
      wherein all carbon atoms of the compounds and intermediates derive from plant sources.
**BL.** The deoxycholic acid or a pharmaceutically acceptable salt thereof according to embodiment BK, wherein the deoxycholic acid or a pharmaceutically acceptable salt thereof comprises a fossil carbon percentage of less than 10 percent, like less than 8 percent, such as less than 5 percent, like less than 3 percent, such as less than 1 percent, like less than 0.1 percent, such as less than 0.01 percent.
**BM.** The deoxycholic acid or a pharmaceutically acceptable salt thereof according to any of the embodiments BK-BL, wherein the deoxycholic acid or a pharmaceutically acceptable salt thereof has a mean δ¹³C value different from the mean δ¹³C value of deoxycholic acid obtained from animal sources, preferable mammal sources.
**BN.** The deoxycholic acid or a pharmaceutically acceptable salt thereof according to any of the embodiments BK-BM, wherein the deoxycholic acid has a mean δ¹³C value in the range from -20‰ to - 40‰, such as from -21‰ to -35‰, like from -20‰ to -32‰, such as from - 25‰ to -28‰, like around - 26‰.
**BO.** The deoxycholic acid or a pharmaceutically acceptable salt thereof according to any of the embodiments BK-BN, wherein the deoxycholic acid or a pharmaceutically acceptable salt thereof comprises a fossil carbon percentage of less than 1 percent and a mean δ¹³C value different from the mean δ¹³C value of deoxycholic acid obtained from animal sources.
**BP.** The deoxycholic acid or a pharmaceutically acceptable salt thereof according to any of the embodiments BK-BO, wherein the carbon chain of the compound of the general formula **Int A8** is elongated to DCA as described below:
   i) converting the primary alcohol in the general formula **Int A8** into a leaving group (X) to obtain an intermediate of the general formula **Int A9:** 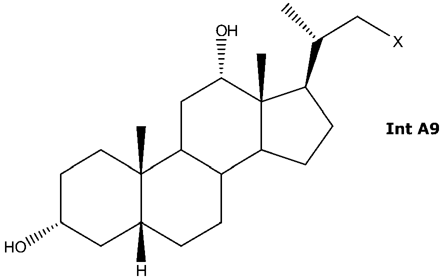 where X is OMs, OTs or halogen, preferably, Cl, Br or I; and
   ii) elongating the carbon chain of the compound of the general formula **Int A9** to obtain **DCA:** 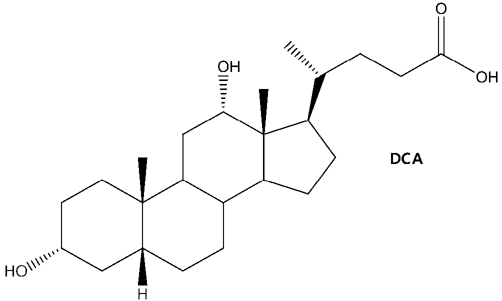
**BQ.** The deoxycholic acid or a pharmaceutically acceptable salt thereof according to embodiment BP, wherein the compound of the general formula **Int A9** is converted into the compound of the general formula **Int A10,** which is then converted into **DCA.** 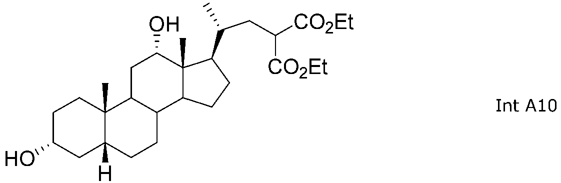
**BR.** The deoxycholic acid or a pharmaceutically acceptable salt thereof according to embodiment BQ, wherein the compound of the general formula **Int A10** is converted into the compound of the general formula **Int A11,** which is converted into **DCA.** 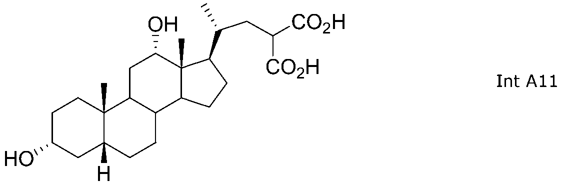
**BS.** The deoxycholic acid or a pharmaceutically acceptable salt thereof according to embodiment BQ, wherein the compound of the general formula **Int A9** is converted into the compound of the general formula **Int A10** using diethyl malonate, preferably obtained from plant sources such as sugar fermentation.
**BT.** The deoxycholic acid or a pharmaceutically acceptable salt thereof according to embodiment BP, wherein the compound of the general formula **Int A9** is converted into **DCA** by substituting the leaving group X with acetonitrile followed by hydrolysis.
**BU.** The deoxycholic acid or a pharmaceutically acceptable salt thereof according to embodiment BT, wherein the acetonitrile is prepared from acetic acid, which is obtained from a plant source such as from a fermentation process.
**BV.** The deoxycholic acid or a pharmaceutically acceptable salt thereof according to embodiment BR, wherein the deoxycholic acid is obtained by refluxing a reaction mixture of sodium chloride and the compound of the general formula **Int A11.**
**BW.** The deoxycholic acid or a pharmaceutically acceptable salt thereof according to embodiment BQ, wherein the deoxycholic acid is obtained by reacting the compound of the general formula **Int A10** with sodium hydroxide.
**BX.** The deoxycholic acid or a pharmaceutically acceptable salt thereof according to any of the embodiments BK-BW further comprising the steps of:
   i) providing an intermediate of the general formula **Int 7:** 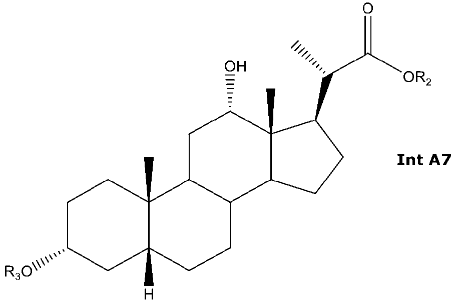 wherein
      R₂ is H or a linear or branched C₁-C₆-alkyl group; and
      R₃ is H, R₂ or an alcohol protection group
   ii) reducing the intermediat of the general formula **Int A7** into the intermediate of the general formula **Int A8.**
**BY.** The deoxycholic acid or a pharmaceutically acceptable salt thereof according to embodiment BX further comprising the steps of:
   i) providing an intermediate of the general formula **Int 6** 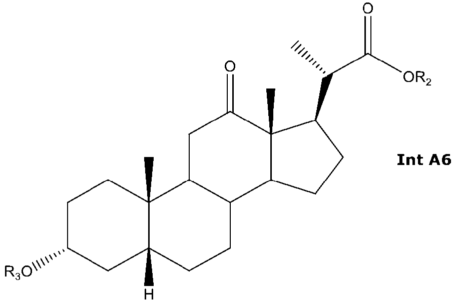 wherein
      R₂ is H or a linear or branched C₁-C₆-alkyl group; and
      R₃ is H, R₂ or an alcohol protection group
   ii) reducing the intermediate of the general formula **Int A6** into the intermediate of the general formula **Int A7.**
**BZ.** The deoxycholic acid or a pharmaceutically acceptable salt thereof according to embodiment BY further comprising the steps of:
   i) providing an intermediate of the general formula **Int A5** 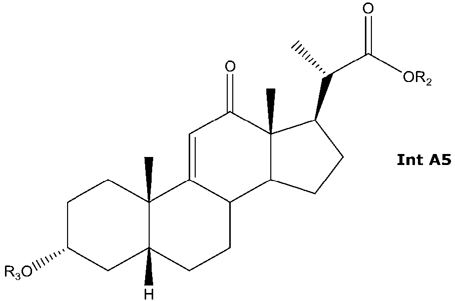 wherein
      R₂ is H or a linear or branched C₁-C₆-alkyl group; and
      R₃ is H, R₂ or an alcohol protection group
   ii) reducing the intermediate of the general formula **Int A5** into the intermediate of the general formula **Int A6.**
**CA.** The deoxycholic acid or a pharmaceutically acceptable salt thereof according to embodiment BZ further comprising the steps of:
   i) providing an intermediate of the general formula **Int A3** 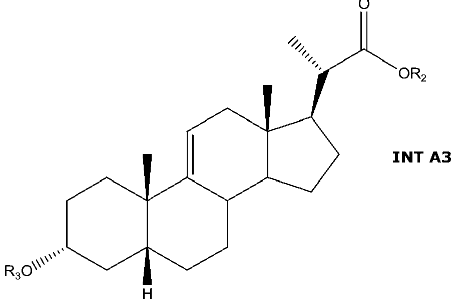 wherein
      R₂ is H or a linear or branched C₁-C₆-alkyl group; and
      R₃ is H, R₂ or an alcohol protection group
   ii) oxidising the intermediate of the general formula **Int A3** into the intermediate of the general formula **Int A5.**
**CB.** The deoxycholic acid or a pharmaceutically acceptable salt thereof according to embodiment CA further comprising the steps of:
   i) providing an intermediate of the general formula **Int A2** 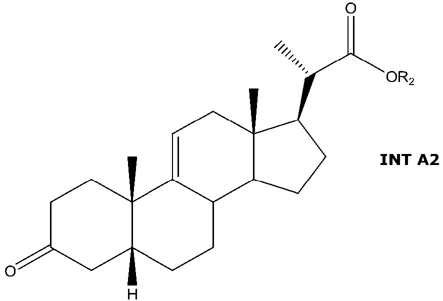 wherein
      R₂ is H or a linear or branched C₁-C₆-alkyl group
   ii) reducing the intermediate of the general formula **Int A2** into the intermediate of the general formula **Int A3.**
**CC.** The deoxycholic acid or a pharmaceutically acceptable salt thereof according to embodiment CB further comprising the steps of:
   i) providing an intermediate of the general formula **Int A1** 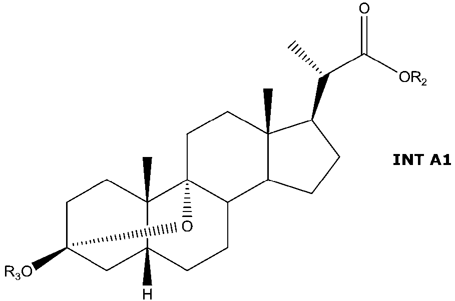 wherein
      R₂ is H or a linear or branched C₁-C₆-alkyl group; and
      R₃ is H, R₂ or an alcohol protection group
   ii) converting the intermediate of the general formula **Int A1** into the intermediate of the general formula **Int A2.**
**CD.** The deoxycholic acid or a pharmaceutically acceptable salt thereof according to embodiment CC further comprising the steps of:
   i) providing a compound of the general formula **SM-a** 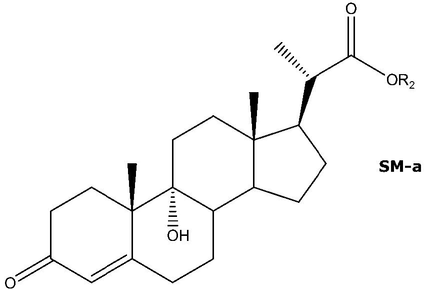 wherein
      R₂ is H or a linear or branched C₁-C₆-alkyl group;
   ii) reducing, and optionally adding an alcohol protection group to, the compound of the general formula **SM-a** to obtain an intermediate of the general formula **Int A1.**
**CE.** The deoxycholic acid or the pharmaceutically acceptable salt thereof according to any of the preceding embodiments BA-CD, wherein the deoxycholic acid or the pharmaceutically acceptable salt thereof is further distinguishable from deoxycholic acid of animal origin by difference in hydrogen isotopes or other naturally occurring isotopes or other applicable marker(s).
**CF.** A compound of the general formula **Int A10** 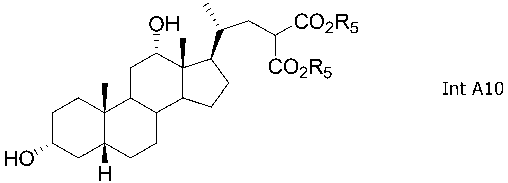
   wherein R₅ is methyl or ethyl; and
   wherein the carbon atoms of the compound are derived solely from plant sources.
**CG.** A compound of the general formula **Int A11** 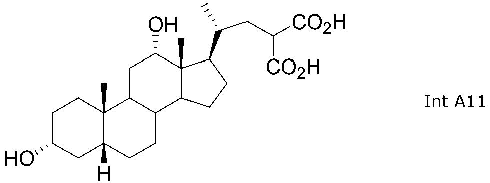 wherein the carbon atoms of the compound are derived solely from plant sources.
**CH.** A deoxycholic acid mixture comprising a deoxycholic acid or a pharmaceutically acceptable salt thereof as described according to any of the embodiments BA-CE and deoxycholic acid or a pharmaceutically acceptable salt thereof obtained from an animal origin.
**CI.** A process for the preparation of deoxycholic acid (DCA) or a pharmaceutically acceptable salt thereof, comprising the following steps:
   i) providing a compound of the general formula **SM-a:** 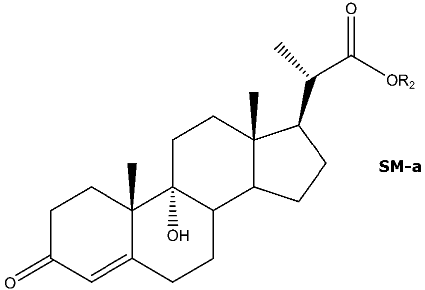
   ii) reducing the compound of the general formula **SM-a** to obtain an intermediate of the general formula **Int A1:** 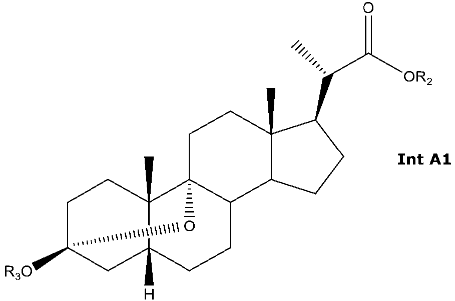
   iii) converting the intermediate of the general formula **Int A1** into an intermediate of the general formula **Int A2:** 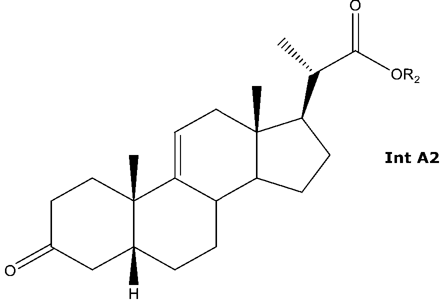
   iv) reducing the intermediate of the general formula **Int A2** into an intermediate of the general formula **Int A3:** 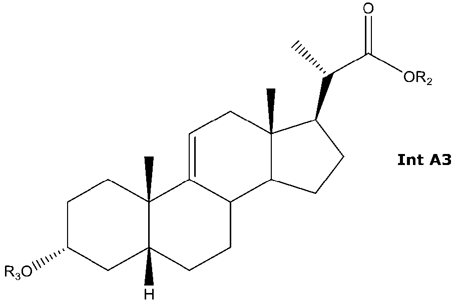
   v) oxidising the intermediate of the general formula **Int A3** into an intermediate of the general formula **Int A5:** 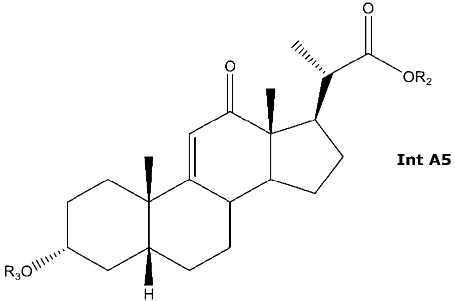
   vi) reducing the intermediate of the general formula **Int A5** into an intermediate of the general formula **Int A6:** 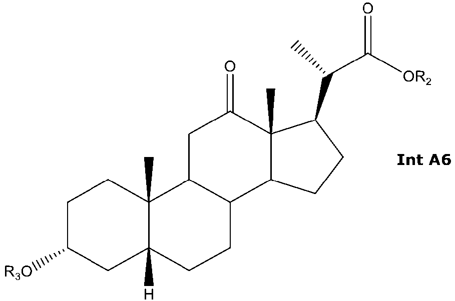
   vii) reducing the intermediate of the general formula **Int A6** into an intermediate of the general formula **Int A7:** 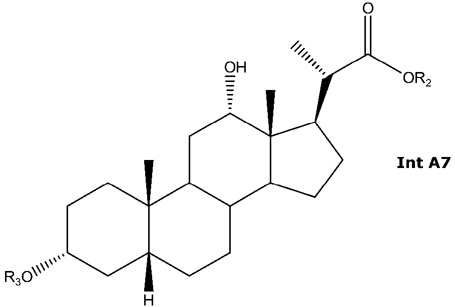
   viii) reducing the compound of the general formula **Int A7** into an intermediate of the general formula **Int A8:** 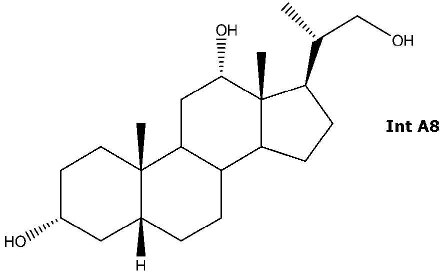
   ix) converting the primary alcohol in the genral formula **Int A8** into a leaving group (X) to obtain an intermediate of the genral formula **Int A9:** 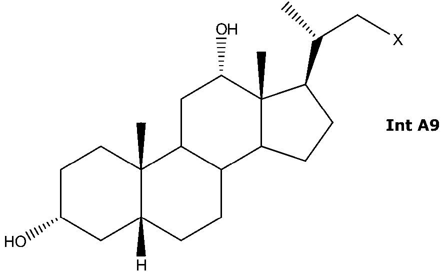 where X is OMs, OTs or halogen, preferably, Cl, Br or I;
   x) converting the compound of the general formula **Int A9** into deoxycholic acid (DCA):

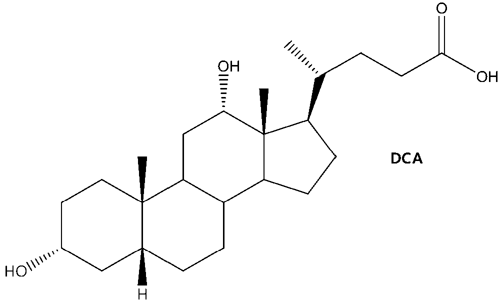

   By means of route P1:
      Pi) converting the compound of the general formula **Int A9** into the compound of the general formula **Int A10:** 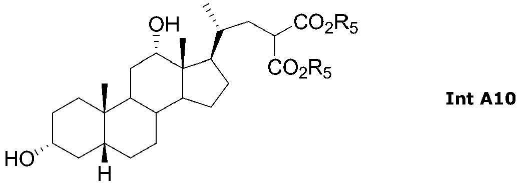
      Pii) optionally, converting the compound of the general formula **Int A10** into the compound of the general formula **Int A11:** 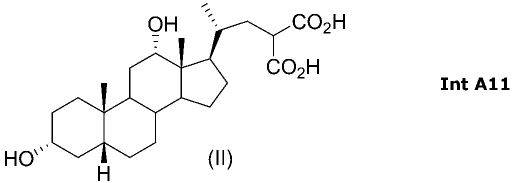
      Piii) elongating the carbon chain of the compound of the general formula **Int A10** or **Int A11** to obtain deoxycholic acid (DCA)
      or by means of route P2:
         Pi') converting the general formula **Int A9** into the compound of general formula **A10b** 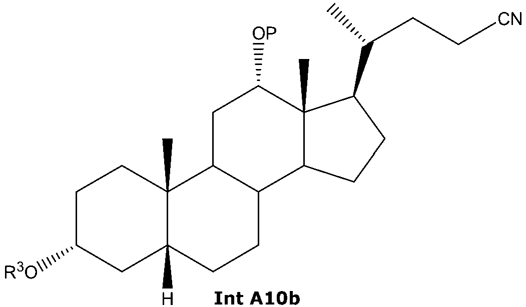
         Pii') hydrolysing the coumpound of general formula **Int A10b** to obtain deoxycholic acid (DCA)
         x) optionally converting deoxycholic acid to a pharmaceutically acceptable salt thereof,
         wherein
         R₂ is H or a linear or branched C₁-C₆-alkyl group;
         P is an alcohol protection group;
         R₃ is H, R₂ or an alcohol protection group;
         R₅ is Methyl or Ethyl.
**CJ.** The process according to claim 13 wherein wherein the compound of the general formula **Int A9** is converted into the compound of the general formula **Int A10** using diethyl malonate, preferably obtained from plant sources such as sugar fermentation.
**CK.** The process according to any of the claims 13, wherein the acetonitrile is prepared from acetic acid, which is obtained from a plant source such as from a fermentation process.
**CL.** The process according to any of the claims 13-14, wherein the deoxycholic acid is obtained by refluxing a reaction mixture of sodium chloride and the compound of the general formula **Int A11.**
**CM.** The process according to any of the claims 13-14 and 16, wherein the deoxycholic acid is obtained by reacting the compound of the general formula **Int A10** with sodium hydroxide.
**CN.** A compound of the general formula Int A10 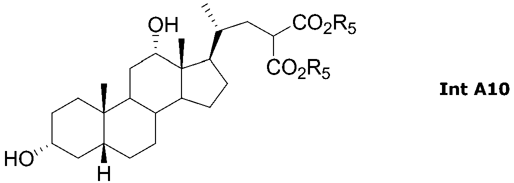
   wherein R₅ is methyl or ethyl
   obtained by a method as described in any of the embodiments CI-CM.
**CO.** A compound of the general formula Int A11 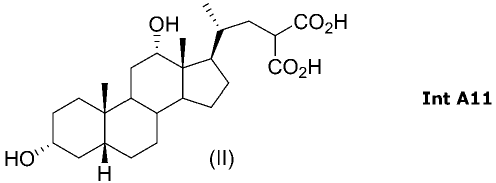 obtained by a method as described in any of the embodiments CI-CM.
**CP.** A compound of the general formula **Int A10b** 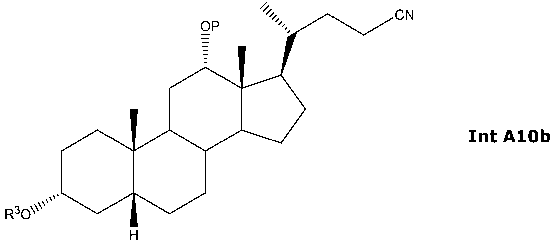 obtained by a method as described in any of the embodiments CI-CM and wherein
   R₂ is H or a linear or branched C₁-C₆-alkyl group;
   P is an alcohol protection group; and
   R₃ is H, R₂ or an alcohol protection group.

### Items

1. A deoxycholic acid or a pharmaceutically acceptable salt thereof of formula (DCA): 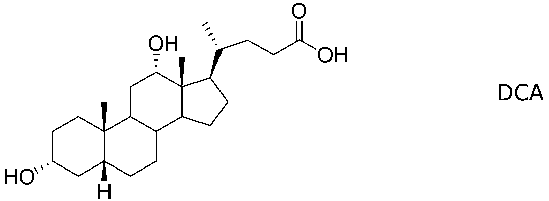 wherein the deoxycholic acid or the pharmaceutically acceptable salt thereof comprises a fossil carbon percentage of less than 3 percent, such as less than 1 percent, like less than 0.1 percent, such as less than 0.01 percent, and wherein the deoxycholic acid has a mean δ¹³C value in the range from -20‰ to -40‰.
2. The deoxycholic acid or a pharmaceutically acceptable salt thereof according to item 1, wherein the carbon atoms of the deoxycholic acid or the pharmaceutically acceptable salt thereof are derived solely from plant sources.
3. The deoxycholic acid or a pharmaceutically acceptable salt thereof according to any of the items 1-2, wherein the carbon atoms of the deoxycholic acid or the pharmaceutically acceptable salt thereof is derived solely or partially from plant sources, such as partially from phytosterols or phytosterol derivatives.
4. The deoxycholic acid or a pharmaceutically acceptable salt thereof according to any of the items 1-3, wherein the deoxycholic acid has a mean δ¹³C value in the range from -21‰ to -35‰, like from -20‰ to -32‰, such as from - 25‰ to - 28‰, like around - 26‰.
5. A deoxycholic acid or a pharmaceutically acceptable salt thereof of formula (DCA): 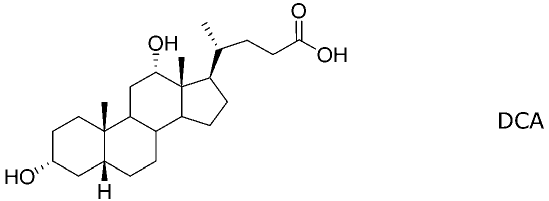 obtained by
   i) providing an intermediate of the general formula **Int A8:** 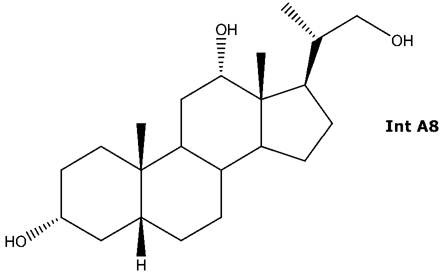 and
   ii) elongating the carbon chain of the compound of the general formula **Int A8** to obtain deoxycholic acid (DCA): 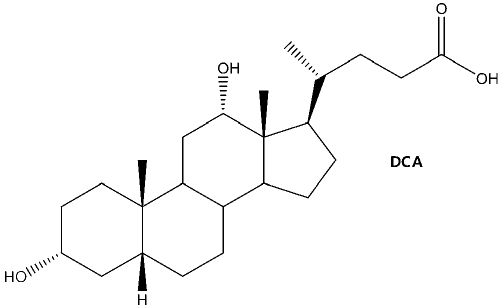 wherein all carbon atoms of the compounds and intermediates derive from plant sources.
6. The deoxycholic acid or a pharmaceutically acceptable salt thereof according to item 5, wherein the deoxycholic acid or a pharmaceutically acceptable salt thereof comprises a fossil carbon percentage of less than 3 percent, such as less than 1 percent, like less than 0.1 percent, such as less than 0.01 percent, and wherein the deoxycholic acid has a mean δ¹³C value in the range from -20‰ to - 40‰.
7. The deoxycholic acid or a pharmaceutically acceptable salt thereof according to item 6, wherein the deoxycholic acid has a mean δ¹³C value in the range from - 21‰ to -35‰, like from -20‰ to -32‰, such as from - 25‰ to -28‰, like around - 26‰.
8. The deoxycholic acid or a pharmaceutically acceptable salt thereof according to any of the items 6-7, wherein the carbon chain of the compound of the general formula **Int A8** is elongated to DCA as described below:
   i) converting the primary alcohol in the general formula **Int A8** into a leaving group (X) to obtain an intermediate of the general formula **Int A9:** 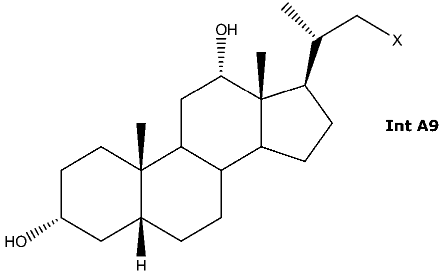 where X is OMs, OTs or halogen, preferably, Cl, Br or I; and
   ii) elongating the carbon chain of the compound of the general formula **Int A9** to obtain **DCA:** 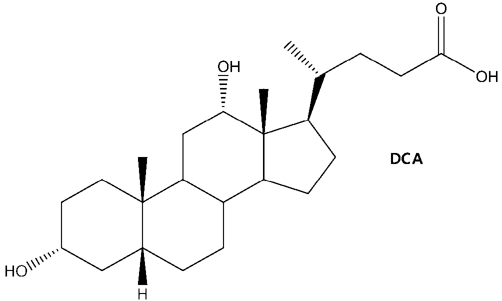
9. The deoxycholic acid or a pharmaceutically acceptable salt thereof according to item 8, wherein the compound of the general formula **Int A9** is converted into **Int A10,** which is then converted into **DCA.** 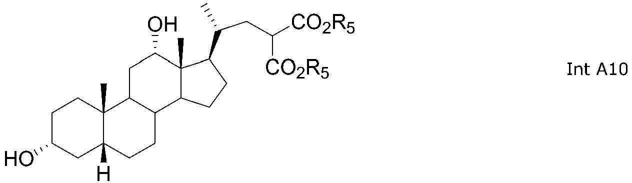 wherein R₅ is methyl or ethyl.
10. The deoxycholic acid or a pharmaceutically acceptable salt thereof according to item 9, wherein **Int A10** is converted into **Int A11,** which is converted into **DCA.** 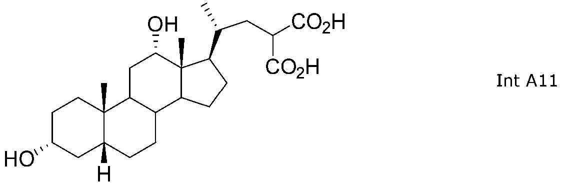
11. A compound of the general formula **Int A10** 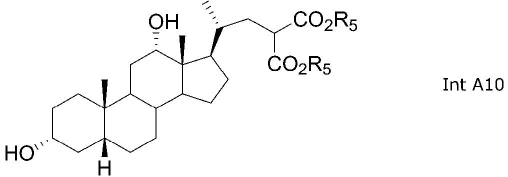
   wherein R₅ is methyl or ethyl; and
   wherein the carbon atoms of the compound are derived solely from plant sources.
12. A compound of the general formula **Int A11** 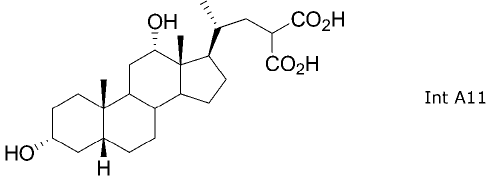 wherein the carbon atoms of the compound are derived solely from plant sources.
13. A deoxycholic acid mixture comprising a deoxycholic acid or a pharmaceutically acceptable salt thereof as described according to any of the items 1-10 and deoxycholic acid or a pharmaceutically acceptable salt thereof obtained from an animal origin.
14. A process for the preparation of deoxycholic acid (DCA) or a pharmaceutically acceptable salt thereof, comprising the following steps:
   i) providing a compound of the general formula **SM-a:** 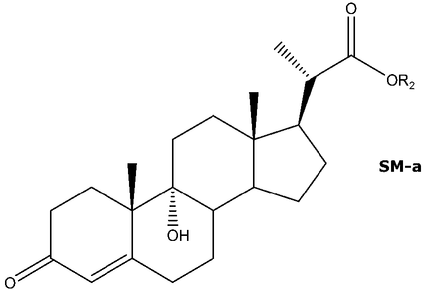
   ii) reducing the compound of the general formula **SM-a** to obtain an intermediate of the general formula **Int A1:** 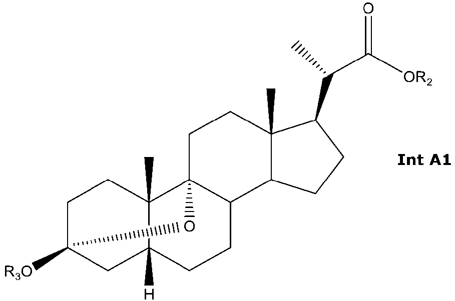
   iii) converting the intermediate of the general formula **Int A1** into an intermediate of the general formula **Int A2:** 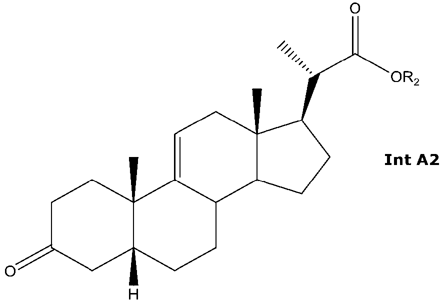
   iv) reducing the intermediate of the general formula **Int A2** into an intermediate of the general formula **Int A3:** 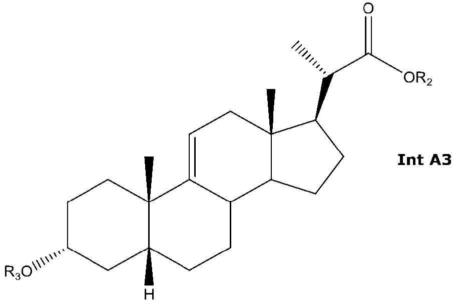
   v) oxidising the intermediate of the general formula **Int A3** into an intermediate of the general formula **Int A5:** 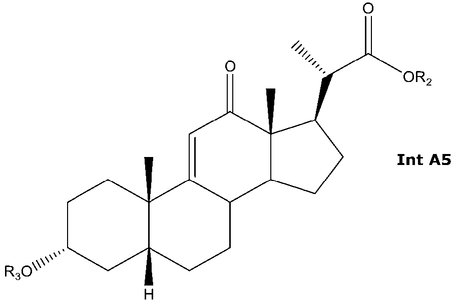
   vi) reducing the intermediate of the general formula **Int A5** into an intermediate of the general formula **Int A6:** 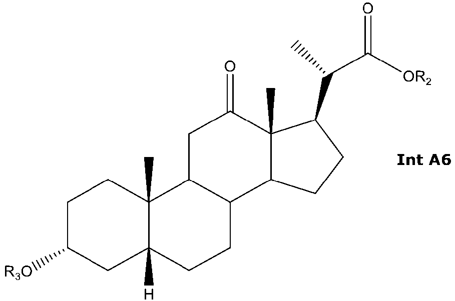
   vii) reducing the intermediate of the general formula **Int A6** into an intermediate of the general formula **Int A7:** 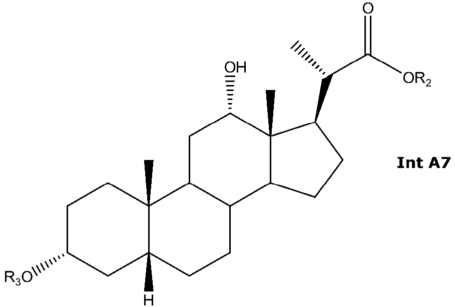
   viii) reducing the compound of the general formula **Int A7** into an intermediate of the general formula **Int A8:** 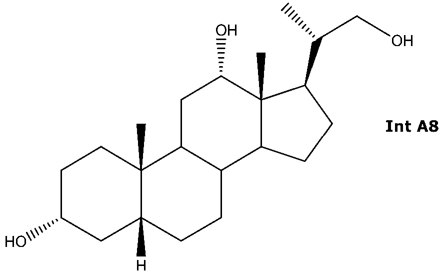
   ix) converting the primary alcohol in the genral formula **Int A8** into a leaving group (X) to obtain an intermediate of the genral formula **Int A9:** 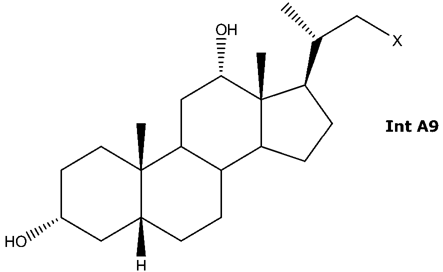 where X is OMs, OTs or halogen, preferably, Cl, Br or I;
   x) converting the compound of the general formula **Int A9** into deoxycholic acid (DCA):

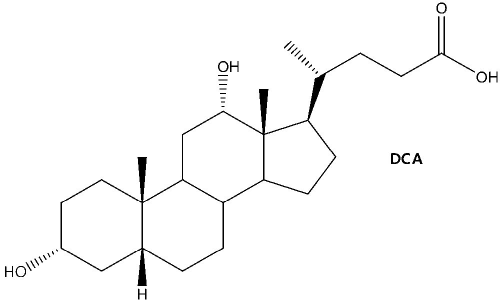

   By means of route P1:
      Pi) converting the compound of the general formula **Int A9** into the compound of the general formula **Int A10:** 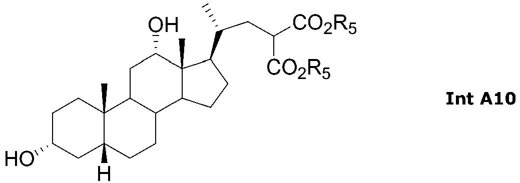
      Pii) optionally, converting the compound of the general formula **Int A10** into the compound of the general formula **Int A11:** 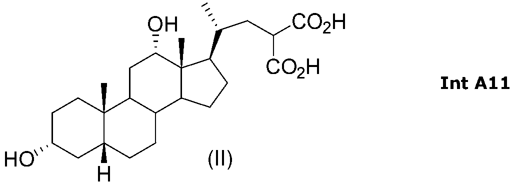
      Piii) elongating the carbon chain of the compound of the general formula **Int A10** or **Int A11** to obtain deoxycholic acid (DCA)
   Or by means of route P2:
      Pi') converting the general formula **Int A9** into the compound of general formula **A10b** 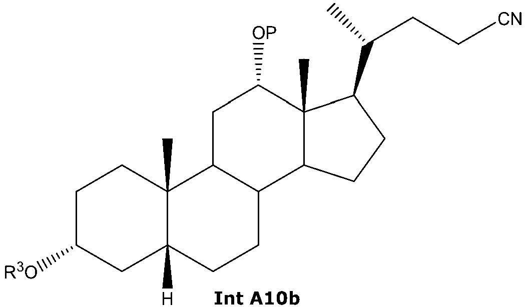
      Pii') hydrolysing the coumpound of general formula **Int A10b** to obtain deoxycholic acid (DCA)
      x) optionally converting deoxycholic acid to a pharmaceutically acceptable salt thereof,
         wherein
         R₂ is H or a linear or branched C₁-C₆-alkyl group;
         P is an alcohol protection group;
         R₃ is H, R₂ or an alcohol protection group; and
         R₅ is methyl or ethyl.
15. The process according to item 14, wherein the compound of the general formula **Int A9** is converted into the compound of the general formula **Int A10** using diethyl malonate, preferably obtained from plant sources such as sugar fermentation.
16. The process according to any of the items 14, wherein the acetonitrile is prepared from acetic acid, which is obtained from a plant source such as from a fermentation process.
17. The process according to any of the items 14-15, wherein the deoxycholic acid is obtained by refluxing a reaction mixture of sodium chloride and the compound of the general formula **Int A11.**
18. The process according to any of the items 14-15 and 17, wherein the deoxycholic acid is obtained by reacting the compound of the general formula **Int A10** with sodium hydroxide.
19. A compound of the general formula **Int A10** 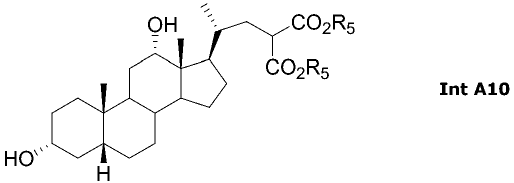
   wherein R₅ is methyl or ethyl; and
   obtained by a method as described in any of the items 14-18.
19. A compound of the general formula **Int A11** 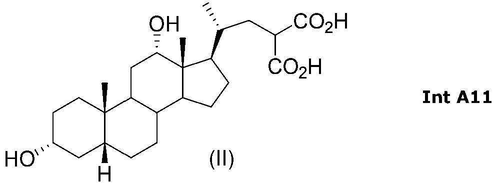 obtained by a method as described in any of the items 14-18.
20. A compound of the general formula **Int A10b** 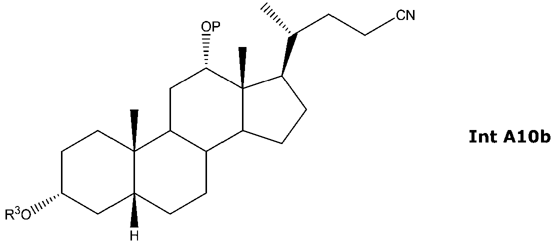 obtained by a method as described in any of the items 14-18 and wherein
   R₂ is H or a linear or branched C₁-C₆-alkyl group;
   P is an alcohol protection group;
   R₃ is H, R₂ or an alcohol protection group.

## Claims

1. A deoxycholic acid or a pharmaceutically acceptable salt thereof of formula (DCA): 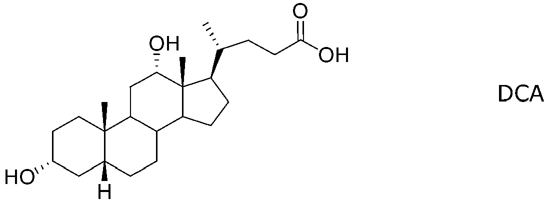 wherein the deoxycholic acid or the pharmaceutically acceptable salt thereof comprises a fossil carbon percentage of less than 0.1 percent, and wherein the deoxycholic acid has a mean δ¹³C value in the range from -25‰ to -32‰, preferably -26‰ to -32‰, more preferably 28‰ to -32‰, and wherein the carbon atoms of the deoxycholic acid or the pharmaceutically acceptable salt thereof are derived solely from plant sources.

2. The deoxycholic acid or a pharmaceutically acceptable salt thereof according to claim 1, wherein the carbon atoms of the deoxycholic acid or the pharmaceutically acceptable salt thereof is derived solely from plant sources, such as partially from phytosterols or phytosterol derivatives.

3. A deoxycholic acid or a pharmaceutically acceptable salt thereof of formula (DCA): 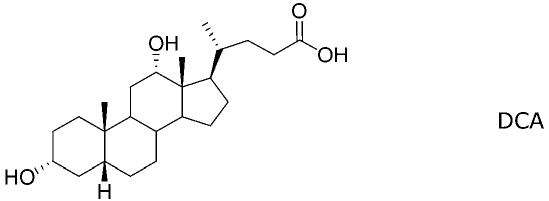 obtained by
i) providing an intermediate of the general formula **Int A8:** 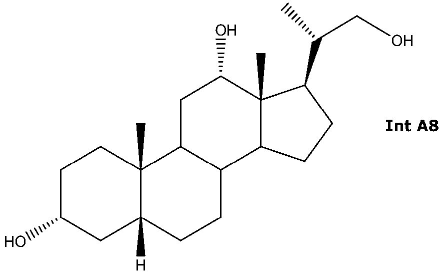 and
ii) elongating the carbon chain of the compound of the general formula **Int A8** to obtain deoxycholic acid (DCA): 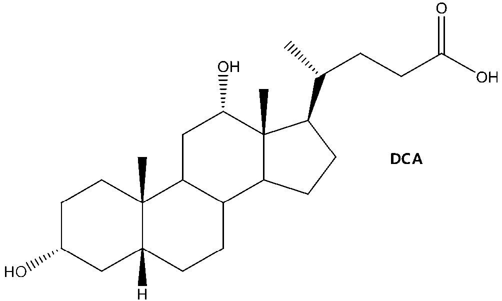 wherein all carbon atoms of the compounds and intermediates derive from plant sources, wherein the deoxycholic acid or a pharmaceutically acceptable salt thereof comprises a fossil carbon percentage of less than 0.1 percent, and wherein the deoxycholic acid has a mean δ¹³C value in the range from -25‰ to - 32‰ preferably -26‰ to -32‰, more preferably 28‰ to -32‰.

4. The deoxycholic acid or a pharmaceutically acceptable salt thereof according to claim 3, wherein the carbon chain of the compound of the general formula **Int A8** is elongated to DCA as described below:
i) converting the primary alcohol in the general formula **Int A8** into a leaving group (X) to obtain an intermediate of the general formula **Int A9:** 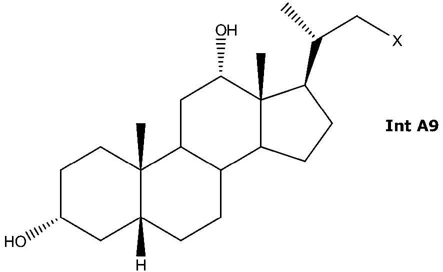 where X is OMs, OTs or halogen, preferably, Cl, Br or I; and
ii) elongating the carbon chain of the compound of the general formula **Int A9** to obtain **DCA:**

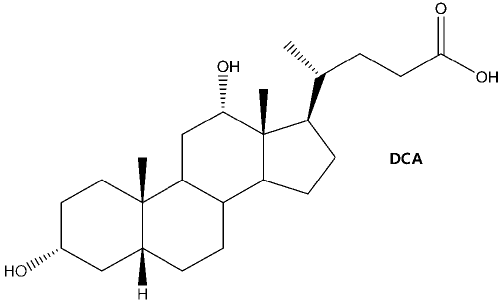

5. The deoxycholic acid or a pharmaceutically acceptable salt thereof according to claim 4, wherein the compound of the general formula **Int A9** is converted into **Int A10,** which is then converted into **DCA.** 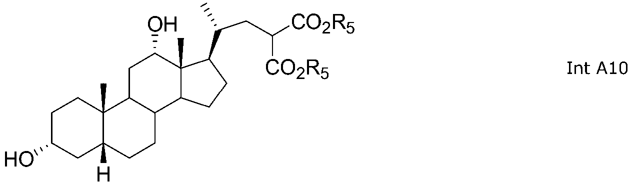 wherein R₅ is methyl or ethyl.

6. The deoxycholic acid or a pharmaceutically acceptable salt thereof according to claim 5, wherein **Int A10** is converted into **Int A11,** which is converted into **DCA.**

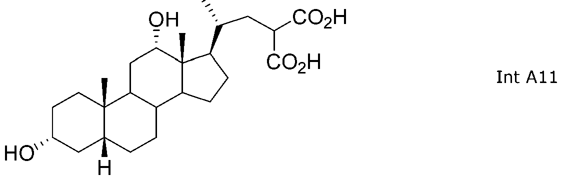

7. A process for the preparation of deoxycholic acid (DCA) or a pharmaceutically acceptable salt thereof, comprising the following steps:
I) providing a compound of the general formula **SM:** 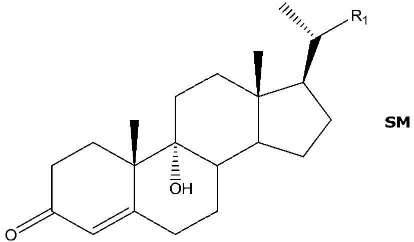
II) reducing the compound of the general formula **SM** to obtain an intermediate of the general formula **INT** 1: 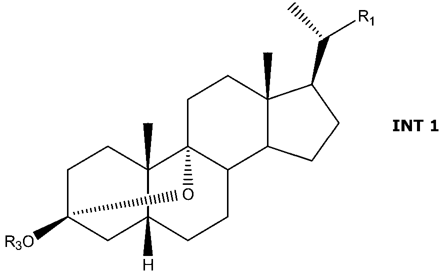
III) converting the intermediate of the general formula **INT** 1 into an intermediate of the general formula **INT 2:** 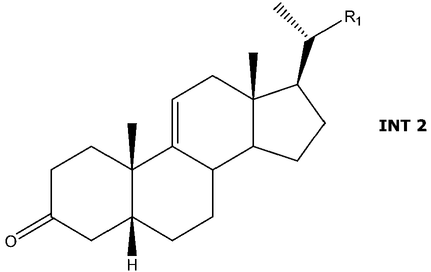
IVa) reducing the intermediate of the general formula **INT** 2 into an intermediate of the general formula **INT 3:** 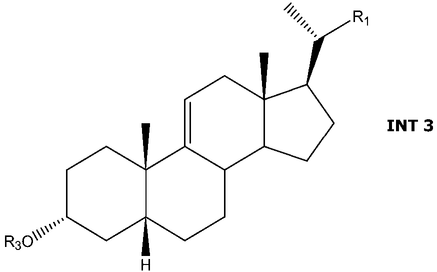 followed by converting the intermediate of the general formula **INT** 3 into an intermediate of the general formula **INT B:** 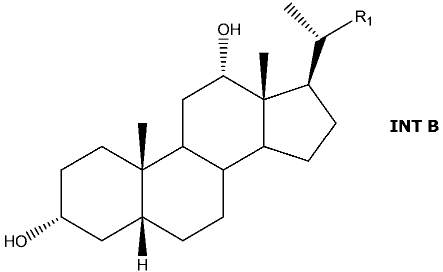 or
IVb) converting the intermediate of the general formula **INT** 2 into an intermediate with the general formula **INT B:** 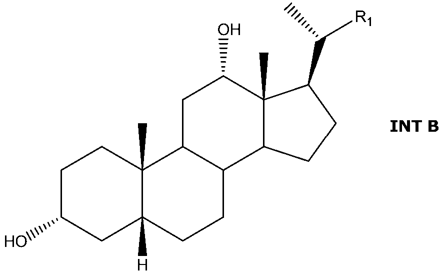
V) converting the intermediate of the general formula **INT** B into deoxycholic acid (DCA): 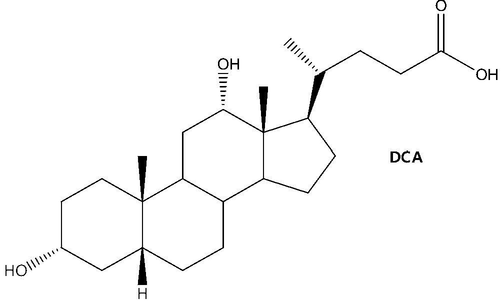
VI) optionally converting deoxycholic acid to a pharmaceutically acceptable salt thereof,
wherein
R₁ is COOR₂, CH₂OH, CH₂OP, CH₂X, CH₂CHO, CH₂-CH₂-OH, CH₂-CH₂OP, or CH₂-CH₂X or CH₂-CH₂-CHO;
R₂ is H or a linear or branched C₁-C₆-alkyl group;
P is an alcohol protection group;
R₃ either P or R₂; and
X is a halogen atom.

8. A process for the preparation of deoxycholic acid (DCA) or a pharmaceutically acceptable salt thereof, comprising the following steps:
I) providing a compound of the general formula **INT** 3: 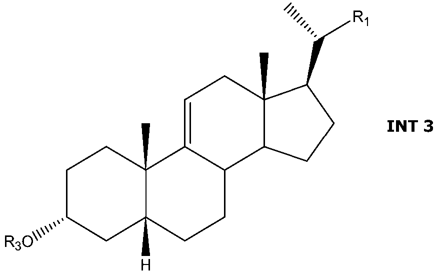
II) converting the intermediate of the general formula **INT** 3 into an intermediate of the general formula **INT B:** 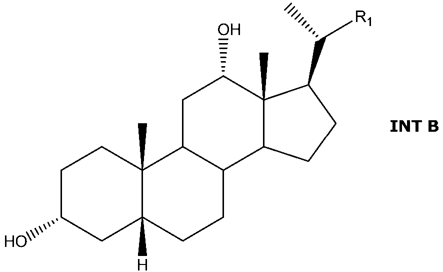
III) converting the intermediate of the general formula **INT** B into deoxycholic acid (DCA): 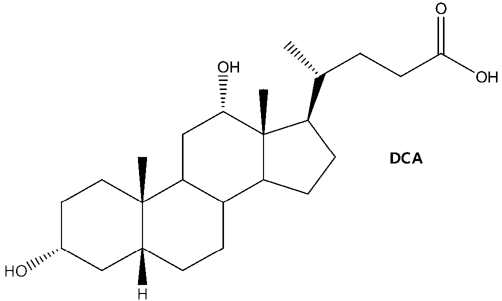
IV) optionally converting deoxycholic acid to a pharmaceutically acceptable salt thereof,
wherein
R₁ is COOR₂, CH₂OH, CH₂OP, CH₂X, CH₂CHO, CH₂-CH₂-OH, CH₂-CH₂OP, or CH₂-CH₂X or CH₂-CH₂-CHO;
R₂ is H or a linear or branched C₁-C₆-alkyl group;
P is an alcohol protection group;
R₃ either P or R₂; and
X is a halogen atom.

9. The process according to claim 8, comprising the following steps:
i) providing a compound of the general formula **Int A3:** 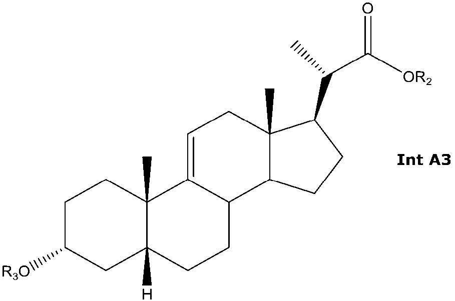
ii) oxidising the intermediate of the general formula **Int A3** into an intermediate of the general formula **Int A5:** 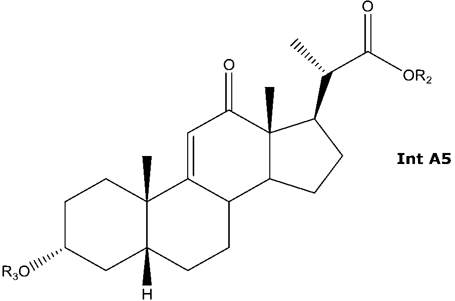
iii) reducing the intermediate of the general formula **Int A5** into an intermediate of the general formula **Int A6:** 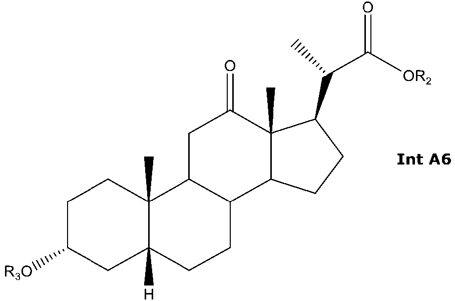
iv) reducing the intermediate of the general formula **Int A6** into an intermediate of the general formula **Int A7:** 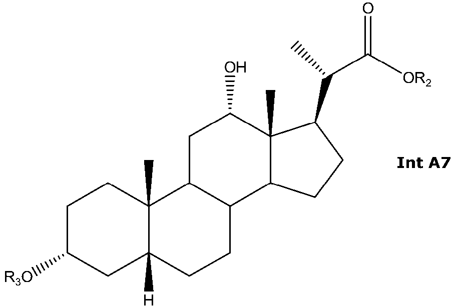
v) reducing the compound of the general formula **Int A7** into an intermediate of the general formula **Int A8:** 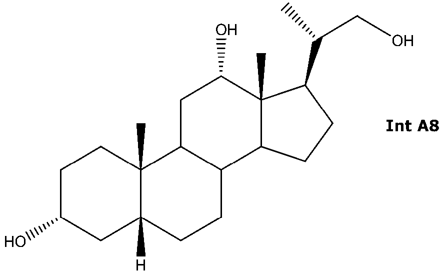
vi) elongating the carbon chain of the compound of the general formula **Int A8** to obtain deoxycholic acid (DCA): 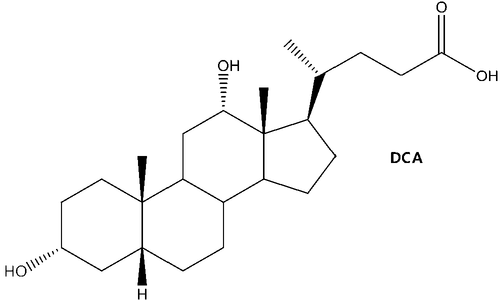
vii) optionally converting deoxycholic acid to a pharmaceutically acceptable salt thereof,
wherein
R₂ is H or a linear or branched C₁-C₆-alkyl group;
R₃ is H, R₂ or an alcohol protection group.

10. The process according to claim 9, wherein **INT A3** is provided from **INT A2** by reducing the intermediate of the general formula **Int A2** into an intermediate of the general formula **Int A3** and/or wherein **INT A2** is provided from **INT A1** by reducing the compound of the general formula **SM-a** to obtain an intermediate of the general formula **Int A1**,and converting the intermediate of the general formula **Int A1** into an intermediate of the general formula **Int** A2, preferably wherein **INT A1** is provided from **SM-a** by reducing the compound of the general formula **SM-a** to obtain an intermediate of the general formula **Int A1.**

11. The compound having the general formula **SM** 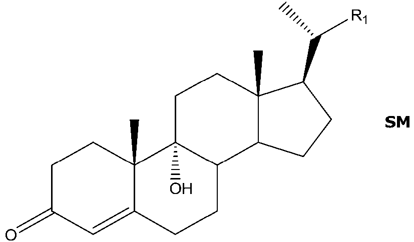 wherein
R₁ is COOR₂, CH₂OP, CH₂X, CH₂CHO, CH₂-CH₂-OH, CH₂-CH₂OP, or CH₂-CH₂XR₂ is a linear or branched C₁-C₆-alkyl group with the proviso that R₂ is not CH₃;
P is an alcohol protection group with the proviso that P is not Ac; and
X is a halogen atom.

12. The compound having the general formula **INT 1** 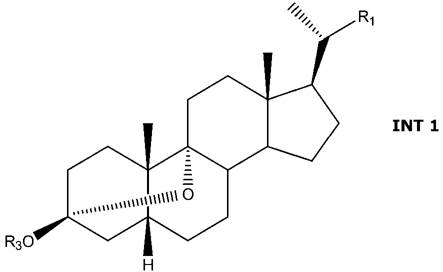 wherein
R₁ is COOR₂, CH₂OH, CH₂OP, CH₂X, CH₂CHO, CH₂-CH₂-OH, CH₂-CH₂OP, CH₂-CH₂X or CH₂-CH₂-CHO;
R₂ is H or a linear or branched C₁-C₆-alkyl group;
P is an alcohol protection group;
R₃ is either P or R₂; and
X is a halogen atom.

13. The compound having the general formula **INT 2** 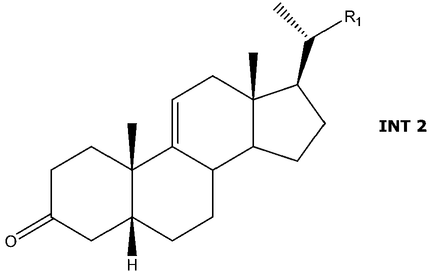 wherein
R₁ is COOR₂, CH₂OP, CH₂X, CH₂CHO, CH₂-CH₂-OH, CH₂-CH₂OP or CH₂-CH₂X;
R₂ is H or a linear or branched C₁-C₆-alkyl group;
P is an alcohol protection group; and
X is a halogen atom.

14. The compound having the general formula **INT 3** 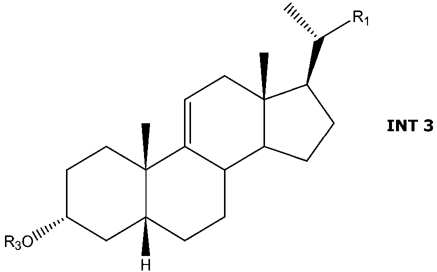 wherein
R₁ is COOR₂, CH₂OP, CH₂X, CH₂CHO, CH₂-CH₂-OH, CH₂-CH₂OP or CH₂-CH₂X;
R₂ is H or a linear or branched C₁-C₆-alkyl group;
P is an alcohol protection group;
R₃ is either P or R₂; and
X is a halogen atom.

15. The compound according to any of claims 11-14, wherein R₁ is COOR₂, , CH₂OP or CH₂X, where
R₂ is H or a linear or branched C₁-C₆-alkyl group;
P is an alcohol protection group;
R₃ is either P or R₂; and
X is a halogen atom.
